# EUROPEAN PATENT APPLICATION

(11) **EP 4 458 861 A1**
(43) Date of publication of application: **06.11.2024**
(21) Application number: 22914863.0
(22) Date of filing: 28.12.2022
(51) Int. Cl.: C07K 16/46, A61P 35/00, A61K 39/395

(54) **ANTIBODY TARGETING CD3, MULTISPECIFIC ANTIBODY, AND USES THEREOF**

(30) Priority: 29.12.2021 CN 202111639610
(71) Applicant: Sichuan Huiyu Pharmaceutical Co., Ltd., Neijiang, Sichuan 641000 (CN); Sichuan Huiyu Seacross Pharmaceutical Technology Co., Ltd., Chengdu, Sichuan 610200 (CN)
(72) Inventor: HU, Yan, NEIJIANG, Sichuan 641000 (CN); GUO, Sanyou, NEIJIANG, Sichuan 641000 (CN); TENG, Yumin, NEIJIANG, Sichuan 641000 (CN); WEI, Tao, NEIJIANG, Sichuan 641000 (CN); DING, Zhao, NEIJIANG, Sichuan 641000 (CN)
(74) Representative: Casalonga
(86) International application number: PCT/CN2022/142626
(87) International publication number: WO 2023/125611

(57) **Abstract**

An antibody targeting CD3 or an antigen-binding fragment thereof, which has a moderate ability to activate T cells and can effectively reduce cell factor release. A multispecific antibody targeting CD3 and other antigens, such as tumor-associated antigens and/or immune checkpoint molecules, which can significantly reduce cytokine release while ensuring efficient and specific killing of target cells, and has a significantly improved safety. The present invention also relates to uses of the antibody targeting CD3 or the antigen-binding fragment thereof, and the multispecific antibody or a composition comprising same in disease treatment.

## Description

### Technical Field

The present disclosure relates to a CD3-targeting antibody or antigen-binding fragment thereof, which has an ability to moderately activate T cells and can effectively reduce cytokine release. The present disclosure also relates to a multispecific antibody targeting CD3 and other antigens (e.g., tumor-associated antigen and/or immune checkpoint molecule). The multispecific antibody can kill target cells efficiently and specifically while significantly reduce cytokine release at the same time, thereby having a significantly improved safety. The present disclosure also relates to the uses of the CD3-targeting antibody or antigen-binding fragment thereof, the multispecific antibody or a composition comprising them in the treatment of diseases.

### Background Art

CD3 is a protein complex and T cell co-receptor that is mainly expressed on T cells and involved in the activation of cytotoxic T cells (CD8+ naive T cells) and T helper cells (CD4+ naive T cells). CD3 consists of one CD3γ chain, one CD3δ chain and two CD3ε chains. These chains bind to T-cell receptors (TCR) and ζ chains (zeta chains) to generate activation signals in T lymphocytes. CD3 antibodies currently under clinical developments or on the market are mainly derived from humanized CD3 antibodies (OKT3/UCHT1/L2K/TR66, etc.). However, their affinity to CD3 is relatively high, which can easily lead to excessive activation of T cells and release of a large amount of cytokines, and thus cause cytokine storm syndrome that may have serious side effects in human and even threaten life in severe cases. To address the risk of cytokine storm syndrome (CRS), their dosages are generally very low, which results in a narrow therapeutic window and is detrimental to patient benefits.

Therefore, there is a need in the field to develop novel CD3 antibodies and bispecific antibodies to provide the ability of moderate T cells activation with reduced probability of cytokine storm syndrome, thereby improving safety and benefiting the treatment of diseases, especially the treatment of cancers.

### Contents of the present disclosure

The inventors of the present application have screened and obtained novel CD3 antibodies through extensive research. These antibodies have reduced cytokine release, indicating that they can moderately activate T cells. The inventors of the present application also have screened and obtained novel MSLN single-domain antibodies, which have good binding affinity to MSLN. Based on this, multispecific antibodies targeting CD3 and an additional antigen (e.g., MSLN, CD19, CD20, Trop2, Her2 or Caludin18.2) are further developed, which exhibit enhanced tumor cell localization and elicit effective activation of T cells, thereby specifically killing tumor cells with significantly reduced cytokine release, thus having significantly improved safety. The following aspects are thus provided.

In a first aspect, the present disclosure relates to an antibody or antigen-binding fragment thereof capable of specifically binding to CD3, the antibody or antigen-binding fragment thereof comprising a heavy chain variable region (VH) and a light chain variable region (VL), wherein, the VH comprises: an HCDR1 comprising with the sequence as set forth in SEQ ID NO: 107, an HCDR2 comprising the sequence as set forth in SEQ ID NO: 108, and an HCDR3 comprising the sequence as set forth in X₁X₂X₃X₄X₅X₆X₇X₈X₉X₁₀WX₁₁X₁₂X₁₃ (SEQ ID NO: 112); wherein X₁ is A, H or P; X₂ is A, E, G, H, K, Q or S; X₃ is D, N or R; X₄ is F or P; X₅ is G, K, L, P, Q, R, S, V, W or Y; X₆ is M, N, Q or R; X₇ is G, N, S or T; X₈ is A, Q, R or Y; X₉ is G, I or V; X₁₀ is N or S; X₁₁ is F or W; X₁₂ is A, E, K or Q; X₁₃ is H, L, M, S or Y; with the proviso that the HCDR3 is not set forth in SEQ ID NO: 90.

In a second aspect, the present disclosure relates to a single-domain antibody or antigen-binding fragment thereof capable of specifically binding to MSLN, the single-domain antibody or antigen-binding fragment thereof comprising:
(1) the following CDRs defined by the IMGT numbering system: a CDR1 comprising the sequence as set forth in SEQ ID NO: 150 or variant thereof, a CDR2 comprising the sequence as set forth in SEQ ID NO: 151 or variant thereof, and a CDR3 comprising the sequence as set forth in SEQ ID NO: 152 or variant thereof;
(2) the following CDRs defined by the Kabat numbering system: a CDR1 comprising the sequence as set forth in SEQ ID NO: 153 or variant thereof, a CDR2 comprising the sequence as set forth in SEQ ID NO: 154 or variant thereof, and a CDR3 comprising the sequence as set forth in SEQ ID NO: 155 or variant thereof;
(3) the following CDRs defined by the AbM numbering system: a CDR1 comprising the sequence as set forth in SEQ ID NO: 156 or variant thereof, a CDR2 comprising the sequence as set forth in SEQ ID NO: 157 or variant thereof, and a CDR3 comprising the sequence as set forth in SEQ ID NO: 155 or variant thereof;
(4) the following CDRs defined by the Chothia numbering system: a CDR1 comprising the sequence as set forth in SEQ ID NO: 158 or variant thereof, a CDR2 comprising the sequence as set forth in SEQ ID NO: 159 or variant thereof, and a CDR3 comprising the sequence as set forth in SEQ ID NO: 155 or variant thereof; or,
(5) the following CDRs defined by the Contact numbering system: a CDR1 comprising the sequence as set forth in SEQ ID NO: 160 or variant thereof, a CDR2 comprising the sequence as set forth in SEQ ID NO: 161 or variant thereof, and a CDR3 comprising the sequence as set forth in SEQ ID NO: 162 or variant thereof;
   wherein, the variant described in any one of (1) to (5) has a substitution, deletion or addition of one or several amino acids as compared to the sequence from which it is derived; preferably, the substitution is a conservative substitution;
   preferably, the single-domain antibody or antigen-binding fragment thereof comprises:
      (1) the following CDRs defined by the IMGT numbering system: a CDR1 comprising the sequence as set forth in SEQ ID NO: 150, a CDR2 comprising the sequence as set forth in SEQ ID NO: 151, and a CDR3 comprising the sequence as set forth in SEQ ID NO: 152;
      (2) the following CDRs defined by the Kabat numbering system: a CDR1 comprising the sequence as set forth in SEQ ID NO: 153, a CDR2 comprising the sequence as set forth in SEQ ID NO: 154, and a CDR3 comprising the sequence as set forth in SEQ ID NO: 155;
      (3) the following CDRs defined by the AbM numbering system: a CDR1 comprising the sequence as set forth in SEQ ID NO: 156, a CDR2 comprising the sequence as set forth in SEQ ID NO: 157, and a CDR3 comprising the sequence as set forth in SEQ ID NO: 155;
      (4) the following CDRs defined by the Chothia numbering system: a CDR1 comprising the sequence as set forth in SEQ ID NO: 158, a CDR2 comprising the sequence as set forth in SEQ ID NO: 159, and a CDR3 comprising the sequence as set forth in SEQ ID NO: 155; or,
      (5) the following CDRs defined by the Contact numbering system: a CDR1 comprising the sequence as set forth in SEQ ID NO: 160, a CDR2 comprising the sequence as set forth in SEQ ID NO: 161, and a CDR3 comprising the sequence as set forth in SEQ ID NO: 162.

In a third aspect, the present disclosure relates to a multispecific antibody, which comprises an CD3-targeting antigen-binding domain and at least one antigen-binding domain targeting other antigen, the CD3-targeting antigen-binding domain is selected from the antibody or antigen-binding fragment thereof according to the first aspect, and the other antigen is selected from tumor-associated antigen (TAA) and/or immune checkpoint molecule.

In a fourth aspect, the present disclosure relates to an isolated nucleic acid molecule, which encodes:
- the antibody or antigen-binding fragment thereof, or its heavy chain variable region and/or light chain variable region according to the first aspect;
- the single-domain antibody or antigen-binding fragment thereof according to the second aspect; or,
- the multispecific antibody, or its polypeptide chain according to the third aspect.

In a fifth aspect, the present disclosure relates to a vector, which comprises the nucleic acid molecule according to the fourth aspect.

In a sixth aspect, the present disclosure relates to a host cell, which comprises the nucleic acid molecule according to the fourth aspect or the vector according to the fifth aspect.

In a seventh aspect, the present disclosure relates to a pharmaceutical composition, which comprises the antibody or antigen-binding fragment thereof according to the first aspect, the single-domain antibody or antigen-binding fragment thereof according to the second aspect, the multispecific antibody according to the third aspect, the isolated nucleic acid molecule according to the fourth aspect, the vector according to the fifth aspect, or the host cell according to the sixth aspect, and a pharmaceutically acceptable carrier and/or excipient.

In an eighth aspect, the present disclosure relates to a use of the antibody or antigen-binding fragment thereof according to the first aspect, the single-domain antibody or antigen-binding fragment thereof according to the second aspect, the multispecific antibody according to the third aspect, the isolated nucleic acid molecule according to the fourth aspect, the vector according to the fifth aspect, the host cell according to the sixth aspect, or the pharmaceutical composition according to the seventh aspect for preventing and/or treating a disease, or in the manufacture of a medicament for preventing and/or treating a disease.

In a ninth aspect, the present disclosure relates to a method for preventing and/or treating a disease, which comprises administering to a subject in need thereof the antibody or antigen-binding fragment thereof according to the first aspect, the single-domain antibody or antigen-binding fragment thereof according to the second aspect, the multispecific antibody according to the third aspect, the isolated nucleic acid molecule according to the fourth aspect, the vector according to the fifth aspect, the host cell according to the sixth aspect, or the pharmaceutical composition according to the seventh aspect.

### Brief Description of the Drawings

FIG. 1 shows a schematic structural diagram of the CD3-MSLN bispecific antibody in Example 13.
FIG. 2 shows the determination measurement results of the binding activity of the CD3-MSLN bispecific antibody to MC38-MSLN cells in Example 14.
FIGS. 3A to 3D show the determination results of the dynamic affinity of the CD3-MSLN bispecific antibody to the human CD3εγ recombinant antigen in Example 14.
FIG. 4 shows the determination results of the dynamic affinity of the CD3-MSLN bispecific antibody to the MSLN recombinant antigen in Example 14.
FIGS. 5A to 5F show the detection results of the stability of Jurkat-TIGIT-luc cells in Example 15.
FIGS. 6A to 6F show the determination results of the activation of T cell activation signaling pathway by the CD3-MSLN bispecific antibody in Example 16.
FIGS. 7A to 7I show the determination results of TDCC mediated by the CD3-MSLN bispecific antibody in Example 17.
FIG. 8 shows a schematic structural diagram of the CD3-CD19 bispecific antibody in Example 18.
FIG. 9 shows the determination results of TDCC mediated by the CD3-CD19 bispecific antibody in Example 18.
FIG. 10 shows the determination results of TDCC mediated by the CD3-CD20 bispecific antibody in Example 19.
FIG. 11 shows the determination results of TDCC mediated by the CD3-trop2 bispecific antibody in Example 20.
FIG. 12 shows the determination results of TDCC mediated by the CD3-Her2 bispecific antibody in Example 21.
FIG. 13 shows the determination results of TDCC mediated by the CD3-Caludin18.2 bispecific antibody in Example 22.
FIGS. 14A to 14I show the detection results of IL-2 release levels mediated by the CD3-MSLN bispecific antibody in Example 23.
FIGS. 15A to 15I show the detection results of INF-γ release levels mediated by the CD3-MSLN bispecific antibody in Example 23.

### Specific Models for Carrying Out the present disclosure

The present disclosure provides novel CD3 antibodies, and specifically provides the following aspects.

### CD3 antibody

In a first aspect, the present disclosure relates to an antibody or antigen-binding fragment thereof capable of specifically binding to CD3, the antibody or antigen-binding fragment thereof comprising a heavy chain variable region (VH) and a light chain variable region (VL), wherein, the VH comprises: an HCDR1 comprising the sequence as set forth in SEQ ID NO: 107, an HCDR2 comprising the sequence as set forth in SEQ ID NO: 108, and an HCDR3 comprising the sequence as set forth in X₁X₂X₃X₄X₅X₆X₇X₈X₉X₁₀WX₁₁X₁₂X₁₃ (SEQ ID NO: 112); wherein X₁ is A, H or P; X₂ is A, E, G, H, K, Q or S; X₃ is D, N or R; X₄ is F or P; X₅ is G, K, L, P, Q, R, S, V, W or Y; X₆ is M, N, Q or R; X₇ is G, N, S or T; X₈ is A, Q, R or Y; X₉ is G, I or V; X₁₀ is N or S; X₁₁ is F or W; X₁₂ is A, E, K or Q; X₁₃ is H, L, M, S or Y; with the proviso that the HCDR3 is not set forth in SEQ ID NO: 90.

In certain embodiments, the VL comprises: an LCDR1 comprising the sequence as set forth in SEQ ID NO: 137, an LCDR2 comprising the sequence as set forth in SEQ ID NO: 138, and an LCDR3 comprising the sequence as set forth in SEQ ID NO: 139.

In certain embodiments, the HCDR3 is not represented by any one of SEQ ID NOs: 60, 64, 77, and 89.

In certain embodiments, the HCDR3 comprises: the sequence as set forth in X₁X₂X₃FX₄NX₅YX₆SWFAX₇ (SEQ ID NO: 148), wherein X₁ is H or P; X₂ is G, E or A; X₃ is N or R; X₄ is G, K, S or P; X₅ is T, S or N; X₆ is V or G; X₇ is M, Y, S or L. In certain embodiments, the HCDR3 comprises the sequence as set forth in any one of SEQ ID NOs: 56, 61, 69, 79, 82, 87.

In certain embodiments, the HCDR3 comprises:
(1) the sequence as set forth in HXzNFGNSYVSWFAY (SEQ ID NO: 113), wherein X₂ is A, E, H, K, Q or S, preferably H;
(2) the sequence as set forth in HGNFX₅NSYVSWFAY (SEQ ID NO: 114), wherein X₅ is K, L, P, Q, R, S, V, W or Y, preferably K;
(3) the sequence as set forth in HGNFGNSX₈VSWFAY (SEQ ID NO: 115), wherein X₈ is A, Q or R, preferably R; or,
(4) the sequence as set forth in HGNFGNSYVSWFX₁₂Y (SEQ ID NO: 116), wherein X₁₂ is E, K or Q.

In certain embodiments, the HCDR3 comprises the sequence as set forth in any one of SEQ ID NOs: 75, 53, 72, 51, 57, 64.

In certain embodiments, the HCDR3 comprises:
(1) the sequence as set forth X₁X₂NFGNSYVSWFAY (SEQ ID NO: 117), wherein X₁ is A or P, preferably A; X₂ is A, E, H, K, Q or S, preferably K; preferably, X₁ is A, and X₂ is K;
(2) the sequence as set forth in X₁GNFGNSYVX₁₀WFAY (SEQ ID NO: 118), wherein X₁ is A or P, preferably A; X₁₀ is N; preferably, X₁ is A, and X₁₀ is N; or,
(3) the sequence as set forth in X₁GNFGNSYVSWFX₁₂Y (SEQ ID NO: 119), wherein X₁ is A or P, preferably A; X₁₂ is E, K or Q, preferably E or Q; preferably, X₁ is A, and X₁₂ is E or Q.

In certain embodiments, the HCDR3 comprises the sequence as set forth in any one of SEQ ID NOs: 50, 66, 55, 85.

In certain embodiments, the HCDR3 comprises:
(1) the sequence as set forth in X₁X₂NFGNSYVSWFAY (SEQ ID NO: 117), wherein X₁ is A or P, preferably A; X₂ is A, E, H, K, Q or S, preferably K; preferably, X₁ is A, and X₂ is K;
(2) the sequence as set forth in HX₂X₃FGNSYVSWFAY (SEQ ID NO: 120), wherein X₂ is A, E, H, K, Q or S, preferably E; X₃ is D or R, preferably R; preferably, X₂ is E, and X₃ is R;
(3) the sequence as set forth in HX₂NFX₅NSYVSWFAY (SEQ ID NO: 121), wherein X₂ is A, E, H, K, Q or S, preferably A or S; X₅ is K, L, P, Q, R, S, V, W or Y, preferably L, P, S, Q, V or R; preferably, X₂ is A or S, and X₅ is L, P, S, Q, V or R; preferably, X₂ and X₅ are A/L, S/P, S/L, A/S, S/Q, S/V or S/R, respectively;
(4) the sequence as set forth in HX₂NFGX₆SYVSWFAY (SEQ ID NO: 122), wherein X₂ is A, E, H, K, Q or S, preferably S; X₆ is M, Q or R, preferably R; preferably, X₂ is S, and X₆ is R;
(5) the sequence as set forth in HX₂NFGNX₇YVSWFAY (SEQ ID NO: 123), wherein X₂ is A, E, H, K, Q or S, preferably S; X₇ is G, N or T, preferably T or G; preferably, X₂ is S, X₇ is T or G;
(6) the sequence as set forth in HX₂NFGNSX₈VSWFAY (SEQ ID NO: 124), wherein X₂ is A, E, H, K, Q or S, preferably S; Xs is A, Q or R, preferably R; preferably, X₂ is S, and Xs is R;
(7) the sequence as set forth in HXzNFGNSYVSWXi iAY (SEQ ID NO: 125), wherein X₂ is A, E, H, K, Q or S, preferably S; X₁₁ is W; preferably, X₂ is S, and X₁₁ is W;
(8) the sequence as set forth in HX₂NFGNSYVSWFX₁₂Y (SEQ ID NO: 126), wherein X₂ is A, E, H, K, Q or S, preferably S; X₁₂ is E, K or Q, preferably E or Q; preferably, X₂ is S or Q, and X₁₂ is E or Q; preferably, X₂ and X₁₂ are S/E, Q/Q or S/Q, respectively; or,
(9) the sequence as set forth in HX₂NFGNSYVSWFAX₁₃ (SEQ ID NO: 127), wherein X₂ is A, E, H, K, Q or S, preferably A; X₁₃ is H, L, M or S, preferably S; preferably, X₂ is A, and X₁₃ is S.

In certain embodiments, the HCDR3 comprises the sequence as set forth in any one of SEQ ID NOs: 50, 69, 49, 58, 59, 60, 70, 82, 86, 71, 68, 77, 78, 81, 62, 84, 89, 74.

In certain embodiments, the HCDR3 comprises:
(1) the sequence as set forth in HX₂NFX₅NSYVSWFAY (SEQ ID NO: 121), wherein X₂ is A, E, H, K, Q or S, preferably A or S; X₅ is K, L, P, Q, R, S, V, W or Y, preferably L, P, S, Q, V or R; preferably, X₂ is A or S, and X₅ is L, P, S, Q, V or R; preferably, X₂ and X₅ are A/L, S/P, S/L, A/S, S/Q, S/V or S/R, respectively;
(2) the sequence as set forth in HGNFX₅X₆SYVSWFAY (SEQ ID NO: 129), wherein X₅ is K, L, P, Q, R, S, V, W or Y, preferably P or K; X₆ is M, Q or R, preferably M or Q; preferably, X₅ is P or K, and X₆ is M or Q; preferably, X₅ and X₆ are P/M or K/Q, respectively;
(3) the sequence as set forth in HGNFX₅NSYX₉SWFAY (SEQ ID NO: 130), wherein X₅ is K, L, P, Q, R, S, V, W or Y, preferably K or Q; X₉ is G or I; preferably, X₅ is K or Q, and X₉ is G or I; preferably, X₅ and X₉ are K/G or Q/I, respectively;
(4) the sequence as set forth in HGNFXsNSYVSWFXizY (SEQ ID NO: 131), wherein X₅ is K, L, P, Q, R, S, V, W or Y, preferably Q or L; X₁₂ is E, K or Q, preferably Q; preferably, X₅ is Q or L, and X₁₂ is Q; or,
(5) the sequence as set forth in HGNFX₅NSYVSWFAX₁₃ (SEQ ID NO: 132), wherein X₅ is K, L, P, Q, R, S, V, W or Y, preferably Y or W; X₁₃ is H, L, M or S, preferably H or S; preferably, X₅ is Y or W, and X₁₃ is H or S; preferably, X₅ and X₁₃ are Y/H or W/S, respectively.

In certain embodiments, the HCDR3 comprises the sequence as set forth in any one of SEQ ID NOs: 49, 58, 59, 60, 70, 82, 86, 76, 80, 61, 88, 52, 83, 54, 65.

In certain embodiments, the HCDR3 comprises:
(1) the sequence as set forth in XiGNFGNSYVSWFXizY (SEQ ID NO: 119), wherein X₁ is A or P, preferably A; X₁₂ is E, K or Q, preferably Q; preferably, X₁ is A, and X₁₂ is Q or E;
(2) the sequence as set forth in HX₂NFGNSYVSWFX₁₂Y (SEQ ID NO: 126), wherein X₂ is A, E, H, K, Q or S, preferably S; X₁₂ is E, K or Q, preferably E or Q; preferably, X₂ is S, and X₁₂ is E or Q; preferably, X₂ and X₁₂ are SZE, Q/Q or S/Q, respectively;
(3) the sequence as set forth in HGNFX₅NSYVSWFX₁₂Y (SEQ ID NO: 131), wherein X₅ is K, L, P, Q, R, S, V, W or Y, preferably Q or L; X₁₂ is E, K or Q, preferably Q; preferably, X₅ is Q or L, and X₁₂ is Q; or,
(4) the sequence as set forth in HGNFGNSX₈VSWFX₁₂Y (SEQ ID NO: 134), wherein X₈ is A, Q or R, preferably A or Q; X₁₂ is E, K or Q, preferably Q; preferably, X₈ is A or Q, and X₁₂ is Q.

In certain embodiments, the HCDR3 comprises the sequence as set forth in any one of SEQ ID NOs: 55, 85, 62, 84, 89, 52, 83, 67, 73.

In certain embodiments, the HCDR3 comprises:
(1) the sequence as set forth in HX₂NFGNSYVSWFAX₁₃ (SEQ ID NO: 127), wherein X₂ is A, E, H, K, Q or S, preferably A; X₁₃ is H, L, M or S, preferably S; preferably, X₂ is A, and X₁₃ is S;
(2) the sequence as set forth in HGNFX₅NSYVSWFAX₁₃ (SEQ ID NO: 132), wherein X₅ is K, L, P, Q, R, S, V, W or Y, preferably Y or W; X₁₃ is H, L, M or S, preferably H or S; preferably, X₅ is Y or W, and X₁₃ is H or S; preferably, X₅ and X₁₃ are Y/H or W/S, respectively; or,
(3) the sequence as set forth in HGNFGNX₇YVSWFAX₁₃ (SEQ ID NO: 133), wherein X₇ is G, N or T, preferably T or N; X₁₃ is H, L, M or S, preferably M or S; preferably, X₇ is T or N, and X₁₃ is M or S; preferably, X₇ and X₁₃ are T/M or N/S, respectively.

In certain embodiments, the HCDR3 comprises the sequence as set forth in any one of SEQ ID NOs: 74, 54, 65, 56, and 79.

In certain embodiments, the HCDR3 comprises:
(1) the sequence as set forth in HX₂X₃FGNSYVSWFAY (SEQ ID NO: 120), wherein X₂ is A, E, H, K, Q or S, preferably E; X₃ is D or R, preferably R; preferably, X₂ is E, and X₃ is R; or,
(2) the sequence as set forth in HGX₃X₄GNSYVSWFAY (SEQ ID NO: 128), wherein X₃ is D or R, preferably D; X₄ is P; preferably, X₃ is D, and X₄ is P.

In certain embodiments, the HCDR3 comprises the sequence as set forth in any one of SEQ ID NOs: 69, 63.

In certain embodiments, the HCDR3 comprises: the sequence as set forth in X₁GNFX₅NSYVSWFAX₁₃ (SEQ ID NO: 135), wherein X₁ is A or P, preferably P; X₅ is K, L, P, Q, R, S, V, W or Y, preferably P; X₁₃ is H, L, M or S, preferably L; preferably, X₁ is P, X₅ is P, and X₁₃ is L.

In certain embodiments, the HCDR3 comprises the sequence as set forth in SEQ ID NO: 87.

In certain embodiments, the VH comprises: an HCDR1 comprising the sequence as set forth in SEQ ID NO: 107, an HCDR2 comprising the sequence as set forth in SEQ ID NO: 108, and an HCDR3 comprising the sequence as set forth in any one of SEQ ID NOs: 49-89; and, the VL comprises: an LCDR1 comprising the sequence as set forth in SEQ ID NO: 137, an LCDR2 comprising the sequence as set forth in SEQ ID NO: 138, and an LCDR3 comprising the sequence as set forth in SEQ ID NO: 139. Preferably, the proviso is that the HCDR3 is not represented by any one of SEQ ID NOs: 60, 64, 77, and 89.

In certain embodiments, the antibody or antigen-binding fragment thereof further comprises a framework region derived from a human immunoglobulin.

In certain embodiments, the antibody or antigen-binding fragment thereof comprises a framework region contained in an amino acid sequence encoded by a gene derived from a human germline. In certain embodiments, the antibody or antigen-binding fragment thereof comprises a heavy chain framework region contained in an amino acid sequence encoded by a gene derived from a human heavy chain germline, and/or a light chain framework region contained in an amino acid sequence encoded by a gene derived from a human light chain germline.

In certain embodiments, the VH comprises an HFR1, an HFR2, an HFR3 and an HFR4, wherein:
the HFR1 comprises the sequence as set forth in SEQ ID NO: 109;
the HFR2 comprises the sequence as set forth in SEQ ID NO: 110;
the HFR3 comprises the sequence as set forth in VKX₁RFTISRDDSKSX₂LYLQMNX₃LK TEDTAX₄YYCVR (SEQ ID NO: 136); wherein, X₁ is G or D, X₂ is I or S, X₃ is N or S, and X₄ is M or V;
the HFR4 comprises the sequence as set forth in SEQ ID NO: 111.

In certain embodiments, the HFR3 comprises the sequence as set forth in any one of SEQ ID NOs: 91-106.

In certain embodiments, the VH comprises: the amino acid sequence as set forth in any one of SEQ ID NOs: 1-46 or variant thereof, wherein the variant has a substitution, deletion or addition of one or more amino acids, or has a sequence identity of at least 90%, at least 91%, at least 92%, at least 93%, at least 94%, at least 95%, at least 96%, at least 97%, at least 98%, at least 99%, or 100%, as compared to the sequence from which it is derived.

In certain embodiments, the VH comprises: the amino acid sequence as set forth in any one of SEQ ID NOs: 8, 13, 22, 33, 37, 43 or variant thereof, wherein the variant has a substitution, deletion or addition of one or more amino acids, or has a sequence identity of at least 90%, at least 91%, at least 92%, at least 93%, at least 94%, at least 95%, at least 96%, at least 97%, at least 98%, at least 99%, or 100%, as compared to the sequence from which it is derived.

In certain embodiments, the VH comprises: the amino acid sequence as set forth in any one of SEQ ID NOs: 3, 5, 9, 16, 20, 25, 28 or variant thereof, wherein the variant has a substitution, deletion or addition of one or more amino acids, or has a sequence identity of at least 90%, at least 91%, at least 92%, at least 93%, at least 94%, at least 95%, at least 96%, at least 97%, at least 98%, at least 99%, or 100%, as compared to the sequence from which it is derived.

In certain embodiments, the VH comprises: the amino acid sequence as set forth in any one of SEQ ID NOs: 2, 7, 18, 40 or variant thereof, wherein the variant has a substitution, deletion or addition of one or more amino acids, or has a sequence identity of at least 90%, at least 91%, at least 92%, at least 93%, at least 94%, at least 95%, at least 96%, at least 97%, at least 98%, at least 99%, or 100%, as compared to the sequence from which it is derived.

In certain embodiments, the VH comprises: the amino acid sequence as set forth in any one of SEQ ID NOs: 2, 22, 1, 10, 11, 12, 23, 36, 37, 41, 24, 21, 30, 32, 35, 14, 31, 39, 45, 27, 46 or variant thereof, wherein the variant has a substitution, deletion or addition of one or more amino acids, or has a sequence identity of at least 90%, at least 91%, at least 92%, at least 93%, at least 94%, at least 95%, at least 96%, at least 97%, at least 98%, at least 99%, or 100%, as compared to the sequence from which it is derived.

In certain embodiments, the VH comprises: the amino acid sequence as set forth in any one of SEQ ID NOs: 1, 10, 11, 12, 23, 36, 37, 41, 29, 34, 13, 44, 4, 38, 42, 6, 17 or variant thereof, wherein the variant has a substitution, deletion or addition of one or more amino acids, or has a sequence identity of at least 90%, at least 91%, at least 92%, at least 93%, at least 94%, at least 95%, at least 96%, at least 97%, at least 98%, at least 99%, or 100%, as compared to the sequence from which it is derived.

In certain embodiments, the VH comprises: the amino acid sequence as set forth in any one of SEQ ID NOs: 7, 40, 14, 31, 39, 45, 4, 38, 42, 19, 26 or variant thereof, wherein the variant has a substitution, deletion or addition of one or more amino acids, or has a sequence identity of at least 90%, at least 91%, at least 92%, at least 93%, at least 94%, at least 95%, at least 96%, at least 97%, at least 98%, at least 99%, or 100%, as compared to the sequence from which it is derived.

In certain embodiments, the VH comprises: the amino acid sequence as set forth in any one of SEQ ID NOs: 27, 46, 6, 17, 8, 33 or variant thereof, wherein the variant has a substitution, deletion or addition of one or more amino acids, or has a sequence identity of at least 90%, at least 91%, at least 92%, at least 93%, at least 94%, at least 95%, at least 96%, at least 97%, at least 98%, at least 99%, or 100%, as compared to the sequence from which it is derived.

In certain embodiments, the VH comprises: the amino acid sequence as set forth in SEQ ID NO: 22 or 15 or variant thereof, wherein the variant has a substitution, deletion or addition of one or more amino acids, or has a sequence identity of at least 90%, at least 91%, at least 92%, at least 93%, at least 94%, at least 95%, at least 96%, at least 97%, at least 98%, at least 99%, or 100%, as compared to the sequence from which it is derived.

In certain embodiments, the VH comprises: the amino acid sequence as set forth in SEQ ID NO: 43 or variant thereof, wherein the variant has a substitution, deletion or addition of one or more amino acids, or has a sequence identity of at least 90%, at least 91%, at least 92%, at least 93%, at least 94%, at least 95%, at least 96%, at least 97%, at least 98%, at least 99%, or 100%, as compared to the sequence from which it is derived.

In certain embodiments, the VH comprises: the amino acid sequence as set forth in any one of SEQ ID NOs: 1-11,13-15, 17-19, 21-29, 31-35, 37-44, 46 or variant thereof, wherein the variant has a substitution, deletion or addition of one or more amino acids, or has a sequence identity of at least 90%, at least 91%, at least 92%, at least 93%, at least 94%, at least 95%, at least 96%, at least 97%, at least 98%, at least 99%, or 100%, as compared to the sequence from which it is derived.

In certain embodiments, the VL comprises an LFR1, an LFR2, an LFR3 and an LFR4, wherein:
the LFR1 comprises the sequence as set forth in SEQ ID NO: 140;
the LFR2 comprises the sequence as set forth in WX₁QQTPGQAX₂RX₃LIX₄ (SEQ ID NO: 144); wherein, X₁ is V or Y, X₂ is F or P, X₃ is G or T, and X₄ is G or Y;
the LFR3 comprises the sequence as set forth in GVPARFSGSX₄X₅GX₆KAALTITGAQA DDESX₇YFCA (SEQ ID NO: 147); wherein, X₄ is L or I, X₅ is L or I, X₆ is D or N, and X₇ is I or D;
the LFR4 comprises the sequence as set forth in SEQ ID NO: 141.

In certain embodiments, the LFR2 comprises the sequence as set forth in SEQ ID NO: 142 or 143.

In certain embodiments, the LFR3 comprises the sequence as set forth in SEQ ID NO: 145 or 146.

In certain embodiments, the VL comprises: an LFR1 as set forth in SEQ ID NO: 140, an LFR2 as set forth in SEQ ID NO: 142, an LFR3 as set forth in SEQ ID NO: 145, and an LFR4 as set forth in SEQ ID NO: 141.

In certain embodiments, the VL comprises: an LFR1 as set forth in SEQ ID NO: 140, an LFR2 as set forth in SEQ ID NO: 143, an LFR3 as set forth in SEQ ID NO: 146, and an LFR4 as set forth in SEQ ID NO: 141.

In certain embodiments, the VL comprises: the amino acid sequence as set forth in SEQ ID NO: 47 or 48 or variant thereof, wherein the variant has a substitution, deletion or addition of one or more amino acids, or has a sequence identity of at least 90%, at least 91%, at least 92%, at least 93%, at least 94%, at least 95%, at least 96%, at least 97%, at least 98%, at least 99%, or 100%, as compared to the sequence from which it is derived.

In certain embodiments, the antibody or antigen-binding fragment thereof comprises: a VH comprising the sequence as set forth in any one of SEQ ID NOs: 1-46 or variant thereof, and a VL comprising the sequence as set forth in SEQ ID NO: 47 or variant thereof; wherein the variant has a substitution, deletion or addition of one or more amino acids, or has a sequence identity of at least 90%, at least 91%, at least 92%, at least 93%, at least 94%, at least 95%, at least 96%, at least 97%, at least 98%, at least 99%, or 100%, as compared to the sequence from which it is derived.

In certain embodiments, the antibody or antigen-binding fragment thereof comprises: a VH comprising the sequence as set forth in any one of SEQ ID NOs: 1-46 or variant thereof, and a VL comprising the sequence as set forth in SEQ ID NO: 48 or variant thereof; wherein the variant has a substitution, deletion or addition of one or more amino acids, or has a sequence identity of at least 90%, at least 91%, at least 92%, at least 93%, at least 94%, at least 95%, at least 96%, at least 97%, at least 98%, at least 99%, or 100%, as compared to the sequence from which it is derived.

In certain embodiments, the antibody or antigen-binding fragment thereof comprises: a VH comprising the sequence as set forth in any one of SEQ ID NOs: 1-11, 13-15, 17-19, 21-29, 31-35, 37-44, 46 or variant thereof, and a VL comprising the sequence as set forth in SEQ ID NO: 47 or variant thereof; wherein the variant has a substitution, deletion or addition of one or more amino acids, or has a sequence identity of at least 90%, at least 91%, at least 92%, at least 93%, at least 94%, at least 95%, at least 96%, at least 97%, at least 98%, at least 99%, or 100%, as compared to the sequence from which it is derived.

In certain embodiments, the antibody or antigen-binding fragment thereof comprises: a VH comprising the sequence as set forth in any one of SEQ ID NOs: 1-11, 13-15, 17-19, 21-29, 31-35, 37-44, 46 or variant thereof, and a VL comprising the sequence as set forth in SEQ ID NO: 48 or variant thereof; wherein the variant has a substitution, deletion or addition of one or more amino acids, or has a sequence identity of at least 90%, at least 91%, at least 92%, at least 93%, at least 94%, at least 95%, at least 96%, at least 97%, at least 98%, at least 99%, or 100%, as compared to the sequence from which it is derived.

In certain embodiments, the antibody or antigen-binding fragment thereof comprises: a VH comprising the sequence as set forth in any one of SEQ ID NOs: 8, 13, 22, 33, 37, 43 or variant thereof, and a VL comprising the sequence as set forth in SEQ ID NO: 47 or variant thereof wherein the variant has a substitution, deletion or addition of one or more amino acids, or has a sequence identity of at least 90%, at least 91%, at least 92%, at least 93%, at least 94%, at least 95%, at least 96%, at least 97%, at least 98%, at least 99%, or 100%, as compared to the sequence from which it is derived.

In certain embodiments, the antibody or antigen-binding fragment thereof comprises: a VH comprising the sequence as set forth in any one of SEQ ID NOs: 8, 13, 22, 33, 37, 43 or variant thereof, and a VL comprising the sequence as set forth in SEQ ID NO: 48 or variant thereof wherein the variant has a substitution, deletion or addition of one or more amino acids, or has a sequence identity of at least 90%, at least 91%, at least 92%, at least 93%, at least 94%, at least 95%, at least 96%, at least 97%, at least 98%, at least 99%, or 100%, as compared to the sequence from which it is derived.

In certain embodiments, the VH comprises: HCDR1 to HCDR3 and HFR1 to HFR4, which have the following characteristics:
Item 1: the HCDR1 comprises the sequence as set forth in SEQ ID NO: 107;
Item 2: the HCDR2 comprises the sequence as set forth in SEQ ID NO: 108;
Item 3: the HCDR3 comprises a sequence selected from the group consisting of:
   (3-1) the sequence as set forth in X₁X₂NFGNSYVSWFAY (SEQ ID NO: 117), wherein X₁ is A or P, preferably A; X₂ is A, E, H, K, Q or S, preferably K; preferably, X₁ is A, and X₂ is K;
   (3-2) the sequence as set forth in HX₂X₃FGNSYVSWFAY (SEQ ID NO: 120), wherein X₂ is A, E, H, K, Q or S, preferably E; X₃ is D or R, preferably R; preferably, X₂ is E, and X₃ is R;
   (3-3) the sequence as set forth in HX₂NFX₅NSYVSWFAY (SEQ ID NO: 121), wherein X₂ is A, E, H, K, Q or S, preferably A or S; X₅ is K, L, P, Q, R, S, V, W or Y, preferably L, P, S, Q, V or R; preferably, X₂ is A or S, and X₅ is L, P, S, Q, V or R; preferably, X₂ and X₅ are A/L, S/P, S/L, A/S, S/Q, S/V or S/R, respectively;
   (3-4) the sequence as set forth in HX₂NFGX₆SYVSWFAY (SEQ ID NO: 122), wherein X₂ is A, E, H, K, Q or S, preferably S; X₆ is M, Q or R, preferably R; preferably, X₂ is S, and X₆ is R;
   (3-5) the sequence as set forth in HX₂NFGNX₇YVSWFAY (SEQ ID NO: 123), wherein X₂ is A, E, H, K, Q or S, preferably S; X₇ is G, N or T, preferably T or G; preferably, X₂ is S, and X₇ is T or G;
   (3-6) the sequence as set forth in HX₂NFGNSX₈VSWFAY (SEQ ID NO: 124), wherein X₂ is A, E, H, K, Q or S, preferably S; X₈ is A, Q or R, preferably R; preferably, X₂ is S, and X₈ is R;
   (3-7) the sequence as set forth in HX₂NFGNSYVSWX₁₁AY (SEQ ID NO: 125), wherein X₂ is A, E, H, K, Q or S, preferably S; X₁₁ is W; preferably, X₂ is S, and X₁₁ for W;
   (3-8) the sequence as set forth in HX₂NFGNSYVSWFX₁₂Y (SEQ ID NO: 126), wherein X₂ is A, E, H, K, Q or S, preferably S; X₁₂ is E, K or Q, preferably E or Q; preferably, X₂ is S, X₁₂ is E or Q; preferably, X₂ and X₁₂ are S/E, Q/Q or S/Q, respectively; and,
   (3-9) the sequence as set forth in HX₂NFGNSYVSWFAX₁₃ (SEQ ID NO: 127), wherein X₂ is A, E, H, K, Q or S, preferably A; X₁₃ is H, L, M or S, preferably S; preferably, X₂ is A, and X₁₃ is S;
Item 4: the HFR1 comprises the sequence as set forth in SEQ ID NO: 109;
Item 5: the HFR2 comprises the sequence as set forth in SEQ ID NO: 110;
Item 6: the HFR3 comprises the sequence as set forth in VKX₁RFTISRDDSKSX₂LYL QMNX₃LKTEDTAX₄YYCVR (SEQ ID NO: 136); wherein, X₁ is G or D, X₂ is I or S, X₃ is N or S, and X₄ is M or V;
Item 7: the HFR4 comprises the sequence as set forth in SEQ ID NO: 111;
preferably, the VL has the following characteristics: Item 8: the VL comprises the sequence as set forth in SEQ ID NO: 47; or Item 9: the VL comprises the sequence as set forth in SEQ ID NO: 48.

In some embodiments, the VH has the following characteristics: Item 1, Item 2, Item 3, Item 4, Item 5, Item 6', Item 7, wherein Item 6': the HFR3 comprises the sequence as set forth in any one of SEQ ID NOs: 91-106. The VL preferably has the following characteristics: Item 8 or Item 9.

In some embodiments, the VH has the following characteristics: Item 1, Item 2, Item 3', Item 4, Item 5, Item 6, Item 7, wherein Item 3': the HCDR3 comprises the sequence as set forth in any one of SEQ ID NOs: 50, 69, 49, 58, 59, 60, 70, 82, 86, 71, 68, 77, 78, 81, 62, 84, 89, and 74. The VL preferably has the following characteristics: Item 8 or Item 9.

In some embodiments, the VH has the following characteristics: Item 1, Item 2, Item 3', Item 4, Item 5, Item 6', and Item 7. The VL preferably has the following characteristics: Item 8 or Item 9.

In certain embodiments, the VH comprises: the amino acid sequence as set forth in any one of SEQ ID NOs: 2, 22, 1, 10, 11, 12, 23, 36, 37, 41, 24, 21, 30, 32, 35, 14, 31, 39, 45, 27, and 46. The VL preferably comprises the sequence as set forth in SEQ ID NO: 47 or 48, such as the sequence as set forth in SEQ ID NO: 47.

In certain embodiments, the VH comprises: HCDR1 to HCDR3 and HFR1 to HFR4, which have the following characteristics:
Item 11: the HCDR1 comprises the sequence as set forth in SEQ ID NO: 107;
Item 12: the HCDR2 comprises the sequence as set forth in SEQ ID NO: 108;
Item 13: the HCDR3 comprises a sequence selected from the group consisting of:
   (13-1) the sequence as set forth in HX₂NFX₅NSYVSWFAY (SEQ ID NO: 121), wherein X₂ is A, E, H, K, Q or S, preferably A or S; X₅ is K, L, P, Q, R, S, V, W or Y, preferably L, P, S, Q, V or R; preferably, X₂ is A or S, and X₅ is L, P, S, Q, V or R; preferably, X₂ and X₅ are A/L, S/P, S/L, A/S, S/Q, S/V or S/R, respectively;
   (13-2) the sequence as set forth in HGNFX₅X₆SYVSWFAY (SEQ ID NO: 129), wherein X₅ is K, L, P, Q, R, S, V, W or Y, preferably P or K; X₆ is M, Q or R, preferably M or Q; preferably, X₅ is P or K, and X₆ is M or Q; preferably, X₅ and X₆ are P/M or K/Q, respectively;
   (13-3) the sequence as set forth in HGNFX₅NSYX₉SWFAY (SEQ ID NO: 130), wherein X₅ is K, L, P, Q, R, S, V, W or Y, preferably K or Q; X₉ is G or I; preferably, X₅ is K or Q, and X₉ is G or I; preferably, X₅ and X₉ are K/G or Q/I, respectively;
   (13-4) the sequence as set forth in HGNFX₅NSYVSWFX₁₂Y (SEQ ID NO: 131), wherein X₅ is K, L, P, Q, R, S, V, W or Y, preferably Q or L; X₁₂ is E, K or Q, preferably Q; preferably, X₅ is Q or L, and X₁₂ is Q; and,
   (13-5) the sequence as set forth in HGNFX₅NSYVSWFAX₁₃ (SEQ ID NO: 132), wherein X₅ is K, L, P, Q, R, S, V, W or Y, preferably Y or W; X₁₃ is H, L, M or S, preferably H or S; preferably, X₅ is Y or W, and X₁₃ is H or S; preferably, X₅ and X₁₃ are Y/H or W/S, respectively;
Item 14: the HFR1 comprises the sequence as set forth in SEQ ID NO: 109;
Item 15: the HFR2 comprises the sequence as set forth in SEQ ID NO: 110;
Item 16: the HFR3 comprises the sequence as set forth in VKX₁RFTISRDDSKSX₂L YLQMNX₃LKTEDTAX₄YYCVR (SEQ ID NO: 136); wherein, X₁ is G or D, X₂ is I or S, X₃ is N or S, and X₄ is M or V;
Item 17: the HFR4 comprises the sequence as set forth in SEQ ID NO: 111;

Preferably, the VL has the following characteristics: Item 18: the VL comprises the sequence as set forth in SEQ ID NO: 47; or Item 19: the VL comprises the sequence as set forth in SEQ ID NO: 48.

In some embodiments, the VH has the following characteristics: Item 11, Item 12, Item 13, Item 14, Item 15, Item 16', Item 17, wherein, Item 16': the HFR3 comprises the sequence as set forth in any one of SEQ ID NOs: 91-106. The VL preferably has the following characteristics: Item 18 or Item 19.

In some embodiments, the VH has the following characteristics: Item 11, Item 12, Item 13', Item 14, Item 15, Item 16, Item 17, wherein Item 13': the HCDR3 comprises the sequence as set forth in any one of SEQ ID NOs: 49, 58, 59, 60, 70, 82, 86, 76, 80, 61, 88, 52, 83, 54, and 65. The VL preferably has the following characteristics: Item 18 or Item 19.

In some embodiments, the VH has the following characteristics: Item 11, Item 12, Item 13', Item 14, Item 15, Item 16', Item 17. The VL preferably has the following characteristics: Item 18 or Item 19.

In certain embodiments, the VH comprises: the amino acid sequence as set forth in any one of SEQ ID NOs: 1, 10, 11, 12, 23, 36, 37, 41, 29, 34, 13, 44, 4, 38, 42, 6, 17. The VL preferably comprises the sequence as set forth in SEQ ID NO: 47 or 48, for example the sequence as set forth in SEQ ID NO: 47.

In certain embodiments, the antibody or antigen-binding fragment thereof further comprises a constant region derived from a human immunoglobulin.

In certain embodiments, the heavy chain of the antibody or antigen-binding fragment thereof comprises a heavy chain constant region derived from a human immunoglobulin. In certain embodiments, the heavy chain constant region is an IgG heavy chain constant region, such as IgG1, IgG2, IgG3 or IgG4 heavy chain constant region.

In certain embodiments, the light chain of the antibody or antigen-binding fragment thereof comprises a light chain constant region derived from a human immunoglobulin. In certain embodiments, the light chain constant region is a κ light chain constant region. In certain embodiments, the antibody or antigen-binding fragment thereof comprises the light chain constant region (CL) as set forth in SEQ ID NO: 165.

In certain embodiments, the Fc domain comprised by the antibody or antigen-binding fragment thereof is a native Fc region, which comprises an amino acid sequence identical to the amino acid sequence of an Fc region found in nature. The native Fc region can have an effector function. Exemplary "effector function" comprises binding to Fc receptor; Clq binding and complement-dependent cytotoxicity (CDC); antibody-dependent cell-mediated cytotoxicity (ADCC); antibody-dependent cellular phagocytosis (ADCP); downregulation of cell surface receptor (e.g., B cell receptor); and B cell activation, etc.

In certain embodiments, the Fc domain comprised by the antibody or antigen-binding fragment thereof may also be a variant Fc region, which may comprise a mutation or chemical modification of one or more (e.g., 1 to 10, for example, 1 to 5) amino acids as compared to the native Fc region so as to alter one or more of the following properties of the antibody of the present disclosure: Fc receptor binding, antibody glycosylation, number of cysteine residues, effector cell function or complement function, etc. The functional alteration can be made by substituting at least one amino acid residue in the native Fc region with a different residue or by chemical modification, for example, the alteration of effector function (e.g. reduce or enhance) may be made by changing the affinity of the antibody for an effector ligand (e.g., FcR or complement C1q).

In certain embodiments, the antibody or antigen-binding fragment thereof comprises a mutated or chemically modified Fc region that has a reduced or enhanced antibody-dependent cellular cytotoxicity (ADCC), a reduced or enhanced antibody-dependent cellular phagocytosis (ADCP) and/or a reduced or enhanced complement-dependent cytotoxicity (CDC) as compared to a wild-type Fc region. The methods for obtaining the above-mentioned altered (e.g., reduced) effector functions are known in the art, for example, one or more (e.g., 1, 2, 3 or 4) mutations selected from the following may be introduced into the heavy chain constant region: L234A, L235A, G237A, K322A according to the EU numbering. In certain embodiments, the antibody or antigen-binding fragment thereof comprises the heavy chain constant region (CH) as set forth in SEQ ID NO: 164.

In certain embodiments, the antigen-binding fragment is selected from the group consisting of Fab, Fab', (Fab')₂, Fv, disulfide-linked Fv, scFv, and diabody.

In certain embodiments, the antibody is an IgG antibody, such as an IgG1, IgG2, IgG3 or IgG4 antibody.

In certain embodiments, the antibody or antigen-binding fragment thereof specifically binds to the ε and/or γ chain of CD3 (e.g., human and/or cynomolgus CD3).

In certain embodiments, when measured by surface plasmon resonance (SPR), the antibody or antigen-binding fragment thereof binds to CD3 (e.g., human and/or cynomolgus CD3, for example, ε and/or γ chain ) with a K_{D} not less than 6×10⁻¹⁰M, not less than 6.5 × 10⁻¹⁰M, not less than 7×10⁻¹⁰M, not less than 7.5×10⁻¹⁰M, not less than 8×10⁻¹⁰M, not less than 8.5×10⁻¹⁰M, not less than 9×10⁻¹⁰M, not less than 9.5×10⁻¹⁰M, not less than 1×10⁻⁹M, not less than 1.5×10⁻⁹M, not less than 2×10⁻⁹M, not less than 2.5×10⁻⁹M, not less than 3×10⁻⁹M, not less than 3.5×10⁻⁹M, not less than 4×10⁻⁹M, not less than 4.5×10⁻⁹M, not less than 5×10⁻⁹M, not less than 5.5×10⁻⁹M, not less than 6×10⁻⁹M, not less than 6.5×10⁻⁹M, not less than 7×10⁻⁹M, not less than 7.5×10⁻⁹M, not less than 8×10⁻⁹M, not less than 8.5×10⁻⁹M, not less than 9×10⁻⁹M, not less than 9.5×10⁻⁹M, not less than 1×10⁻⁸M, not less than 1.5×10⁻⁸M, or not less than 2×10⁻⁸M. In certain embodiments, when measured by SPR, the antibody or antigen-binding fragment thereof binds to CD3 (e.g., human and/or cynomolgus CD3, for example, ε and/or γ chain ) with a K_{D} not more than 10⁻⁷M, for example, not more than 9.5×10⁻⁸M, not more than 9×10⁻⁸M, not more than 8.5×10⁻⁸M, not more than 8×10⁻⁸M, not more than 7.5×10⁻⁸M, not more than 7×10⁻⁸M, not more than 6.5×10⁻⁸M, not more than 6×10⁻⁸M, not more than 5.5×10⁻⁸M, not more than 5×10⁻⁸M, not more than 4.5×10⁻⁸M, not more than 4×10⁻⁸M, not more than 3.5×10⁻⁸M, not more than 3×10⁻⁸M, not more than 2.5×10⁻⁸M, or not more than 2×10⁻⁸M. In certain embodiments, when measured by SPR, the antibody or antigen-binding fragment thereof binds to CD3 (e.g., human and/or cynomolgus CD3, for example, ε and/or γ chain ) with a K_{D} of 10⁻⁸ to 10⁻¹⁰M.

In certain embodiments, when measured by flow cytometry, the antibody or antigen-binding fragment thereof binds to CD3 (e.g., human and/or cynomolgus CD3, for example, cells expressing CD3) with an EC₅₀ not less than 0.05 nM, not less than 0.1 nM, not less than 0.2 nM, not less than 0.3 nM, not less than 0.4nM, not less than 0.5nM, not less than 0.6nM, not less than 0.7nM, not less than 0.8nM, not less than 0.9nM, not less than 1nM, not less than 1.5nM, not less than 2nM, not less than 2.5nM, not less than 3nM, not less than 3.5nM, not less than 4nM, not less than 4.5nM, not less than 5nM, not less than 5.5nM, not less than 6nM, not less than 6.5nM, not less than 7 nM, not less than 7.5 nM, not less than 8 nM, not less than 8.5 nM, not less than 9 nM. In certain embodiments, when measured by flow cytometry, the antibody or antigen-binding fragment thereof binds to CD3 (e.g., human and/or cynomolgus CD3, for example, cells expressing CD3) with a EC₅₀ not more than 20 nM, for example, not more than 15 nM, not more than 10 nM, not more than 9.5 nM, not more than 9nM, not more than 8.5nM, not more than 8nM, not more than 7.5nM, not more than 7nM, not more than 6.5nM, not more than 6nM, not more than 5.5nM, not more than 5nM, not more than 4.5 nM, not more than 4 nM, not more than 3.5 nM, not more than 3 nM, not more than 2.5 nM, not more than 2 nM, not more than 1.5 nM, or not more than 1 nM.

### MSLN single-domain antibody

In a second aspect, the present disclosure relates to a single-domain antibody or antigen-binding fragment thereof capable of specifically binding to MSLN (e.g., human MSLN). The single-domain antibody is typically composed of four framework regions (FRs) and three complementarity determining regions (CDRs), called FR1, CDR1, FR2, CDR2, FR3, CDR3 and FR4, and the antigen-binding fragment thereof comprises at least a part of the single-domain antibody, and the part is sufficient to confer the ability of specifically binding to antigen (e.g., MSLN). The single-domain antibody can be truncated at the N- or C-terminal so that it comprises only part of FR1 and/or FR4, or lacks one or two of those backbone regions, as long as it substantially retains the ability of specifically binding antigen.

In certain embodiments, the single-domain antibody or antigen-binding fragment thereof of the present disclosure comprises the following CDRs defined by the IMGT numbering system: a CDR1 comprising the sequence as set forth in SEQ ID NO: 150 or variant thereof, a CDR2 comprising the sequence as set forth in SEQ ID NO: 151 or variant thereof, and a CDR3 comprising the sequence as set forth in SEQ ID NO: 152 or variant thereof; wherein the variant has a substitution, deletion or addition of one or several amino acids (e.g. a substitution, deletion or addition of 1, 2 or 3 amino acids) as compared to the sequence from which it is derived. Preferably, the substitution is a conservative substitution. In certain embodiments, the single-domain antibody or antigen-binding fragment thereof comprises the following CDRs defined by the IMGT numbering system: a CDR1 comprising the sequence as set forth in SEQ ID NO: 150, a CDR2 comprising the sequence as set forth in SEQ ID NO: 151, and a CDR3 comprising the sequence as set forth in SEQ ID NO: 152.

In certain embodiments, the single-domain antibody or antigen-binding fragment thereof of the present disclosure comprises the following CDRs defined by the Kabat numbering system: a CDR1 comprising the sequence as set forth in SEQ ID NO: 153 or variant thereof, a CDR2 comprising the sequence as set forth in SEQ ID NO: 154 or variant thereof, and a CDR3 comprising the sequence as set forth in SEQ ID NO: 155 or variant thereof; wherein the variant has a substitution, deletion or addition of one or several amino acids (e.g. a substitution, deletion or addition of 1, 2 or 3 amino acids) as compared to the sequence from which it is derived. Preferably, the substitution is a conservative substitution. In certain embodiments, the single-domain antibody or antigen-binding fragment thereof comprises the following CDRs as defined by the Kabat numbering system: a CDR1 comprising the sequence as set forth in SEQ ID NO: 153, a CDR2 comprising the sequence as set forth in SEQ ID NO: 154, and a CDR3 comprising the sequence as set forth in SEQ ID NO: 155.

In certain embodiments, the single-domain antibody or antigen-binding fragment thereof of the present disclosure comprises the following CDRs defined by the AbM numbering system: a CDR1 comprising the sequence as set forth in SEQ ID NO: 156 or variant thereof, a CDR2 comprising the sequence as set forth in SEQ ID NO: 157 or variant thereof, and a CDR3 comprising the sequence as set forth in SEQ ID NO: 155 or variant thereof; wherein the variant has a substitution, deletion or addition of one or several amino acids (e.g. a substitution, deletion or addition of 1, 2 or 3 amino acids) as compared to the sequence from which it is derived. Preferably, the substitution is a conservative substitution. In certain embodiments, the single-domain antibody or antigen-binding fragment thereof comprises the following CDRs defined by the AbM numbering system: a CDR1 comprising the sequence as set forth in SEQ ID NO: 156, a CDR2 comprising the sequence as set forth in SEQ ID NO: 157, and a CDR3 comprising the sequence as set forth in SEQ ID NO: 155.

In certain embodiments, the single-domain antibody or antigen-binding fragment thereof of the present disclosure comprises the following CDRs defined by the Chothia numbering system: a CDR1 comprising the sequence as set forth in SEQ ID NO: 158 or variant thereof, a CDR2 comprising the sequence as set forth in SEQ ID NO: 159 or variant thereof, and a CDR3 comprising the sequence as set forth in SEQ ID NO: 155 or variant thereof; wherein the variant has a substitution, deletion or addition of one or several amino acids (e.g. a substitution, deletion or addition of 1, 2 or 3 amino acids) as compared to the sequence from which it is derived. Preferably, the substitution is a conservative substitution. In certain embodiments, the single-domain antibody or antigen-binding fragment thereof comprises the following CDRs as defined by the Chothia numbering system: a CDR1 comprising the sequence as set forth in SEQ ID NO: 158, a CDR2 comprising the sequence as set forth in SEQ ID NO: 159, and a CDR3 comprising the sequence as set forth in SEQ ID NO: 155.

In certain embodiments, the single-domain antibody or antigen-binding fragment thereof of the present disclosure comprises the following CDRs defined by the Contact numbering system: a CDR1 comprising the sequence as set forth in SEQ ID NO: 160 or variant thereof, a CDR2 comprising the sequence as set forth in SEQ ID NO: 161 or variant thereof, and a CDR3 comprising the sequence as set forth in SEQ ID NO: 162 or variant thereof; wherein the variant has a substitution, deletion or addition of one or several amino acids (e.g. a substitution, deletion or addition of 1, 2 or 3 amino acids) as compared to the sequence from which it is derived. Preferably, the substitution is a conservative substitution. In certain embodiments, the single-domain antibody or antigen-binding fragment thereof comprises the following CDRs defined by the Contact numbering system: a CDR1 comprising the sequence as set forth in SEQ ID NO: 160, a CDR2 comprising the sequence as set forth in SEQ ID NO: 161, and a CDR3 comprising the sequence as set forth in SEQ ID NO: 162.

In certain embodiments, the single-domain antibody or antigen-binding fragment thereof comprises the sequence as set forth in SEQ ID NO: 149 or variant thereof, wherein the variant has a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence from which it derives, or has a substitution, deletion or addition of one or several amino acids (e.g., a substitution, deletion or addition of 1, 2, 3, 4, 5, 6, 7, 8, 9 or 10 amino acids) as compared thereto. In certain embodiments, the substitution is a conservative substitution.

In certain embodiments, the single-domain antibody or antigen-binding fragment thereof of the present disclosure, can be humanized VHHs, i.e., VHHs in which one or more backbone regions have been substantially replaced with human backbone regions. In certain embodiments, the single-domain antibody or antigen-binding fragment thereof further comprises a heavy chain framework region of a human immunoglobulin (e.g., a heavy chain framework region contained in the amino acid sequence encoded by an human heavy chain germline antibody gene), the heavy chain framework region optionally comprises one or more (e.g., 1, 2, 3, 4, 5, 6, 7, 8, 9 or 10 ) back mutation from a human residue to a camel residue.

In certain embodiments, when measured by SPR, the single-domain antibody or antigen-binding fragment thereof binds to MSLN (e.g., human MSLN) with a K_{D} not more than 10⁻⁸M, not more than 9.5×10⁻⁹M, 9×10⁻⁹M, not more than 8.5×10⁻⁹M, not more than 8×10⁻⁹M, not more than 7.5×10⁻⁹M, not more than 7×10⁻⁹M, not more than 6.5×10⁻⁹M, not more than 6×10⁻⁹M, not more than 5.5×10⁻⁹M, not more than 5×10⁻⁹M, not more than 4.5×10⁻⁹M, not more than 4×10⁻⁹M, not more than 3.5×10⁻⁹M, not more than 3×10⁻⁹M, not more than 2.5×10⁻⁹M, not more than 2×10⁻⁹M, not more than 1.5×10⁻⁹M, not more than 10⁻⁹M, not more than 9.5×10⁻¹⁰M, not more than 9×10⁻¹⁰M, not more than 8.5×10⁻¹⁰M, not more than 8×10⁻¹⁰M, not more than 7.5×10⁻¹⁰M, not more than 7×10⁻¹⁰M.

In certain embodiments, when measured by flow cytometry, the single-domain antibody or antigen-binding fragment thereof binds to MSLN (e.g., human MSLN, for example, cells expressing MSLN) with an EC₅₀ less than about 100 nM, 10 nM, 1 nM, 0.9 nM, 0.8 nM, 0.7 nM, 0.6 nM, 0.5 nM, 0.4 nM, 0.3 nM, 0.2 nM, 0.1 nM or less.

### Multispecific antibody

The antibody or antigen-binding fragment thereof of the first aspect and/or the single-domain antibody or antigen-binding fragment thereof of the second aspect of the present disclosure may be used to form a multispecific antibody. The multispecific antibody is an antibody capable of specifically binding to at least two (e.g., two, three, or four) different antigens, thereby being able to bind to at least two different binding sites and/or target molecules.

Accordingly, in a third aspect, the present disclosure relates to a multispecific antibody.

### Multispecific antibody 1

The present disclosure provides a multispecific antibody 1, which comprises a CD3-targeting antigen-binding domain and at least one antigen-binding domain targeting an additional antigen, and the CD3-targeting antigen-binding domain is selected from the antibody or antigen-binding fragment thereof of the first aspect, and the additional antigen is selected from tumor-associated antigen (TAA) and/or immune checkpoint molecule.

In certain embodiments, the multispecific antibody is a bispecific antibody, which comprises the CD3-targeting antigen-binding domain of the first aspect and an antigen-binding domain targeting the tumor-associated antigen. When a bispecific antibody comprises a first antigen-binding region (e.g., the antibody of the first aspect) that binds a T cell-specific target (e.g., CD3) and a second antigen-binding region that binds a tumor-specific target, the bispecific antibody is capable of promoting the targeting and recruitment of T cells to tumor cells by binding to CD3 present on T cells and specific target antigens on tumor cells, thereby inducing the killing activity of tumor-specific (MHC-independent) cytotoxic T cells.

In certain embodiments, the multispecific antibody is a trispecific antibody, which comprises the CD3-targeting antigen-binding domain of the first aspect, an antigen-binding domain targeting the tumor-associated antigen, and an antigen-binding domain targeting the immune checkpoint molecule.

Those skilled in the art will understand that all bispecific antibody structural forms known in the art can be used in the present disclosure. In certain embodiments, the bispecific antibody of the present disclosure may be: (i) a single antibody with two arms comprising different antigen-binding domains, (ii) a single chain antibody formed by linking two scFvs in series, for example, through an additional peptide linker, that is specific for two different epitopes; (iii) a dual variable domain antibody (DVD-Ig^{™}), in which each of light chain and heavy chain contains two variable domains linked in series by a short peptide; (iv) a chemically linked bispecific (Fab')₂ fragment; (v) a TandAb, which is a fusion of two single-chain diabodies, resulting in a tetravalent bispecific antibody with two binding sites for each target antigen; (vi) a flexibody, which is a combination of scFv and diabody, resulting in a multivalent molecule; (vii) a so-called "dock and lock" molecule, which is based on the "polymerization and docking domain" in protein kinase A, when it is applied to Fab, a trivalent bispecific binding protein consisting of two identical Fab fragments connected to different Fab fragments is obtained; (viii) a so-called scorpion molecule, which comprises, for example, two scFv fused to both ends of a human Fab arm; and (ix) a diabody.

In certain embodiments, the CD3-targeting antigen-binding domain is a full-length antibody, Fv fragment, Fab fragment, F(ab')₂ fragment, or scFv. In certain embodiments, the additional antigen-binding domains comprised by the multispecific antibody (e.g., an antigen-binding domain targeting tumor-associated antigen, an antigen-binding domain targeting immune checkpoint molecule) are independently selected from the group consisting of full length antibody, Fv fragment, Fab fragment, F(ab')₂ fragment, scFv and VHH.

In certain embodiments, the antigen binding domains comprised by the multispecific antibody are each linked by a peptide linker. In certain embodiments, the antigen-binding domain targeting tumor-associated antigen or immune checkpoint molecule is optionally linked via a linker to the N-terminal and/or C-terminal of a heavy chain of the CD3-targeting antigen-binding domain, and/or the N-terminal and/or C-terminal of a light chain of the CD3-targeting antigen-binding domain. In certain embodiments, the CD3-targeting antigen-binding domain comprises: at least one heavy chain and at least one light chain, and the antigen-binding domain targeting tumor-associated antigen or immune checkpoint molecule is linked to the heavy chain; or the CD3-targeting antigen-binding domain comprises: two identical heavy chains and two identical light chains, and the antigen-binding domain targeting tumor-associated antigen or immune checkpoint molecule is linked to two heavy chains.

In certain embodiments, the tumor-associated antigen is selected from the group consisting of CD19, BCMA, EGFR, HER2, HER3, HER4, PSMA, EpCAM, EphA2, CD33, CD123, CD38, CLDN18, MSLN, TROP2, Mucin1, AFP, CD79b, GUCY2C, LRRC15, gp100, STEAP1, ROR1, 5T4, CEA, DLL3, CD20, CD7, PRAME, CDH19, CDH17, GPA33, HLA-A2, CD34, FAP, GPRC5D, GPC3, B7-H3, CLL-1, CLDN6, Flt3, NY-ESO-1, PSCA, NECTIN-4, ENPP3, IGFR-1, TSA1, Melan-A, MUC16 (CA125), MUC17, SSTR2, c-Met, B7-H6, CSPG4, CAIX, MCSP, BIRC5, BIRC7, BRCA1, BORIS, CCR5, GD2, GD3, GloboH, GM3, hTERT, LMP2, p53, PAP, PAX₃, PAXs, PCTA-1, PLAC1, PRLR, Ras, SART-3, TRP-1, TRP-2, CD22, CD30, FOLR1, and any combination thereof.

In certain preferred embodiments, the tumor-associated antigen is selected from the group consisting of MSLN, CD19, CD20, TROP2, HER2, and Caludin18.2.

In certain embodiments, the immune checkpoint molecule is selected from the group consisting of PD-1, PD-L1, PD-L2 CTLA-4, TIM-3, Lag-3, TIGIT, CD73, VISTA, B7-H3, NKG2D, NKG2A, OX40, OX40L, CD40, CD47, LIGHT, ICOS, HVEM, BTLA, B7-H4, 4-1BB, 4-1BBL, and any combination thereof.

In certain embodiments, the multispecific antibody comprises a first polypeptide chain and a second polypeptide chain, wherein the first polypeptide chain comprises a heavy chain of the antibody of the first aspect, and an antigen-binding domain that is capable of specifically binding to tumor-associated antigen (e.g., MSLN, CD19, CD20, Trop2, Her2 or Caludin18.2) and is linked to the N- or C-terminal of the heavy chain; and the second polypeptide chain comprises a light chain of the antibody of the first aspect.

In certain embodiments, the multispecific antibody is a CD3/MSLN-targeting bispecific antibody, which comprises an antigen-binding domain capable of specifically binding CD3 and an antigen-binding domain capable of specifically binding MSLN, the CD3-targeting antigen-binding domain is selected from the antibody or antigen-binding fragment thereof described in the first aspect; and the MSLN-targeting antigen-binding domain is selected from the single-domain antibody or antigen-binding fragment thereof described in the second aspect.

In certain embodiments, the CD3/MSLN-targeting bispecific antibody comprises a first polypeptide chain and a second polypeptide chain, wherein the first polypeptide chain comprises the structure shown below: [VH]-[CH]-[L]-[VHH], wherein [VH] is selected from the heavy chain variable region sequence provided in the first aspect (e.g., the sequence as set forth in any one of SEQ ID NOs: 1-46), [CH] is selected from the heavy chain constant region sequence provided in the first aspect (e.g., the sequence as set forth in SEQ ID NO: 164), [L] is a peptide linker (e.g., a peptide linker containing one or more glycines and/or one or more serines, such as the sequence as set forth in SEQ ID NO: 163), [VHH] is selected from the single-domain antibody sequence provided in the second aspect (e.g., the sequence as set forth in SEQ ID NO: 149); the second polypeptide chain comprises the structure shown below: [VL]-[CL], wherein [VL] is selected from the light chain variable region sequence provided in the first aspect (e.g., the sequence as set forth in SEQ ID NO: 47 or 48), [CL] is selected from the light chain constant region sequence provided in the first aspect (e.g., the sequence as set forth in SEQ ID NO: 165).

In certain embodiments, when measured by SPR, the bispecific antibody targeting CD3 and MSLN of the present disclosure binds to CD3 (e.g., human and /or cynomolgus CD3, e.g. ε and/or γ chains) with a K_{D} not less than 6×10⁻¹⁰M, not less than 6.5×10⁻¹⁰M, not less than 7×10⁻¹⁰M, not less than 7.5×10⁻¹⁰M, not less than 8×10⁻¹⁰M, not less than 8.5×10⁻¹⁰M, not less than 9×10⁻¹⁰M, not less than 9.5×10⁻¹⁰M, not less than 1×10⁻⁹M, not less than 1.5×10⁻⁹M, not less than 2×10⁻⁹M, not less than 2.5×10⁻⁹M, not less than 3×10⁻⁹M, not less than 3.5×10⁻⁹M, not less than 4×10⁻⁹M, not less than 4.5×10⁻⁹M, not less than 5×10⁻⁹M, not less than 5.5×10⁻⁹M, not less than 6×10⁻⁹M, not less than 6.5×10⁻⁹M, not less than 7×10⁻⁹M, not less than 7.5×10⁻⁹M, not less than 8×10⁻⁹M, not less than 8.5×10⁻⁹M, not less than 9×10⁻⁹M, not less than 9.5×10⁻⁹M, not less than 1×10⁻⁸M, not less than 1.5×10⁻⁸M, or not less than 2×10⁻⁸M. In certain embodiments, when measured by SPR, the bispecific antibody targeting CD3 and MSLN of the present disclosure binds to CD3 (e.g., human and /or cynomolgus CD3, e.g. ε and/or γ chains) with a K_{D} not more than 10⁻⁷M, for example, not more than 9.5×10⁻⁸M, not more than 9×10⁻⁸M, not more than 8.5×10⁻⁸M, not more than 8×10⁻⁸M, not more than 7.5×10⁻⁸M, not more than 7×10⁻⁸M, not more than 6.5×10⁻⁸M, not more than 6×10⁻⁸M, not more than 5.5×10⁻⁸M, not more than 5×10⁻⁸M, not more than 4.5×10⁻⁸M, not more than 4×10⁻⁸M, not more than 3.5×10⁻⁸M, not more than 3×10⁻⁸M, not more than 2.5×10⁻⁸M, or not more than 2×10⁻⁸M. In certain embodiments, when measured by SPR, the bispecific antibody binds to CD3 (e.g., human and/or cynomolgus CD3, e.g. ε and/or γ chains) with a K_{D} of 10⁻⁸ to 10⁻¹⁰ M.

In certain embodiments, when measured by flow cytometry, the bispecific antibody targeting CD3 and MSLN of the present disclosure binds to CD3 (e.g., human and/or cynomolgus CD3, such as CD3-expressing cells) with an EC₅₀ not less than 0.05 nM, not less than 0.1 nM, not less than 0.2 nM, not less than 0.3nM, not less than 0.4nM, not less than 0.5nM, not less than 0.6nM, not less than 0.7nM, not less than 0.8nM, not less than 0.9nM, not less than InM, not less than 1.5nM, not less than 2nM, not less than 2.5nM, not less than 3nM, not less than 3.5nM, not less than 4nM, not less than 4.5nM, not less than 5nM, not less than 5.5nM, not less than 6 nM, not less than 6.5 nM, not less than 7 nM, not less than 7.5 nM, not less than 8 nM, not less than 8.5 nM, not less than 9 nM. In certain embodiments, when measured by flow cytometry, the bispecific antibody targeting CD3 and MSLN of the present disclosure binds to CD3 (e.g., human and/or cynomolgus CD3, such as CD3-expressing cells) with an EC₅₀ not more than 20 nM, for example, not more than 15 nM, not more than 10 nM, not more than 9.5nM, not more than 9nM, not more than 8.5nM, not more than 8nM, not more than 7.5nM, not more than 7nM, not more than 6.5nM, not more than 6nM, not more than 5.5nM, not more than 5 nM, not more than 4.5 nM, not more than 4 nM, not more than 3.5 nM, not more than 3 nM, not more than 2.5 nM, not more than 2 nM, not more than 1.5 nM or not more than 1 nM.

In certain embodiments, when measured by SPR, the bispecific antibody targeting CD3 and MSLN of the present disclosure binds to MSLN (e.g., human MSLN) with a K_{D} not more than 10⁻⁸M, not more than 9.5×10⁻⁹M, 9×10⁻⁹M, not more than 8.5×10⁻⁹M, not more than 8×10⁻⁹M, not more than 7.5×10⁻⁹M, not more than 7×10⁻⁹M, not more than 6.5×10⁻⁹M, not more than 6×10 ⁹M, not more than 5.5×10⁻⁹M, not more than 5×10⁻⁹M, not more than 4.5×10⁻⁹M, not more than 4×10⁻⁹M, not more than 3.5×10⁻⁹M, not more than 3×10⁻⁹M, not more than 2.5×10⁻⁹M, not more than 2×10⁻⁹M, not more than 1.5×10⁻⁹M, not more than 10⁻⁹M, not more than 9.5×10⁻¹⁰M, not more than 9×10⁻¹⁰M, not more than 8.5×10⁻¹⁰M, not more than 8×10⁻¹⁰M, not more than 7.5×10⁻¹⁰M, not more than 7×10⁻¹⁰M.

In certain embodiments, when measured by flow cytometry, the bispecific antibody targeting CD3 and MSLN of the present disclosure binds to MSLN (e.g., human MSLN, for example, cells expressing MSLN) with an EC₅₀ less than about 100 nM, 10 nM, 1 nM, 0.9 nM, 0.8 nM, 0.7 nM, 0.6 nM, 0.5 nM, 0.4 nM, 0.3 nM, 0.2 nM, 0.1 nM or less.

In certain embodiments, the multispecific antibody is a CD3/CD19-targeting bispecific antibody, which comprises an antigen-binding domain capable of specifically binding CD3 and an antigen-binding domain capable of specifically binding CD19, the CD3-targeting antigen-binding domain is selected from the antibody or antigen-binding fragment thereof described in the first aspect; the CD19-targeting antigen-binding domain comprises a light chain variable region and a heavy chain variable region, the light chain variable region comprises the sequence as set forth in SEQ ID NO: 171 or variant thereof, and the heavy chain variable region comprises the sequence as set forth in SEQ ID NO: 170 or variant thereof.

In certain embodiments, the multispecific antibody is a CD3/CD20-targeting bispecific antibody, which comprises an antigen-binding domain targeting CD3 and an antigen-binding domain capable of specifically binding CD20, the CD3-targeting antigen-binding domain is selected from the antibody or antigen-binding fragment thereof described in the first aspect; the CD20-targeting antigen-binding domain comprises a light chain variable region and a heavy chain variable region, the light chain variable region comprises the sequence as set forth in SEQ ID NO: 173 or variant thereof, and the heavy chain variable region comprises the sequence as set forth in SEQ ID NO: 172 or variant thereof.

In certain embodiments, the multispecific antibody is a CD3/trop2-targeting bispecific antibody, which comprises an antigen-binding domain targeting CD3 and an antigen-binding domain capable of specifically binding trop2, the CD3-targeting antigen-binding domain is selected from the antibody or antigen-binding fragment thereof described in the first aspect; and the trop2-targeting antigen-binding domain is a VHH, which comprises the sequence as set forth in SEQ ID NO: 174 or variant thereof.

In certain embodiments, the multispecific antibody is a CD3/Her2-targeting bispecific antibody, which comprises an antigen-binding domain targeting CD3 and an antigen-binding domain capable of specifically binding Her2, the antigen-binding domain targeting CD3 is selected from the antibody or antigen-binding fragment thereof described in the first aspect; and the antigen-binding domain targeting Her2 is a VHH, which comprises the sequence as set forth in SEQ ID NO: 175 or variant thereof.

In certain embodiments, the multispecific antibody is a CD3/Caludin18.2-targeting bispecific antibody, which comprises an antigen-binding domain targeting CD3 and an antigen-binding domain capable of specifically binding Caludin18.2, the CD3-targeting antigen-binding domain is selected from the antibody or antigen-binding fragment thereof described in the first aspect; and the Caludin18.2-targeting antigen-binding domain is a VHH, which comprises the sequence as set forth in SEQ ID NO: 176 or variant thereof.

### Multispecific antibody 2

The present disclosure also provides a multispecific antibody 2, which comprises the single-domain antibody or antigen-binding fragment thereof described in the second aspect; in certain preferred embodiments, the multispecific antibody is a bispecific antibody, a trispecific antibody, or a tetraspecific antibody.

### Preparation of antibodies

The antibody of the first aspect, the single-domain antibody of the second aspect, or the multispecific antibody of the third aspect of the present disclosure can be prepared by various methods known in the art, such as by genetic engineering and recombinant technology. For example, the DNA molecules encoding them are obtained by chemical synthesis or PCR amplification. The resulting DNA molecule is inserted into an expression vector and then transfected into host cells. Then, the transfected host cells are cultured under specific conditions and express the antibody, single-domain antibody or multispecific antibody of the present disclosure.

In a fourth aspect, the present disclosure provides an isolated nucleic acid molecule, which encodes:
- the antibody or antigen-binding fragment thereof, or its heavy chain variable region and/or light chain variable region according to the first aspect;
- the single-domain antibody or antigen-binding fragment thereof according to the second aspect; or,
- the multispecific antibody 1 or the multispecific antibody 2, or a polypeptide chain thereof according to the third aspect.

In certain embodiments, the isolated nucleic acid molecule comprises a first nucleotide sequence encoding a heavy chain or heavy chain variable region of the antibody or antigen-binding fragment thereof of the first aspect, and a second nucleotide sequence encoding a light chain or light chain variable region of the antibody or antigen-binding fragment thereof of the first aspect, wherein the first nucleotide sequence and the second nucleotide sequence are present on the same or different isolated nucleic acid molecules.

In certain embodiments, the isolated nucleic acid molecule comprises a first nucleotide sequence encoding a first polypeptide chain of the bispecific antibody of the third aspect, and a second nucleotide sequence encoding a second polypeptide chain of the bispecific antibody of the third aspect, wherein the first nucleotide sequence and the second nucleotide sequence are present on the same or different isolated nucleic acid molecules.

In a fifth aspect, the present disclosure provides a vector, which comprises the nucleic acid molecule as described above. In certain embodiments, the vector is a cloning vector or an expression vector.

In a sixth aspect, the present disclosure provides a host cell, which comprises the nucleic acid molecule or vector as described above. Such host cells comprise, but are not limited to, prokaryotic cells such as bacterial cells (e.g., *E. coli* cells), and eukaryotic cells such as fungal cells (e.g., yeast cells), insect cells, plant cells, and animal cells (e.g., mammalian cells, for example, mouse cells, human cells, etc.).

In another aspect, the present disclosure provides a method for preparing the antibody or antigen-binding fragment thereof according to the first aspect, the single-domain antibody according to the second aspect, or the multispecific antibody according to the third aspect, which comprises: culturing the host cell as described above under conditions that allow protein expression, and collecting the antibody or antigen-binding fragment thereof, single-domain antibody or multispecific antibody from a culture of the cultured host cell.

### Pharmaceutical composition

The antibody disclosed herein (also referred to as active ingredient) can be incorporated into a pharmaceutical composition suitable for administration.

Therefore, in the seventh aspect, the present disclosure relates to a pharmaceutical composition, which comprises the antibody or antigen-binding fragment thereof according to the first aspect, the single-domain antibody or antigen-binding fragment thereof according to the second aspect, the multispecific antibody according to the third aspect, the isolated nucleic acid molecule according to the fourth aspect, the vector according to the fifth aspect, or the host cell according to the sixth aspect, and a pharmaceutically acceptable carrier and/or excipient.

In certain embodiments, the pharmaceutical composition may further comprise an additional pharmaceutically active agent, such as an antineoplastic agent. In certain embodiments, the additional pharmaceutically active agent and the antibody or antigen-binding fragment thereof, single-domain antibody or antigen-binding fragment thereof, multispecific antibody, isolated nucleic acid molecule, vector, or host cell of the present disclosure are provided separately, or provided as a component of the same composition.

### Therapeutic use

In an eighth aspect, the present disclosure relates to a method for preventing and/or treating a disease, which comprises administering to a subject in need thereof the antibody or antigen-binding fragment thereof according to the first aspect, an isolated nucleic acid molecule, vector or host cell encoding the antibody or antigen-binding fragment thereof, or a pharmaceutical composition containing the same. The present disclosure also relates to a use of the antibody or antigen-binding fragment thereof according to the first aspect, an isolated nucleic acid molecule, vector or host cell encoding the antibody or antigen-binding fragment thereof, or a pharmaceutical composition containing them, for preventing and/or treating a disease, or in the manufacture of a medicament for preventing and/or treating a disease.

The antibody or antigen-binding fragment thereof described in the first aspect of the present disclosure can be used in the treatment of any disease, as long as the effector mechanism of cytotoxic T cells is required in the treatment of the disease.

In certain embodiments, the disease is a tumor, inflammatory disease, or autoimmune disease.

In certain embodiments, the tumor is a solid tumor or hematological tumor, such as gastric cancer, lung cancer, ovarian cancer, esophageal cancer, pancreatic cancer, cervical cancer, mesothelioma, breast cancer, prostate cancer, bladder cancer, ovarian cancer, colorectal cancer, head and neck squamous cell carcinoma, pancreatic cancer, testicular cancer, cholangiocarcinoma, colon cancer, fallopian tube cancer, malignant melanoma, soft tissue cancer (e.g., synovial sarcoma), painless or aggressive form of B-cell lymphoma, chronic lymphocytic leukemia, acute myeloid leukemia, or acute lymphoblastic leukemia.

In certain embodiments, the treatment of the disease involves effector mechanism of T cells (e.g., cytotoxic T cells). In certain embodiments, the antibody or antigen-binding fragment thereof is used to enhance the activity of T cells (e.g., cytotoxic T cells) to treat or prevent the disease.

In a ninth aspect, the present disclosure relates to a method for preventing and/or treating a tumor, comprising administering to a subject in need thereof the single-domain antibody or antigen-binding fragment thereof according to the second aspect, an isolated nucleic acid molecule, vector or host cell encoding the antibody or antigen-binding fragment thereof, or a pharmaceutical composition containing the same. The present disclosure also relates to a use of the single-domain antibody or antigen-binding fragment thereof according to the second aspect, an isolated nucleic acid molecule, vector or host cell encoding the antibody or antigen-binding fragment thereof, or a pharmaceutical composition containing the same, for preventing and/or treating a tumor, or in the manufacture of a medicament for preventing and/or treating a tumor.

In certain embodiments, the tumor is an MSLN-positive tumor.

In certain embodiments, the tumor is a solid tumor or hematological tumor, such as gastric cancer, lung cancer, ovarian cancer, esophageal cancer, pancreatic cancer, cervical cancer, mesothelioma, breast cancer, prostate cancer, bladder cancer, ovarian cancer, colorectal cancer, head and neck squamous cell carcinoma, pancreatic cancer, testicular cancer, cholangiocarcinoma, colon cancer, fallopian tube cancer, malignant melanoma, soft tissue cancer (e.g., synovial sarcoma), painless or aggressive form of B-cell lymphoma, chronic lymphocytic leukemia, acute myeloid leukemia, or acute lymphoblastic leukemia.

In certain embodiments, the tumor is selected from solid tumor, such as mesothelioma, ovarian cancer, pancreatic cancer, breast cancer, cholangiocarcinoma, colon cancer, gastric cancer, fallopian tube cancer, lung cancer, or colorectal cancer.

In certain embodiments, the tumor is selected from hematological tumor, such as acute myeloid leukemia.

In a tenth aspect, the present disclosure relates to a method for preventing and/or treating a disease, which comprises administering to a subject in need thereof the multispecific antibody according to the third aspect, an isolate nucleic acid molecule, vector or host cell encoding the multispecific antibody, or a pharmaceutical composition containing the same. The present disclosure also relates to a use of the multispecific antibody according to the third aspect, an isolated nucleic acid molecule, vector or host cell encoding the multispecific antibody, or a pharmaceutical composition containing the same, for preventing and/or treating a disease, or in the manufacture of a medicament for preventing and/or treating a disease.

The multispecific antibody described in the third aspect of the present disclosure can be used in the treatment of any disease, as long as the effector mechanism of cytotoxic T cells is required in the treatment of the disease. For example, when the multispecific antibody of the present disclosure comprises a CD3-binding arm for T cell recruitment and a tumor-targeting arm specific for tumor-associated antigen (TAA), it brings the T cells into close contact with the target tumor cells, activates locally the T cells, and subsequently destroys the target cells with perforin and granzymes released from the toxic granules of T cells. For example, when the multispecific antibody of the present disclosure comprises a CD3-binding arm for recruitment of T cells and a targeting arm specific for immune checkpoint molecules, it activates T cells in the tumor microenvironment and subsequently kills tumor cells.

In certain embodiments, the disease is a tumor, inflammatory disease, or autoimmune disease.

In certain embodiments, the tumor is a solid tumor or hematological tumor, such as gastric cancer, lung cancer, ovarian cancer, esophageal cancer, pancreatic cancer, cervical cancer, mesothelioma, breast cancer, prostate cancer, bladder cancer, ovarian cancer, colorectal cancer, head and neck squamous cell carcinoma, pancreatic cancer, testicular cancer, cholangiocarcinoma, colon cancer, fallopian tube cancer, malignant melanoma, soft tissue cancer (e.g., synovial sarcoma), painless or aggressive form of B-cell lymphoma, chronic lymphocytic leukemia, acute myeloid leukemia, or acute lymphoblastic leukemia.

In certain embodiments, the treatment of the disease involves effector mechanism of T cells (e.g., cytotoxic T cells). In certain embodiments, the multispecific antibody is used to recruit T cells to the surroundings of target cells, activate T cells, and induce T-cell dependent cellular cytotoxicity (TDCC), thereby effectively killing target cells to treat or prevent the disease.

In certain embodiments, the tumor is an MSLN-positive tumor, such as a solid tumor, for example, mesothelioma, ovarian cancer, pancreatic cancer, breast cancer, cholangiocarcinoma, colon cancer, gastric cancer, fallopian tube cancer, lung cancer, or colorectal cancer; for example, blood tumor, such as acute myeloid leukemia.

In certain embodiments, the tumor is a CD19-positive tumor, such as a B-cell malignancy, for example, lymphoma or leukemia.

In certain embodiments, the tumor is a CD20-positive tumor, such as a B-cell malignancy, for example, lymphoma or leukemia.

In certain embodiments, the tumor is a Trop2-positive tumor, such as a solid tumor, for example, breast cancer.

In certain embodiments, the tumor is an Her2-positive tumor, such as a solid tumor, for example, lung cancer.

In certain embodiments, the tumor is a Caludin18.2-positive tumor, such as a solid tumor, for example gastric cancer.

In any one of the eighth to tenth aspects of the present disclosure, the antibody or antigen-binding fragment thereof, the single-domain antibody or antigen-binding fragment thereof, the multispecific antibody or the pharmaceutical composition comprising the same according to the present disclosure may be formulated into any dosage forms known in the medical field.

In any one of the eighth to tenth aspects of the present disclosure, the antibody or antigen-binding fragment thereof, the single-domain antibody or antigen-binding fragment thereof, the multispecific antibody or the pharmaceutical composition containing the same according to the present disclosure can be administered by any suitable methods known in the art.

In any one of the eighth to tenth aspects of the present disclosure, the antibody or antigen-binding fragment thereof, the single-domain antibody or antigen-binding fragment thereof, the multispecific antibody or the pharmaceutical composition comprising the same according to the present disclosure can be formulated into a dosage unit form for ease of administration.

In any one of the eighth to tenth aspects of the present disclosure, the antibody or antigen-binding fragment thereof, the single-domain antibody or antigen-binding fragment thereof, the multispecific antibody or the pharmaceutical composition comprising the same according to the present disclosure may be administered alone, may also be administered in combination with an additional pharmaceutically active agent (e.g., an antineoplastic agent) or additional therapy (e.g., an antineoplastic therapy).

In any of the eighth to tenth aspects of the present disclosure, the subject may be a mammal, such as a human.

### Definition of Terms

In the present disclosure, unless otherwise stated, scientific and technical terms used herein have the meanings commonly understood by those skilled in the art. Moreover, the virology, biochemistry, and immunology laboratory procedures used herein are routine procedures widely used in the corresponding fields. Meanwhile, in order to better understand the present disclosure, definitions and explanations of relevant terms are provided below.

When the terms "e.g.," "such as," "for example," "comprise," "contain," or variations thereof are used herein, these terms will not be considered as limiting terms and will instead be interpreted to mean "but not limited to" or "without limitation."

Unless otherwise indicated herein or clearly contradicted by context, the terms "a" and "an" as well as "the" and similar referents in the context of describing the present disclosure (especially in the context of the following claims) are to be construed to cover singular and plural forms.

As used herein, the term "antibody" refers to an immunoglobulin molecule typically composed of two pairs of polypeptide chains, each pair having a light chain (LC) and a heavy chain (HC). Antibody light chains can be classified into κ (kappa) and λ (lambda) light chains. Heavy chains can be classified as µ, δ, γ, α, or ε, and define the antibody's isotypes as IgM, IgD, IgG, IgA, and IgE, respectively. Within the light and heavy chains, the variable and constant regions are connected by a "J" region of approximately 12 or more amino acids, and the heavy chain also contains a "D" region of approximately 3 or more amino acids. Each heavy chain consists of a heavy chain variable region (VH) and a heavy chain constant region (CH). The heavy chain constant region consists of 3 domains (CH1, CH2 and CH3). Each light chain consists of a light chain variable region (VL) and a light chain constant region (CL). The light chain constant region consists of one domain, CL. The constant domain is not directly involved in the binding of antibody to antigen, but exhibits a variety of effector functions, such as mediating the interaction of immunoglobulin with host tissue or factor, including the binding of various cells of the immune system (e.g., effector cells) to the first component of the classical complement system (Clq). The VH and VL regions can also be subdivided into regions of high variability called complementarity determining regions (CDRs), interspersed with more conservative regions called framework regions (FRs). Each of V_{H} and V_{L} consists of 3 CDRs and 4 FRs arranged from the amino terminus to the carboxyl terminus in the following sequence: FR1, CDR1, FR2, CDR2, FR3, CDR3, FR4. The variable regions (VH and VL) of each heavy chain/light chain pair respectively form an antigen-binding site. The assignment of amino acids to regions or domains can follow the definitions of Kabat, Sequences of Proteins of Immunological Interest (National Institutes of Health, Bethesda, Md. (1987 and 1991)), or Chothia & Lesk (1987) J. Mol. Biol. 196 :901-917; Chothia et al. (1989) Nature 342:878-883.

As used herein, the term "complementarity determining region" or "CDR" refers to those amino acid residues in a variable region of an antibody that are responsible for antigen binding. The variable regions of the heavy chain and light chain each contain three CDRs, named as CDR1, CDR2 and CDR3. The precise boundaries of these CDRs can be defined according to various numbering systems known in the art, such as the Kabat numbering system (Kabat et al., Sequences of Proteins of Immunological Interest, 5th Ed. Public Health Service, National Institutes of Health, Bethesda, Md., 1991), the Chothia numbering system (Chothia & Lesk (1987) J. Mol. Biol. 196:901-917; Chothia et al. (1989) Nature 342:878-883), the IMGT numbering system (Lefranc et al. al., Dev. Comparat. Immunol. 27:55-77, 2003), the AbM numbering system (Martin ACR, Cheetham JC, Rees AR (1989), Modeling antibody hypervariable loops: A combined algorithm. Proc Natl Acad Sci USA 86:9268-9272), or the Contact numbering system (MacCallum, R. M., Martin, A. C. R., & Thornton, J. M. (1996). Antibody-antigen Interactions: Contact Analysis and Binding Site Topography. Journal of Molecular Biology, 262(5), 732-745.). For a given antibody, one skilled in the art will readily identify the CDRs defined by each numbering system. Moreover, the correspondence between different numbering systems is well known to those skilled in the art (e.g., see Lefranc et al., Dev. Comparat. Immunol. 27:55-77,2003).

In the present disclosure, the CDRs contained in the antibody or antigen-binding fragment thereof of the present disclosure can be determined according to various numbering systems known in the art, such as Kabat, Chothia, IMGT, AbM or Contact numbering systems. In certain embodiments, the anti-CD3 antibody or antigen-binding fragment thereof of the present disclosure contains CDRs determined by the Kabat numbering system. In certain embodiments, the anti-MSLN single-domain antibody or antigen-binding fragment thereof of the present disclosure contains CDRs determined by the Kabat, Chothia, IMGT, AbM or Contact numbering system.

As used herein, the term "framework region" or "FR" residues refers to those amino acid residues in an antibody variable region other than the CDR residues as defined above.

The term "antibody" is not limited to any particular method of producing the antibody. It includes, for example, recombinant antibody, monoclonal antibody, and polyclonal antibody. The antibody may be of different isotypes, for example, IgG (e.g., IgG1, IgG2, IgG3 or IgG4 subtypes), IgA1, IgA2, IgD, IgE or IgM antibody.

As used herein, the term "antigen-binding fragment" of an antibody refers to a polypeptide comprising a fragment of a full-length antibody that retains the ability to specifically bind to the same antigen to which the full-length antibody binds, and/or competes with the full-length antibody for specifically binding to the antigen, which is also called an "antigen-binding moiety." See generally, Fundamental Immunology, Ch. 7 (Paul, W., ed., 2nd ed., Raven Press, N.Y. (1989), which is incorporated herein by reference in its entirety for all purposes. Antigen-binding fragments of an antibody can be obtained by recombinant DNA technology or by enzymatic or chemical cleavage of an intact antibody. Non-limiting examples of antigen-binding fragments include Fab, Fab', F(ab')₂, Fd, Fv, complementarity determining region (CDR) fragment, scFv, diabody, single domain antibody, chimeric antibody, linear antibody, nanobody (technology from Domantis), probody, and such polypeptides, which contain at least a portion of an antibody that is sufficient to confer specificity to the polypeptides with antigen-binding capability.

As used herein, the term "full-length antibody" refers to an antibody consisting of two "full-length heavy chains" and two "full-length light chains." Among them, "full-length heavy chain" refers to a polypeptide chain that consists of a heavy chain variable region (VH), a heavy chain constant region CH1 domain, a hinge region (HR), a heavy chain constant region CH2 domain, a heavy chain constant region CH3 domain in the direction from the N-terminal to the C-terminal; and, when the full-length antibody is of IgE isotype, it optionally also comprises a heavy chain constant region CH4 domain. Preferably, a "full-length heavy chain" is a polypeptide chain consisting of VH, CH1, HR, CH2 and CH3 in the direction from N-terminal to C-terminal. A "full-length light chain" is a polypeptide chain consisting of a light chain variable region (VL) and a light chain constant region (CL) in the direction from N-terminal to C-terminal. The two pairs of full-length antibody chains are linked together by disulfide bonds between CL and CH1 and disulfide bonds between the HRs of the two full-length heavy chains. The full-length antibody of the present disclosure contains two antigen-binding sites formed by VH and VL pairs respectively, and these two antigen-binding sites specifically recognize/bind the same antigen.

As used herein, the term "single-domain antibody (sdAb)" has the meaning commonly understood by those skilled in the art, which refers to an antibody fragment composed of a single monomeric variable antibody domain (e.g., a single heavy chain variable region), usually derived from the variable regions of heavy chain antibodies (e.g., camelid antibodies or shark antibodies). Typically, nanobody is composed of 4 framework regions and 3 complementarity determining regions, with the structure of FR1-CDR1-FR2-CDR2-FR3-CDR3-FR4. The single-domain antibody can be truncated at the N- or C-terminal so that it contains only a part of FR1 and/or FR4, or lacks one or two of those backbone regions, as long as it substantially retains the ability to specifically bind to antigen. The single-domain antibody is also called nanobody, and the two are used interchangeably. As used herein, the term "antigen-binding fragment" of single-domain antibody refers to a polypeptide comprising a fragment of single-domain antibody, which retains the ability to specifically bind to the same antigen to which the single-domain antibody binds, and/or competes with the single-domain antibody for specifically binding to the antigen. The antigen-binding fragment of the single-domain antibody of the present disclosure can be produced by recombinant DNA techniques or by enzymatic or chemical cleavage of the single-domain antibody of the present disclosure. In some embodiments, as compared to the full-length single-domain antibody, the "antigen-binding fragment" of the single-domain antibody can be truncated at the N- or C-terminal so that it contains only a part of FR1 and/or FR4, or lacks one or two of the backbone regions, as long as it substantially retains the ability to specifically bind to antigen.

As used herein, the term "Fab fragment" refers to an antibody fragment consisting of VL, VH, CL and CH1 domains; the term "F(ab')₂ fragment" refers to an antibody fragment consisting of two Fab fragments connected by disulfide bridges on the hinge region; the term "Fab' fragment" refers to a fragment obtained by reducing the disulfide bond connecting the two heavy chain fragments in the F(ab')₂ fragment, and consists of a complete light chain and heavy chain Fd fragment (consisting of VH and CH1 domains).

As used herein, the term "Fv" refers to an antibody fragment consisting of the VL and VH domains of a single arm of an antibody. Fv fragment is generally considered to be the smallest antibody fragment that can form a complete antigen-binding site. It is generally believed that six CDRs confer the antigen-binding specificity on an antibody. However, even a variable region (e.g., an Fd fragment, which contains only three antigen-specific CDRs) can recognize and bind the antigen, although its affinity may be lower than that of the intact binding site.

As used herein, the term "Fc domain" or "Fc region" refers to a portion of the heavy chain constant region that comprises CH2 and CH3. The Fc fragment of an antibody has many different functions but does not participate in antigen binding. "Effector functions" mediated by Fc region comprise: Fc receptor binding; Clq binding and complement-dependent cytotoxicity (CDC); antibody-dependent cell-mediated cytotoxicity (ADCC); phagocytosis; down-regulation on cell surface receptor (e.g., B-cell receptor); and B-cell activation, etc. In some embodiments, the Fc region comprises hinge, CH2, and CH3. When the Fc region comprises a hinge, the hinge mediates the dimerization between two Fc-containing polypeptides. The Fc region can be of any antibody heavy chain constant region isotype, such as IgG1, IgG2, IgG3 or IgG4.

The Fc domain may comprise either a native Fc region or a variant Fc region. Native Fc regions comprise amino acid sequences that are consistent with the amino acid sequences of Fc regions found in nature, for example, native sequence human Fc regions comprise native sequence human IgG1 Fc regions (non-A and A allotypes); native sequence human IgG2 Fc regions; native sequence human IgG3 Fc regions; native sequence human IgG4 Fc regions, and naturally occurring variants thereof. Variant Fc regions comprise amino acid sequences that differ from the amino acid sequences of native sequence Fc regions due to at least one amino acid modification. In some embodiments, variant Fc regions may possess altered effector functions (e.g., Fc receptor binding, antibody glycosylation, number of cysteine residues, effector cell function, or complement function) as compared to native Fc regions.

As used herein, the term "scFv" refers to a single polypeptide chain comprising VL and VH domains, in which the VL and VH are connected via a linker. Such scFv molecules may have the general structure: NH₂-VL-linker-VH-COOH or NHz-VH-linker-VL-COOH. Suitable linkers in the prior art consist of repeated GGGGS amino acid sequences or variants thereof. For example, a linker having the amino acid sequence (GGGGS)₂ can be used, but variants thereof can also be used. In some cases, a disulfide bond may also exist between VH and VL of scFv.

As used herein, the term "diabody" refers to an antibody whose VH and VL domains are expressed on a single polypeptide chain, but the linker used is too short to allow pairing between the two domains on the same chain, which forces the domains to pair with the complementary domains of other chain and generate two antigen-binding sites (see, for example, Holliger P. et al., Proc. Natl. Acad. Sci. USA 90:6444-6448 (1993), and Poljak R. J. et al., Structure 2:1121-1123 (1994)).

Each of the above antibody fragments retains the ability to specifically bind to the same antigen that the full-length antibody binds, and/or competes with the full-length antibody for specific binding to the antigen.

Antigen-binding fragments of antibody (e.g., the above-described antibody fragments) can be obtained from a given antibody (e.g., the antibody provided by the present disclosure) using conventional techniques known to those skilled in the art (e.g., recombinant DNA technology or enzymatic or chemical fragmentation methods), and the antigen-binding fragments of antibody are screened for specificity in the same manner as for the intact antibody.

As used herein, when the term "antibody" is mentioned, it comprises not only intact antibody but also an antigen-binding fragment of the antibody, unless the context clearly indicates otherwise.

As used herein, the term "germline antibody gene" or "germline antibody gene segment" refers to an immunoglobulin-encoding sequence present in the genome of an organism, which have not undergone the maturation process that leads to genetic rearrangements and mutations for the expression of specific immunoglobulins. In the present disclosure, the expression "heavy chain germline gene" refers to a germline antibody gene or gene fragment encoding immunoglobulin heavy chain, which comprises V gene (variable), D gene (diversity), J gene (joining), and C gene (constant); similarly, the expression "light chain germline gene" refers to an germline antibody gene or gene fragment encoding immunoglobulin light chain, which comprises V gene (variable), J gene (joining) and C gene (constant). In the present disclosure, the amino acid sequence encoded by the germline antibody gene or germline antibody gene fragment is also called "germline sequence". Germline antibody genes or germline antibody gene fragments and their corresponding germline sequences are well known to those skilled in the art and can be obtained or queried from professional databases (e.g., IMGT, UNSWIg, NCBI or VBASE2). It is generally believed that germline antibody genes are more likely than mature antibody genes to retain key amino acid sequence structures unique to individuals within a species, and therefore are relatively less likely to be considered as foreign when used therapeutically in that species.

As used herein, the term "identity" is used to refer to the match of sequences between two polypeptides or between two nucleic acids. To determine the percent identity of two amino acid sequences or two nucleic acid sequences, the sequences are aligned for optimal comparison purposes (e.g., gaps may be introduced in the first amino acid sequence or nucleic acid sequence for best alignment with the second amino acid or nucleic acid sequence). The amino acid residues or nucleotides at the corresponding amino acid positions or nucleotide positions are then compared. Molecules are identical when a position in the first sequence is occupied by the same amino acid residue or nucleotide at the corresponding position in the second sequence. The percent identity between two sequences is a function of the number of identical positions shared by the sequences (i.e., percent identity = number of identical overlapping positions/total number of positions × 100%). In certain embodiments, both sequences are of the same length.

Determination of percent identity between two sequences can also be accomplished using mathematical algorithms. One non-limiting example of mathematical algorithm for comparison of two sequences is the algorithm of Karlin and Altschul, 1990, Proc. Natl. Acad. Sci. U.S.A. 87:2264-2268, which was improved by Karlin and Altschul, 1993, Proc. Natl.. Acad. Sci. U.S.A. 90:5873-5877. Such algorithms were integrated into the NBLAST and XBLAST programs of Altschul et al., 1990, J. Mol. Biol. 215:403.

As used herein, the term "variant", in the context of polypeptides (including polypeptides), also refers to a polypeptide or peptide comprising an amino acid sequence that has been altered by introducing a substitution, deletion, or addition of amino acid residues. In some cases, the term "variant" also refers to a polypeptide or peptide that has been modified (i.e., by covalently linking any type of molecule to the polypeptide or peptide). For example, and without limitation, polypeptides may be modified, for example, by glycosylation, acetylation, PEGylation, phosphorylation, amidation, derivatization by known protecting/blocking groups, proteolytic cleavage, attachment to cellular ligand or other proteins, etc. Derivatized polypeptides or peptides can be produced by chemical modification using techniques known to those skilled in the art, including, but not limited to, specific chemical cleavage, acetylation, formylation, metabolic synthesis of tunicamycin, and the like. Furthermore, a variant has a similar, identical or improved function to the polypeptide or peptide from which it is derived. In certain embodiments, the variant has a sequence identity of at least 90%, at least 91%, at least 92%, at least 93%, at least 94%, at least 95%, at least 96%, at least 97 %, at least 98%, at least 99%, or 100% as compared to the sequence from which it is derived.

As used herein, the term "specific binding" refers to a non-random binding reaction between two molecules, such as the reaction between an antibody and the antigen to which is directed. The strength or affinity of a specific binding interaction can be expressed by the equilibrium dissociation constant (K_{D}) of the interaction. In the present disclosure, the term "K_{D}" refers to the dissociation equilibrium constant of a specific antibody-antigen interaction, which is used to describe the binding affinity between an antibody and an antigen. The smaller the equilibrium dissociation constant, the tighter the antibody-antigen binding, and the higher the affinity between the antibody and the antigen.

The specific binding properties between two molecules can be determined using methods known in the art. One approach involves measuring the rate at which antigen binding site/antigen complex forms and dissociates. Both the "association rate constant" (ka or kon) and the "dissociation rate constant" (kdis or koff) can be calculated from concentrations and actual rates of association and dissociation (see Malinqvist M, Nature, 1993, 361 :186-187). The ratio kdis/kon is equal to the dissociation constant K_{D} (see Davies et al., Annual Rev Biochem, 1990; 59:439-473). K_{D}, kon and kdis values can be measured by any valid method. In certain embodiments, dissociation constants can be measured in Biacore using surface plasmon resonance (SPR). Alternatively, bioluminescence interferometry or Kinexa can be used to measure dissociation constants.

As used herein, the term "vector" refers to a nucleic acid delivery vehicle into which a polynucleotide can be inserted. When the vector can express the protein encoded by the inserted polynucleotide, the vector is called an expression vector. The vector can be introduced into a host cell through transformation, transduction or transfection, so that the genetic material elements it carries can be expressed in the host cell. Vectors are well known to those skilled in the art, including but not limited to: plasmids; phagemids; cosmids; artificial chromosomes, such as yeast artificial chromosomes (YAC), bacterial artificial chromosomes (BAC) or P1-derived artificial chromosomes (PAC); phages, such as λ phage or M13 phage and animal viruses, etc. Animal viruses that can be used as vectors include, but are not limited to, retroviruses (including lentiviruses), adenoviruses, adeno-associated viruses, herpesviruses (e.g., herpes simplex virus), poxviruses, baculoviruses, papillomaviruses, papovaviruses (e.g., SV40). A vector can contain a variety of expression-controlling elements, including, but not limited to, promoter sequence, transcription initiation sequence, enhancer sequence, selection element, and reporter gene. In addition, the vector may also contain an origin of replication site.

As used herein, the term "host cell" refers to a cell that can be used to introduce a vector, which includes, but is not limited to, prokaryotic cell such as *E. coli* or *Bacillus subtilis,* fungal cell such as yeast cell or *Aspergillus,* insect cell such as *S2 Drosophila* cell or Sf9, or animal cell such as fibroblast, CHO cell, COS cell, NSO cell, HeLa cell, BHK cell, HEK 293 cell or human cell.

As used herein, the term "conservative substitution" refers to an amino acid substitution that does not adversely affect or alter the expected properties of the protein/polypeptide comprising the amino acid sequence. For example, conservative substitutions can be introduced by standard techniques known in the art, such as site-directed mutagenesis and PCR-mediated mutagenesis. Conservative amino acid substitutions include those in which an amino acid residue is replaced with an amino acid residue having a similar side chain, for example, one that is physically or functionally similar to the corresponding amino acid residue (e.g., having similar size, shape, charge, chemical properties, including ability to form covalent bonds or hydrogen bonds, etc.). Families of amino acid residues with similar side chains have been defined in the art. These families include amino acids with basic side chains (e.g., lysine, arginine, and histidine), acidic side chains (e.g., aspartic acid, glutamic acid), uncharged polar side chains (e.g., glycine, asparagine, glutamine, serine, threonine, tyrosine, cysteine, tryptophan), non-polar side chains (e.g., alanine, valine, leucine, isoleucine, proline, phenylalanine, methionine), β-branched side chains (e.g., threonine, valine, isoleucine) and aromatic side chains (e.g., tyrosine, phenylalanine, tryptophan, histidine). Therefore, it is preferred to replace the corresponding amino acid residue with another amino acid residue from the same side chain family. In addition, amino acid residues can be divided into categories defined by optional physical and functional properties, for example, alcohol-containing residues (S and T), aliphatic residues (I, L, V, and M), cycloalkenyl-related residues (F, H, W, and Y), hydrophobic residues (A, C, F, G, H, I, L, M, R, T, V, W, and Y), negatively charged residues (D and E), polar residues (C, D, E, H, K, N, Q, R, S and T), positively charged residues (H, K and R), small residues (A, C, D, G, N, P, S, T and V), very small residues (A, G and S), residues involved corner formation (A, C, D, E, G, H, K, N, Q, R, S, P and T), flexible residues (Q, T, K, S, G, P, D, E and R). Methods for identifying conservative substitutions of amino acids are well known in the art (see, for example, Brummell et al., Biochem. 32:1180-1187 (1993); Kobayashi et al., Protein Eng. 12(10):879-884 (1999); and Burks et al. Proc. Natl Acad. Set USA 94:412-417 (1997), which are incorporated herein by reference).

The twenty conventional amino acids involved herein have been written in accordance with conventional usage. See, for example, Immunology-A Synthesis (2nd Edition, E. S. Golub and D. R. Gren, Eds., Sinauer Associates, Sunderland, Mass. (1991)), which is incorporated herein by reference. In the present disclosure, the terms "polypeptide" and "protein" have the same meaning and are used interchangeably. And in the present disclosure, amino acids are generally represented by one-letter and three-letter abbreviations well known in the art. For example, alanine can be represented by A or Ala.

As used herein, the term "pharmaceutically acceptable carrier and/or excipient" refers to a carrier and/or excipient that is pharmacologically and/or physiologically compatible with a subject and an active ingredient, and they are well known in the art (see, for example, Remington's Pharmaceutical Sciences. Edited by Gennaro AR, 19th ed. Pennsylvania: Mack Publishing Company, 1995), including, but not limited to: pH adjusting agent, surfactant, adjuvant, ionic strength enhancer, diluent, agent for maintaining osmotic pressure, agent for delaying absorption, preservative. For example, the pH adjusting agent includes, but is not limited to, phosphate buffer. The surfactant includes, but is not limited to, cationic, anionic or nonionic surfactant, such as Tween-80. The ionic strength enhancer includes, but is not limited to, sodium chloride. The agent for maintaining osmotic pressure includes, but is not limited to, sugar, NaCl, and the like. The agent for delaying absorption includes, but is not limited to, monostearate and gelatin. The diluent includes, but is not limited to, water, aqueous buffer (e.g., buffered saline), alcohol and polyol (e.g., glycerol), and the like. The preservative includes, but is not limited to, various antibacterial and antifungal agents, such as thimerosal, 2-phenoxyethanol, paraben, chlorobutanol, phenol, sorbic acid, etc. Stabilizers have the meaning generally understood by those skilled in the art, which can stabilize a desired activity of an active ingredient in medicines, including but not limited to sodium glutamate, gelatin, SPGA, saccharide (e.g., sorbitol, mannitol, starch, sucrose, lactose, dextran, or glucose), amino acid (e.g., glutamic acid, glycine), protein (e.g., dry whey, albumin or casein) or degradation product thereof (e.g., lactalbumin hydrolyzate), etc.

As used herein, the term "prevention" refers to a method performed to prevent or delay the occurrence of a disease or condition or symptom in a subject. As used herein, the term "treatment" refers to a method performed to obtain a beneficial or desired clinical result. For the purposes of the present disclosure, beneficial or desired clinical results include, but are not limited to, alleviation of symptoms, reduction of the extent of disease, stabilization (i.e., no worsening) of the state of disease, delaying or slowing the progression of disease, ameliorating or alleviating the status of disease, and relief of symptoms (whether partial or complete), whether detectable or undetectable. In addition, "treatment" may also refer to prolonging survival compared to expected survival if not receiving treatment.

As used herein, the term "subject" refers to a mammal, such as a primate mammal, for example, a human. In certain embodiments, the subject (e.g., human) has, or is at risk for, a tumor (e.g., an MSLN-expressing tumor), an inflammatory disease, or an autoimmune disease.

As used herein, the term "effective amount" refers to an amount sufficient to achieve, or at least partially achieve, the desired effect. For example, an effective amount for preventing a disease refers to an amount that is sufficient to prevent, arrest or delay the occurrence of the disease (e.g., a tumor, an inflammatory disease, or an autoimmune disease); the effective amount for treating a disease refers to an amount that is sufficient to cure or at least partially prevent the disease and its complications in a patient who already has the disease. Determining such effective amounts is well within the capabilities of those skilled in the art. For example, the amount effective for therapeutic use will depend on the severity of the disease to be treated, the overall status of the patient's own immune system, the patient's general condition such as age, weight and gender, the manner in which the drug is administered, and other treatments administered concurrently, an so on.

### Beneficial effects of the present disclosure

The present disclosure provides novel CD3 antibodies with reduced cytokine release, indicating their ability to moderately activate T cells. The present disclosure also provides multispecific antibodies targeting CD3 and additional antigens (e.g., tumor-associated antigens and/or immune checkpoint molecules), such as bispecific antibodies targeting CD3 and MSLN. The bispecific antibodies can bind CD3 present on T cells and specific target antigens on tumor cells to promote the targeting and recruitment of T cells to tumor cells, thereby inducing tumor-specific (MHC-independent) killing activity of cytotoxic T cells, significantly reducing the release of cell factors, and resulting in significantly improved safety. Therefore, the antibodies of the present disclosure have important clinical value.

The embodiments of the present disclosure will be described in detail below with reference to the drawings and examples, but those skilled in the art will understand that the drawings and examples are only used to illustrate the present disclosure and do not limit the scope of the present disclosure. Various objects and advantageous aspects of the present disclosure will become apparent to those skilled in the art from the following detailed description of the accompanying drawings and examples.

### Sequence information

A description of the sequences covered by the present application is provided in the table below.

**Table 1: Sequence information**

| SEQ ID NO: | Description | SEQ ID NO: | Description |
|---|---|---|---|
| 1 | CD3 antibody VH-1 | 89 | HCDR3 (VH45) |
| 2 | CD3 antibody VH-2 | 90 | HCDR3 |
| 3 | CD3 antibody VH-3 | 91 | HFR3 (VH1) |
| 4 | CD3 antibody VH-4 | 92 | HFR3 (VH2/VH6/VH21) |
| 5 | CD3 antibody VH-5 | 93 | HFR3 (VH3/VH17/VH25/VH28/VH30/VH43/VH46) |
| 6 | CD3 antibody VH-6 | 94 | HFR3 (VH4) |
| 7 | CD3 antibody VH-7 | 95 | HFR3 (VH5/VH19) |
| 8 | CD3 antibody VH-8 | 96 | HFR3 (VH7/VH8/VH39/VH40/VH44/VH45) |
| 9 | CD3 antibody VH-9 | 97 | HFR3 (VH9/VH22/VH23/VH27/VH29) |
| 10 | CD3 antibody VH-10 | 98 | HFR3 (VH10/VH11/VH20) |
| 11 | CD3 antibody VH-11 | 99 | HFR3 (VH12/VH41/VH42) |
| 12 | CD3 antibody VH-12 | 100 | HFR3 (VH13/VH32/VH33) |
| 13 | CD3 antibody VH-13 | 101 | HFR3 (VH14/VH15/VH18/VH36) |
| 14 | CD3 antibody VH-14 | 102 | HFR3 (VH16/VH24) |
| 15 | CD3 antibody VH-15 | 103 | HFR3 (VH26/VH37) |
| 16 | CD3 antibody VH-16 | 104 | HFR3 (VH31) |
| 17 | CD3 antibody VH-17 | 105 | HFR3 (VH34) |
| 18 | CD3 antibody VH-18 | 106 | HFR3 (VH35/VH38) |
| 19 | CD3 antibody VH-19 | 107 | HCDR1 |
| 20 | CD3 antibody VH-20 | 108 | HCDR2 |
| 21 | CD3 antibody VH-21 | 109 | HFR1 |
| 22 | CD3 antibody VH-22 | 110 | HFR2 |
| 23 | CD3 antibody VH-23 | 111 | HFR4 |
| 24 | CD3 antibody VH-24 | 112 | HCDR3 general formula |
| 25 | CD3 antibody VH-25 | 113 | HCDR3 general formula |
| 26 | CD3 antibody VH-26 | 114 | HCDR3 general formula |
| 27 | CD3 antibody VH-27 | 115 | HCDR3 general formula |
| 28 | CD3 antibody VH-28 | 116 | HCDR3 general formula |
| 29 | CD3 antibody VH-29 | 117 | HCDR3 general formula |
| 30 | CD3 antibody VH-30 | 118 | HCDR3 general formula |
| 31 | CD3 antibody VH-31 | 119 | HCDR3 general formula |
| 32 | CD3 antibody VH-32 | 120 | HCDR3 general formula |
| 33 | CD3 antibody VH-33 | 121 | HCDR3 general formula |
| 34 | CD3 antibody VH-34 | 122 | HCDR3 general formula |
| 35 | CD3 antibody VH-35 | 123 | HCDR3 general formula |
| 36 | CD3 antibody VH-36 | 124 | HCDR3 general formula |
| 37 | CD3 antibody VH-37 | 125 | HCDR3 general formula |
| 38 | CD3 antibody VH-38 | 126 | HCDR3 general formula |
| 39 | CD3 antibody VH-39 | 127 | HCDR3 general formula |
| 40 | CD3 antibody VH-40 | 128 | HCDR3 general formula |
| 41 | CD3 antibody VH-41 | 129 | HCDR3 general formula |
| 42 | CD3 antibody VH-42 | 130 | HCDR3 general formula |
| 43 | CD3 antibody VH-43 | 131 | HCDR3 general formula |
| 44 | CD3 antibody VH-44 | 132 | HCDR3 general formula |
| 45 | CD3 antibody VH-45 | 133 | HCDR3 general formula |
| 46 | CD3 antibody VH-46 | 134 | HCDR3 general formula |
| 47 | CD3 antibody VL-1 | 135 | HCDR3 general formula |
| 48 | CD3 antibody VL-2 | 136 | HFR3 general formula |
| 49 | HCDR3 (VH1) | 137 | LCDR1 (VL1/VL2) |
| 50 | HCDR3 (VH2) | 138 | LCDR2 (VL1/VL2) |
| 51 | HCDR3 (VH3) | 139 | LCDR3 (VL1/VL2) |
| 52 | HCDR3 (VH4/VH42) | 140 | LFR1 (VL1/VL2) |
| 53 | HCDR3 (VH5) | 141 | LFR4 (VL1/VL2) |
| 54 | HCDR3 (VH6) | 142 | LFR2 (VL1) |
| 55 | HCDR3 (VH7) | 143 | LFR2 (VL2) |
| 56 | HCDR3 (VH8) | 144 | LFR2 general formula |
| 57 | HCDR3 (VH9) | 145 | LFR3 (VL1) |
| 58 | HCDR3 (VH10) | 146 | LFR3 (VL2) |
| 59 | HCDR3 (VH11) | 147 | LFR3 general formula |
| 60 | HCDR3 (VH12/VH36) | 148 | HCDR3 general formula |
| 61 | HCDR3 (VH13) | 149 | MSLN VHH |
| 62 | HCDR3 (VH14/VH31) | 150 | MSLN VHH-CDR1(IMGT) |
| 63 | HCDR3 (VH15) | 151 | MSLN VHH-CDR2(IMGT) |
| 64 | HCDR3 (VH16/VH20) | 152 | MSLN VHH-CDR3(IMGT) |
| 65 | HCDR3 (VH17) | 153 | MSLN VHH-CDR1(Kabat) |
| 66 | HCDR3 (VH18) | 154 | MSLN VHH-CDR2(Kabat) |
| 67 | HCDR3 (VH19) | 155 | MSLN VHH-CDR3(Kabat/ AbM/ Chothia) |
| 68 | HCDR3 (VH21) | 156 | MSLN VHH-CDR1(AbM) |
| 69 | HCDR3 (VH22) | 157 | MSLN VHH-CDR2(AbM) |
| 70 | HCDR3 (VH23) | 158 | MSLN VHH-CDR1 (Chothia) |
| 71 | HCDR3 (VH24) | 159 | MSLN VHH-CDR2(Chothia) |
| 72 | HCDR3 (VH25) | 160 | MSLN VHH-CDR1 (Contact) |
| 73 | HCDR3 (VH26) | 161 | MSLN VHH-CDR2(Contact) |
| 74 | HCDR3 (VH27/VH46) | 162 | MSLN VHH-CDR3(Contact) |
| 75 | HCDR3 (VH28) | 163 | Linker |
| 76 | HCDR3 (VH29) | 164 | IgG1 heavy chain constant region |
| 77 | HCDR3 (VH30) | 165 | Light chain constant region |
| 78 | HCDR3 (VH32) | 166 | Positive control antibody OKT3-VH |
| 79 | HCDR3 (VH33) | 167 | Positive control antibody OKT3-VL |
| 80 | HCDR3 (VH34) | 168 | Control antibody 1-VH |
| 81 | HCDR3 (VH35) | 169 | Control antibody 1-VL |
| 82 | HCDR3 (VH37) | 170 | Anti-CD19 VH |
| 83 | HCDR3 (VH38) | 171 | Anti-CD19 VL |
| 84 | HCDR3 (VH39) | 172 | Anti-CD20 VH |
| 85 | HCDR3 (VH40) | 173 | Anti-CD20 VL |
| 86 | HCDR3 (VH41) | 174 | Anti-trop2 VHH |
| 87 | HCDR3 (VH43) | 175 | Anti-Her2 VHH |
| 88 | HCDR3 (VH44) | 176 | Anti-Caludin18.2 VHH |

Unless otherwise specified, the molecular biology experimental methods and immunoassay methods used in the present disclosure are performed by basically referring to the methods described by J. Sambrook et al., Molecular Cloning: Laboratory Manual, 2nd Edition, Cold Spring Harbor Laboratory Press, 1989, and F. M. Ausubel et al., Compiled Experimental Guide to Molecular Biology, 3rd Edition, John Wiley & Sons, Inc., 1995; the restriction enzymes are used in accordance with the conditions recommended by the product manufacturers. Those skilled in the art will appreciate that the examples describe the present disclosure by way of example and are not intended to limit the scope sought to be protected by the present disclosure.

### Example 1: Construction of CD3 monoclonal antibodies

In this example, mutations were introduced into the heavy chain variable region and light chain variable region of CD3 monoclonal antibody to establish a scFv phage mutation library. The heavy chain variable regions of the constructed CD3 monoclonal antibodies were named as VH1 to VH46, which had the amino acid sequences as set forth in SEQ ID NOs: 1-46, respectively. The light chain variable regions of the constructed CD3 monoclonal antibodies were named as VL-1 to VL-2, which had the amino acid sequences as set forth in SEQ ID NOs: 47 and 48, respectively. The CDR information of each variable region was shown in Table 1 and Table 2. The constructed VH1 to VH46 were paired with VL-1 respectively to establish an scFv phage mutation library, and construct CD3 monoclonal antibodies. The specific structure of the CD3 monoclonal antibody was an IgG antibody, the VH of the CD3 monoclonal antibody was connected to the IgG1 mutant heavy chain constant region (SEQ ID NO: 164) to form a structure shown as [VH]-[CH]; and the VL-1 of the CD3 monoclonal antibody was connected to the light chain constant region (SEQ ID NO: 165) to form a structure shown as [VL]-[CL]. For convenience of writing, the CD3 monoclonal antibody containing VH1 and VL-1 was named as M1, the CD3 monoclonal antibody containing VH2 and VL-1 was named as M2, and so on, and thus a total of 46 CD3 monoclonal antibodies were named as M1 to M46. Coating CD3εγ (Company: acrobiosystems, Cat. No.: CDG-H5253) was carried out, the packaged phages were added, incubated at room temperature for 1 hour, washed 10 times, dried by patting, then the phages were eluted, the eluted phage infected TG1 competent cells overnight, and the phages were collected; the previous operations were repeated twice; a single colony was picked, and subjected to IPTG induction and expression overnight. Positive clones were determined by ELISA method.

**Table 2: CDR sequences of CD3 monoclonal antibodies**

| Name of variable region | SEQ ID NO: | | | | | | | |
|---|---|---|---|---|---|---|---|---|
| | Full length | FR1 | CDR1 | FR2 | CDR2 | FR3 | CDR3 | FR4 |
| VH1 | 1 | 109 | 107 | 110 | 108 | 91 | 49 | 111 |
| VH2 | 2 | 109 | 107 | 110 | 108 | 92 | 50 | 111 |
| VH3 | 3 | 109 | 107 | 110 | 108 | 93 | 51 | 111 |
| VH4 | 4 | 109 | 107 | 110 | 108 | 94 | 52 | 111 |
| VH5 | 5 | 109 | 107 | 110 | 108 | 95 | 53 | 111 |
| VH6 | 6 | 109 | 107 | 110 | 108 | 92 | 54 | 111 |
| VH7 | 7 | 109 | 107 | 110 | 108 | 96 | 55 | 111 |
| VH8 | 8 | 109 | 107 | 110 | 108 | 96 | 56 | 111 |
| VH9 | 9 | 109 | 107 | 110 | 108 | 97 | 57 | 111 |
| VH10 | 10 | 109 | 107 | 110 | 108 | 98 | 58 | 111 |
| VH11 | 11 | 109 | 107 | 110 | 108 | 98 | 59 | 111 |
| VH12 | 12 | 109 | 107 | 110 | 108 | 99 | 60 | 111 |
| VH13 | 13 | 109 | 107 | 110 | 108 | 100 | 61 | 111 |
| VH14 | 14 | 109 | 107 | 110 | 108 | 101 | 62 | 111 |
| VH15 | 15 | 109 | 107 | 110 | 108 | 101 | 63 | 111 |
| VH16 | 16 | 109 | 107 | 110 | 108 | 102 | 64 | 111 |
| VH17 | 17 | 109 | 107 | 110 | 108 | 93 | 65 | 111 |
| VH18 | 18 | 109 | 107 | 110 | 108 | 101 | 66 | 111 |
| VH19 | 19 | 109 | 107 | 110 | 108 | 95 | 67 | 111 |
| VH20 | 20 | 109 | 107 | 110 | 108 | 98 | 64 | 111 |
| VH21 | 21 | 109 | 107 | 110 | 108 | 92 | 68 | 111 |
| VH22 | 22 | 109 | 107 | 110 | 108 | 97 | 69 | 111 |
| VH23 | 23 | 109 | 107 | 110 | 108 | 97 | 70 | 111 |
| VH24 | 24 | 109 | 107 | 110 | 108 | 102 | 71 | 111 |
| VH25 | 25 | 109 | 107 | 110 | 108 | 93 | 72 | 111 |
| VH26 | 26 | 109 | 107 | 110 | 108 | 103 | 73 | 111 |
| VH27 | 27 | 109 | 107 | 110 | 108 | 97 | 74 | 111 |
| VH28 | 28 | 109 | 107 | 110 | 108 | 93 | 75 | 111 |
| VH29 | 29 | 109 | 107 | 110 | 108 | 97 | 76 | 111 |
| VH30 | 30 | 109 | 107 | 110 | 108 | 93 | 77 | 111 |
| VH31 | 31 | 109 | 107 | 110 | 108 | 104 | 62 | 111 |
| VH32 | 32 | 109 | 107 | 110 | 108 | 100 | 78 | 111 |
| VH33 | 33 | 109 | 107 | 110 | 108 | 100 | 79 | 111 |
| VH34 | 34 | 109 | 107 | 110 | 108 | 105 | 80 | 111 |
| VH35 | 35 | 109 | 107 | 110 | 108 | 106 | 81 | 111 |
| VH36 | 36 | 109 | 107 | 110 | 108 | 101 | 60 | 111 |
| VH37 | 37 | 109 | 107 | 110 | 108 | 103 | 82 | 111 |
| VH38 | 38 | 109 | 107 | 110 | 108 | 106 | 83 | 111 |
| VH39 | 39 | 109 | 107 | 110 | 108 | 96 | 84 | 111 |
| VH40 | 40 | 109 | 107 | 110 | 108 | 96 | 85 | 111 |
| VH41 | 41 | 109 | 107 | 110 | 108 | 99 | 86 | 111 |
| VH42 | 42 | 109 | 107 | 110 | 108 | 99 | 52 | 111 |
| VH43 | 43 | 109 | 107 | 110 | 108 | 93 | 87 | 111 |
| VH44 | 44 | 109 | 107 | 110 | 108 | 96 | 88 | 111 |
| VH45 | 45 | 109 | 107 | 110 | 108 | 96 | 89 | 111 |
| VH46 | 46 | 109 | 107 | 110 | 108 | 93 | 74 | 111 |
| VL1 | 47 | 140 | 137 | 142 | 138 | 145 | 139 | 141 |
| VL2 | 48 | 140 | 137 | 143 | 138 | 146 | 139 | 141 |

### Example 2: Purity determination of CD3 monoclonal antibodies

100 µl of each of CD3 monoclonal antibodies M1 to M46 was transferred to a 1.5 ml centrifuge tube, centrifuged at 12000 rpm for 5 min, then 90 µl of sample was taken and transferred to a liquid phase injection bottle, and loaded on a machine (HPLC, Shimadzu, 2030C) for detection. The results were shown in Table 3. It could be seen from Table 3 that the SEC purity values were all greater than 90%, and the in vitro efficacy experiment could be continued.

**Table 3: Purity determination results of CD3 monoclonal antibodies**

| Antibody name | M1 | M2 | M3 | M4 | M5 | M6 | M7 | M8 | M9 | M10 |
|---|---|---|---|---|---|---|---|---|---|---|
| SEC% | 95.5 | 97.1 | 93.5 | 98.2 | 96.1 | 95.2 | 95.3 | 98.9 | 92.7 | 94.6 |

| Antibody name | M11 | M12 | M13 | M14 | M15 | M16 | M17 | M18 | M19 | M20 |
|---|---|---|---|---|---|---|---|---|---|---|
| SEC% | 99.2 | 92.8 | 96.2 | 97.2 | 95.1 | 95.4 | 95.1 | 96.5 | 92.9 | 96.4 |

| Antibody name | M21 | M22 | M23 | M24 | M25 | M26 | M27 | M28 | M29 | M30 |
|---|---|---|---|---|---|---|---|---|---|---|
| SEC% | 97.3 | 96.2 | 96.7 | 95.2 | 95.6 | 95.4 | 95.8 | 97.5 | 94.2 | 93.7 |

| Antibody name | M31 | M32 | M33 | M34 | M35 | M36 | M37 | M38 | M39 | M40 |
|---|---|---|---|---|---|---|---|---|---|---|
| SEC% | 95.8 | 93.7 | 94.8 | 96.1 | 96.2 | 96.7 | 95.1 | 97.5 | 94.3 | 97.1 |

| Antibody name | M41 | M42 | M43 | M44 | M45 | M46 | | | | |
|---|---|---|---|---|---|---|---|---|---|---|
| SEC% | 96.7 | 94.2 | 93.8 | 97.6 | 95.5 | 94.8 | | | | |

### Example 3: Affinity detection of CD3 monoclonal antibodies

In this example, the binding activity of CD3 monoclonal antibodies to Jurkat cells (naturally expressing human CD3) (Company: ATCC; Cat. No.: TIB-152) and cynomolgus HSC-F cells (expressing monkey CD3) (Company: JCRB Cell Bank; Cat. No.: JCRB1164) was investigated.
(1) Preparation of cells: Jurkat and HSC-F cells were adjusted to 1*10⁷ cells/ml with PBS respectively, and 50 µl of cell suspension was prepared for each sample.
(2) Preparation of antibodies: PBS was used to adjust the antibodies to a final concentration of 2 µg/ml, then 3-fold gradient dilution was carried out to obtain 8 gradients. 50 µl of antibody was prepared for each well.
(3) Incubation of cells and antibodies: 50 µl of the cell suspension was taken, that was, the total number of cells was 5*10⁵ cells/well. The final concentration of the antibody was the starting concentration of 2 µg/ml, 3-fold gradient dilution was carried out in 8 wells, and incubation was performed at 4°C for 1 hour.
(4) After centrifugation at 500g for 5min, the supernatant was gently removed, and washing was performed twice by adding 200µl of PBS.
(5) Addition of secondary antibody: Fluorescent secondary antibody diluted in PBS (used at 1:100) was added, and incubated at 4°C for 30min.
(6) The fluorescent secondary antibody was rinsed out, centrifugation was carried out at 500g for 5 min, the supernatant was gently removed, washing was performed once by adding 200µl of PBS, and then upflow detection was carried out.

Two positive control antibodies were used in this experiment. For control antibody OKT3, its VH amino acid sequence was set forth in SEQ ID NO: 166, and its VL amino acid sequence was set forth in SEQ ID NO: 167; while for control antibody 1, its VH amino acid sequence was set forth in SEQ ID NO: 168, and its VL amino acid sequence was set forth in SEQ ID NO: 169.

The affinity detection results were shown in Table 4.

**Table 4: Affinity detection results of CD3 monoclonal antibodies**

| Antibody name | Human CD3-expressing Jurkat cells, (EC₅₀) nM | Monkey CD3-expressing cynomolgus HSC-F cells, (EC₅₀) nM |
|---|---|---|
| M1 | 0.5806 | 0.1174 |
| M2 | 0.2145 | 0.2245 |
| M3 | 0.2609 | 0.3145 |
| M4 | 0.4015 | 0.4337 |
| M5 | 0.1543 | 0.1517 |
| M6 | 0.1694 | 0.1716 |
| M7 | 1.7142 | 0.6898 |
| M8 | 0.2675 | 0.1859 |
| M9 | 1.5102 | 0.2369 |
| M10 | 1.23 | 0.1939 |
| M11 | 0.1303 | 0.1688 |
| M12 | 0.2695 | 0.1621 |
| M13 | 0.8324 | 0.2764 |
| M14 | 1.5066 | 0.271 |
| M15 | 0.1657 | 0.1185 |
| M16 | 0.4775 | 0.1618 |
| M17 | 0.1814 | 0.2099 |
| M18 | 0.5146 | 0.8413 |
| M19 | 0.1901 | 0.2379 |
| M20 | 61.73 | 6.583 |
| M21 | 41.58 | 1.2313 |
| M22 | 2.5476 | 2.3068 |
| M23 | 0.1864 | 0.1639 |
| M24 | 1.3187 | 0.1198 |
| M25 | 0.0954 | 0.1112 |
| M26 | 0.2353 | 0.2234 |
| M27 | 0.4025 | 0.1647 |
| M28 | 0.8541 | 0.2866 |
| M29 | 0.4123 | 0.2718 |
| M30 | 0.3017 | 0.3475 |
| M31 | 1.559 | 0.1538 |
| M32 | 0.1881 | 0.06673 |
| M33 | 4.9185 | 1.5472 |
| M34 | 2.5816 | 0.2662 |
| M35 | 0.7919 | 0.2068 |
| M36 | 0.1981 | 0.1092 |
| M37 | 0.2113 | 0.3977 |
| M38 | 0.1761 | 0.1608 |
| M39 | 0.7595 | 0.4982 |
| M40 | 3.406 | 0.4071 |
| M41 | 0.7515 | 0.1299 |
| M42 | 0.4309 | 0.1432 |
| M43 | 10.16 | 8.2374 |
| M44 | 1.6097 | 0.1413 |
| M45 | 0.4153 | 0.1558 |
| M46 | 0.3383 | 0.1484 |
| OKT3 | 0.0618 | Not binding |
| Control antibody 1 | 0.4735 | 0.4874 |

### Example 4: Dynamic affinity determination of CD3 monoclonal antibodies

Antibodies at three concentrations of 4, 2, and 1 µg/mL were immobilized on the HC200M chip containing anti-human IgG Fc secondary antibody (Xantec, Cat. No.: HC200M), and the antibody binding amount was controlled to approximately 1000RU. After the baseline was stable, the serially diluted human CD3εγ (Company: acrobiosystems, Cat. No.: CDG-H52W5) recombinant antigen (starting from 592.5nM, 3-fold dilution, 8 gradients) flowed through the chip at a flow rate of 1000 µE/min, the binding time was 8 minutes, and the dissociation time was 20 minutes. The kinetic constants were obtained by fitting using the 1:1 binding model of Kinetics software from Carterra.

The results were shown in Table 5. It could be seen from Table 5 that the CD3 monoclonal antibodies of the present disclosure have affinity for human CD3εγ antigen.

**Table 5: Dynamic affinity determination results of CD3 monoclonal antibodies**

| Antibody name | Kon (M-1 s-1) | Koff (s-1) | KD (M) |
|---|---|---|---|
| M1 | 4.09E+06 | 1.05E-02 | 2.57E-09 |
| M2 | 5.50E+05 | 3.63E-03 | 6.60E-09 |
| M3 | 1.10E+06 | 1.50E-03 | 1.36E-09 |
| M4 | 1.52E+06 | 5.17E-03 | 3.40E-09 |
| M5 | 1.74E+06 | 1.01E-02 | 5.84E-09 |
| M6 | 9.73E+05 | 3.54E-03 | 3.63E-09 |
| M7 | 4.60E+05 | 1.10E-03 | 2.39E-09 |
| M8 | 6.20E+05 | 3.20E-03 | 5.16E-09 |
| M9 | 1.30E+06 | 2.50E-03 | 1.92E-09 |
| M10 | 3.04E+06 | 6.53E-03 | 2.15E-09 |
| M11 | 2.30E+06 | 6.77E-03 | 2.95E-09 |
| M12 | 3.97E+06 | 7.68E-03 | 1.93E-09 |
| M13 | 1.35E+05 | 4.50E-04 | 3.33E-09 |
| M14 | 2.05E+06 | 1.28E-03 | 6.24E-10 |
| M15 | NA | NA | 1.60E-07 |
| M16 | 9.29E+05 | 1.88E-03 | 2.02E-09 |
| M17 | 4.29E+05 | 1.08E-03 | 2.52E-09 |
| M18 | NA | NA | 1.79E-07 |
| M19 | 1.63E+06 | 2.56E-03 | 1.57E-09 |
| M20 | 9.08E+05 | 2.32E-03 | 2.56E-09 |
| M21 | 1.75E+06 | 6.49E-03 | 3.71E-09 |
| M22 | 6.66E+05 | 2.22E-03 | 3.33E-09 |
| M23 | 3.50E+06 | 5.49E-03 | 1.57E-09 |
| M24 | 3.38E+06 | 3.58E-03 | 1.06E-09 |
| M25 | 9.10E+05 | 4.80E-03 | 5.27E-09 |
| M26 | 1.55E+06 | 5.43E-03 | 3.51E-09 |
| M27 | 1.15E+06 | 2.80E-03 | 2.44E-09 |
| M28 | 9.38E+05 | 2.37E-03 | 2.53E-09 |
| M29 | 1.32E+06 | 7.76E-03 | 5.88E-09 |
| M30 | 7.98E+05 | 2.34E-03 | 2.93E-09 |
| M31 | 2.92E+06 | 1.26E-03 | 4.30E-10 |
| M32 | 3.05E+06 | 1.33E-02 | 4.37E-09 |
| M33 | 2.80E+05 | 5.40E-03 | 1.93E-08 |
| M34 | 4.71E+05 | 3.40E-03 | 7.22E-09 |
| M35 | 3.36E+06 | 2.83E-03 | 8.42E-10 |
| M36 | 6.46E+06 | 1.09E-02 | 1.69E-09 |
| M37 | 8.10E+05 | 4.30E-03 | 5.31E-09 |
| M38 | 1.15E+06 | 2.68E-03 | 2.33E-09 |
| M39 | 1.02E+06 | 3.05E-02 | 2.97E-08 |
| M40 | 4.31E+05 | 5.50E-04 | 1.28E-09 |
| M41 | 3.38E+06 | 5.13E-03 | 1.52E-09 |
| M42 | 1.19E+06 | 1.37E-03 | 1.15E-09 |
| M43 | 1.20E+05 | 1.60E-03 | 1.33E-08 |
| M44 | 1.00E+06 | 3.34E-03 | 3.33E-09 |
| M45 | 1.85E+06 | 3.44E-03 | 1.87E-09 |
| M46 | 9.99E+05 | 2.05E-03 | 2.05E-09 |

### Example 5: Construction of human MSLN stably transfected cell line

A construct containing the full-length sequence of human MSLN (huMSLN) (cDNA purchased from Sino biological, HG13128-UT) was cloned into a lentiviral vector. The constructed lentiviral plasmid and packaging plasmid were co-transfected into HEK293T cells, and the cell supernatant was collected at 48h and 72h, respectively. The supernatants were added successively to MC38 cells (Nanjing Kebai, Cat. No.: CBP60825), and 4 µg/ml puromycin was added after 24 hours for screening. Single cells with high expression of huMSLN were sorted by a cell sorter (Sony, LE-SH800SBP), and finally the stably transfected monoclonal cell MC38/MSLN with high expression of huMSLN was obtained.

### Example 6: Immunization of alpaca

Anti-huMSLN nanobodies were generated by immunizing alpacas. Alpaca immunization was entrusted to Apak Biotechnology Co., Ltd., which involved immunizing two alpacas. The immunizing antigen was hFc-tagged human MSLN recombinant protein (huMSLN-hFc, Kaika Biotech, MSL-HM280). For the first immunization, complete Freund's adjuvant (CFA) and huMSLN-hFc were mixed to perform subcutaneous immunization, and for the remaining booster immunizations, incomplete Freund's adjuvant (IFA) and huMSLN-hFc were mixed to perform subcutaneous immunization three times.

### Example 7: Establishment of phage antibody library

After the fourth immunization of alpacas, 50 ml of peripheral blood was taken, and alpaca PBMC was isolated according to the instructions for lymphocyte separation solution (Haoyang Biotech, LTS1077). Then RNAiso Plus (takara, 9109) reagent was used to extract total RNA, see the instructions of PrimeScript^{™} II 1 st Strand cDNA Synthesis Kit, and 5 µg of RNA was transcribed to obtain cDNA. The obtained cDNA was used as a template to amplify the heavy chain variable region (VHH) fragment, then ligated with the digested vector, and electrotransduced into TG1 competent cells. One alpaca bacterial library had a size of 2.14×10⁹, and the other alpaca bacterial library had a size of 7.8×10⁹.

### Example 8: MSLN single-domain antibody screening

The two constructed alpaca libraries underwent both cell and protein screening. After three or four rounds of cell screening and two rounds of protein screening, the screened bacterial library plasmids were extracted, subjected to PCR amplification of VHH fragment, ligated to expression vector, and clones were selected to induce VHH secretion expression.

The coating of antigens of human MSLN-his (huMSLN-his, Kaiji Biotech, MSL-HM18D), monkey MSLN-his (cynoMSLN-his, Kaiji Biotech, MSL-CM180), and mouse MSLN-his (muMSLN-his, Kaiji Biotech, MSL-MM180) was carried out, in which the antigens were diluted to 2µg/ml in the antigen coating solution (carbonate buffer with pH 9.6), then added to an enzyme plate, 50µl/well, and coating was carried out at 2-8°C overnight; washing was performed three times with PBST on the next day, 2% BSA was added for blocking for 2 hours at 37°C; the induced expression supernatant was added to each well, 100 µl/well, and incubated at 37°C for 1 hour; washing was performed three times with PBST, then HRP-labeled anti-DYKDDDDK antibody (Wuhan Sanying Biotech, HRP-66008) was added, 50µl/well, and incubated at 37°C for 1 hour; washing was performed three times with PBST, and then TMB was added for color development and reading.

An MSLN single-domain antibody with good crossing over human, monkey and mouse was obtained and named as 3C6. The antibody was sequenced, the sequence analysis was performed using CLC software, and the clone with unique sequence was induced, expressed and purified. The VHH amino acid sequence of 3C6 was set forth in SEQ ID NO: 149, and its CDR sequences were shown in Table 6.

**Table 6: CDR sequences of 3C6 were as follows:**

| Antibody name | CDR coding method | CDR1 (SEQ ID NO:) | CDR2 (SEQ ID NO:) | CDR3 (SEQ ID NO:) |
|---|---|---|---|---|
| 3C6 | IMGT | 150 | 151 | 152 |
| | Kabat | 153 | 154 | 155 |
| | AbM | 156 | 157 | 155 |
| | Chothia | 158 | 159 | 155 |
| | Contact | 160 | 161 | 162 |

### Example 9: Induction, expression and purification of MSLN single-domain antibody

The preserved glycerol bacteria were taken and activated in 2YT medium (containing Amp) at 37°C and 250rpm overnight, inoculated on the next day into a fresh medium at a ratio of 1:100, and cultured (37°C, 250rpm) overnight until OD₆₀₀=0.6; then an induction medium was added for induction and cultured overnight (30°C, 250rpm). After induction, the supernatant was collected by centrifugation and purified using Ni (NTA type) affinity chromatography medium gravity column to obtain MSLN single-domain antibody.

### Example 10: Affinity detection of MSLN single-domain antibody

ELISA affinity determination: The coating of human MSLN-his (huMSLN-his, Kaika Biotech, MSL-HM18D), monkey MSLN-his (cynoMSLN-his, Kaika Biotech, MSL-CM180), and mouse MSLN-his (muMSLN-his, Kaika Biotech, MSL-MM180) was carried out overnight at 4°C. After being blocked with 2% BSA for 2 hours, the single-domain antibody at different dilutions was added to each well (for the antibody, starting from 100 nM, 3-fold dilution, 8 concentrations), and incubated for 1 hour; HRP-labeled anti-DYKDDDDK antibody (Wuhan Sanying Biotech, HRP-66008) was added, after color development with TMB solution, 2M hydrochloric acid was used for termination, and the absorbance at 450nm was read. The EC₅₀ was counted using Graphpad Prism software, and the affinity results were shown in Table 7.

**Table 7: Binding of MSLN single-domain antibody to MSLN proteins**

| Antibody name | huMSLN (nM) | cynoMSLN (nM) | muMSLN (nM) |
|---|---|---|---|
| 3C6 | 0.463 | 0.7877 | 28.64 |

### Example 11: Affinity determination of anti-MSLN single-domain antibody at cellular level

50 µl of MC38/MSLN cells (2_{×}10⁵ cells) obtained in Example 5 was taken and added to a 96-well V-shaped plate, then 50 µl of serially diluted antibody was added to each well (for the antibody, starting from 300 nM, 3-fold dilution, 8 concentrations), and incubated on ice for 1 hour. After washing away the primary antibody, the diluted Alexa Fluro488-labeled anti-DYKDDDDK antibody (Wuhan Mitaka Biotech, HRP-66008) was added, and incubated on ice for 1 hour, after washing 4 times, resuspension was performed in each well with 200 µl of PBS, and then detection was performed on a flow cytometer. The EC₅₀ was counted using Graphpad Prism software, and the cell affinity results were shown in Table 8.

**Table 8: Binding of MSLN single-domain antibody to MSLN stably transfected cells**

| Antibody name | MC38/MSLN cells, EC50(nM) |
|---|---|
| 3C6 | 11.82 |

### Example 12: Determination of dynamic affinity of MSLN single-domain antibody

Anti-DYKDDDDK antibody was bound to HC30M chip through amino coupling, and then captured 2µg/ml MSLN single-domain antibody. After the baseline was stable, serially diluted huMSLN-Fc (Kaika Biotech, MSL-HM280) and cynoMSLN-Fc (Kaika Biotech, MSL-CM280) (starting from 100nM, 3-fold gradient dilution, a total of 8 concentrations) flowed through the chip from low concentration to high concentration, with a binding time of 8 minutes and a dissociation time of 20 minutes. The kinetic constants were obtained by fitting with Carteria software. The results of the dynamic affinity between MSLN single-domain antibody and MSLN proteins were shown in Table 9.

**Table 9: Dynamic affinity of MSLN single-domain antibody for MSLN proteins**

| Antibody name | huMSLN-Fc | | | cynoMSLN-Fc | | |
|---|---|---|---|---|---|---|
| | ka(M-1 s-1) | kd (s-1) | KD (M) | ka(M-1 s-1) | kd (s-1) | KD (M) |
| 3C6 | 5.36E+04 | 3.04E-04 | 5.66E-09 | 4.53E+04 | 4.69E-05 | 1.04E-09 |

### Example 13: Expression and purification of CD3-MSLN bispecific antibody

### 1. Construction of CD3-MSLN bispecific antibody

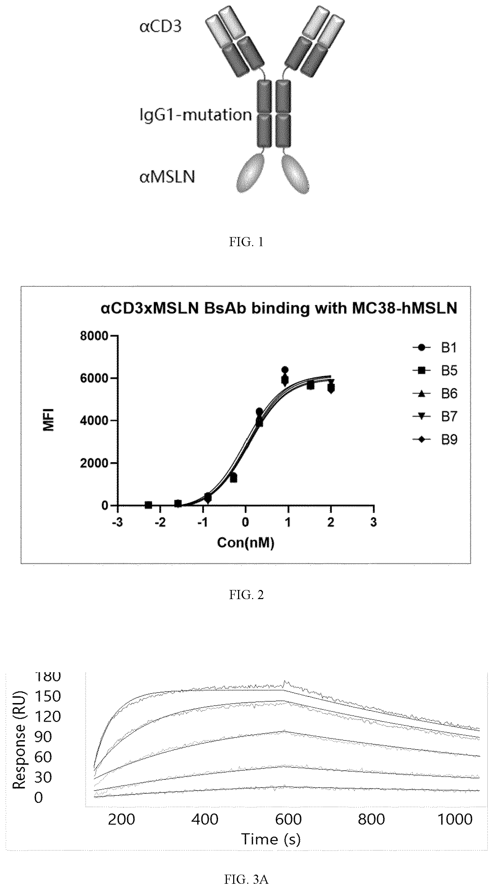

The structure of CD3-MSLN bispecific antibody in this example was shown in FIG. 1, in which the humanized CD3 antibody had two arms (heavy and light chain pairing), and the C-terminal of mutant IgG1 was connected to the MSLN single-domain antibody (VHH antibody) via (GGGGS)₂ to form the CD3-MSLN bispecific antibody (bivalent). Specifically, the VH of the humanized CD3 antibody obtained in Example 1 was connected to the IgG1 mutant heavy chain constant region (SEQ ID NO: 164), and the C-terminal of the heavy chain constant region was further connected to the above-mentioned 3C6 antibody through the linker (GGGGS)₂ to form a first polypeptide chain with the structure shown as [VH]-[CH]-[L]-[VHH]; the VL-1 of the humanized CD3 antibody obtained in Example 1 was connected to the light chain constant region (SEQ ID NO: 165) to form a second polypeptide chain with the structure shown as [VL]-[CL]. The nucleic acid sequences encoding the above polypeptide chains were constructed into the PTT5 plasmid vector (Ubao Biotech, lot: VT2202), and sufficient plasmids were extracted for later use.

### 2. Expression and purification of CD3-MSLN bispecific antibody

On the day before transfection (D-1), the cells were diluted with a culture medium to reach a density of 2_{×}10⁶ cells/ml. On the day of transfection (D0), the cells were taken and counted (cell viability should be ≥95%), and the cell density was adjusted to 4.0×10⁶ cells/ml; the antibody fragment plasmids were mixed at a ratio of heavy chain:light chain = 1:1, then mixed with PEI, and co-transfected CHO-S cells. After transfection was completed, the cells were transferred into an 8% COz incubator and cultured at 37°C, 120rpm. On the first day after transfection (D1), the preheated CHOgro complete culture medium was supplemented by 1/5 volume of expression system, cooled to 32°C and continuously cultured. On the second, fourth and sixth days after transfection, Advanced CHO Feed1 was supplemented by 8%, 5%, and 5% respectively, and the cell viability was monitored every day starting from D7. The cells were harvested when the viability rate was <80%; centrifugation was carried out, and the supernatant was collected and purified using a protein A column for later use. The purity was shown in Table 10. For convenience of writing, the bispecific antibody containing VH1 was named as B1, and the bispecific antibody containing VH2 was named as called B2, and so on.

**Table 10: Purity detection results of bispecific antibodies**

| Antibody name | B1 | B2 | B3 | B4 | B5 | B6 | B7 | B8 | B9 | B10 |
|---|---|---|---|---|---|---|---|---|---|---|
| SEC% | 93.5 | 100 | 92.9 | 97 | 95 | 93.5 | 94.5 | 97.45 | 91.1 | 99.4 |
| Antibody name | B11 | B12 | B13 | B14 | B15 | B16 | B17 | B18 | B19 | B20 |
| SEC% | 98.1 | 97.8 | 96.8 | 96 | 96.6 | 94.7 | 97.6 | 93.7 | 93.8 | 97.8 |
| Antibody name | B21 | B22 | B23 | B24 | B25 | B26 | B27 | B28 | B29 | B30 |
| SEC% | 98.4 | 93.3 | 91.3 | 86 | 95.8 | 94.8 | 91 | 99.2 | 80 | 91.2 |
| Antibody name | B31 | B32 | B33 | B34 | B35 | B36 | B37 | B38 | B39 | B40 |
| SEC% | 94.6 | 96.1 | 93.5 | 95.4 | 95 | 96.2 | 96.2 | 96.49 | 92.8 | 93.7 |
| Antibody name | B41 | B42 | B43 | B44 | B45 | B46 | | | | |
| SEC% | 94.9 | 96.3 | 91.7 | 95.1 | 93.8 | 96.4 | | | | |

### Example 14: Determination of binding activity of CD3-MSLN bispecific antibodies

1. The binding activity of the CD3-MSLN bispecific antibodies to Jurkat (naturally expressing human CD3) and cynomolgus monkey HSC-F cells (Company: JCRB Cell Bank; Cat. No.: JCRB1164) was investigated, in which two positive control antibodies were used.

The bispecific antibody structures of positive control antibody OKT3-3C6 and control antibody 1-3C6 were constructed with reference to the "CD3-MSLN bispecific antibody" in Example 13, in which, for the OKT3-3C6, the CD3 antibody VH sequence had the amino acid sequence as set forth in SEQ ID NO: 166, the CD3 antibody VL sequence had the amino acid sequence as set forth in SEQ ID NO: 167, and VHH was the 3C6 antibody of MSLN; while for the control antibody 1-3C6, the CD3 antibody VH sequence had the amino acid sequence as set forth in SEQ ID NO: 168, the CD3 antibody VL sequence had the amino acid sequence as set forth in SEQ ID NO: 169, and VHH was the 3C6 antibody of MSLN.

The experimental method was as follows:
(1) Preparation of cells: Jurkat and HSC-F cells were adjusted to 1*10⁷ cells/ml with PBS, and 50 µl of cell suspension was prepared and added into each sample.
(2) Preparation of antibodies: PBS was used to adjust the antibodies to a final concentration of 2 µg/ml, then 3-fold gradient dilution was performed to obtain 8 gradients, and 50 µl of antibody was added to each well.
(3) Incubation of cells and antibodies: 50 µl of the cell suspension was taken, that was, the total number of cells was 5*10⁵ cells/well. The final concentration of the antibody was the starting concentration of 2 µg/ml, three-fold gradient dilution was carried out in 8 wells, and incubation was performed at 4°C for 1 hour.
(4) After centrifugation at 500g for 5min, the supernatant was gently removed, and washing was performed twice by adding 200µl of PBS.
(5) Addition of secondary antibody: Fluorescent secondary antibody diluted in PBS (used at 1:100) was added, and incubated at 4°C for 30min.
(6) The fluorescent secondary antibody was rinsed, centrifugation was carried out at 500g for 5 minutes, then the supernatant was gently removed, washing was performed once by adding 200µl of PBS, and then upflow detection was performed.

The results were shown in Table 11. The results showed that most of the CD3-MSLN bispecific antibodies of the present disclosure have the same order of magnitude in binding activity for human CD3-expressing cells and binding activity for cynomolgus monkey CD3-expressing cells.

**Table 11: Determination of binding activity of bispecific antibodies for CD3-expressing cells**

| Antibody name | Human CD3-expressing Jurkat cells (EC₅₀ nM) | Cynomolgus monkey CD3-expressing HSC-F cells (EC₅₀ nM) |
|---|---|---|
| B1 | 0.5675 | 0.2092 |
| B2 | 0.1307 | 0.1824 |
| B3 | 0.2709 | 0.2532 |
| B4 | 0.3251 | 0.5849 |
| B5 | 0.1809 | 0.2303 |
| B6 | 0.1589 | 0.2371 |
| B7 | 1.3634 | 0.6927 |
| B8 | 0.2478 | 0.1655 |
| B9 | 1.3603 | 0.3286 |
| B10 | 2.7062 | 0.2159 |
| B11 | 0.1061 | 0.1447 |
| B12 | 0.2423 | 0.1792 |
| B13 | 0.7781 | 0.2471 |
| B14 | 1.9584 | 0.2903 |
| B15 | 0.1703 | 0.1644 |
| B16 | 0.4332 | 0.2044 |
| B17 | 0.1454 | 0.1466 |
| B18 | 0.5627 | 0.9541 |
| B19 | 0.1412 | 0.1505 |
| B20 | 54.1672 | 9.9997 |
| B21 | 67.7721 | 1.2543 |
| B22 | 2.2294 | 0.2624 |
| B23 | 0.1934 | 0.1628 |
| B24 | 1.1713 | 0.2395 |
| B25 | 0.0973 | 0.1845 |
| B26 | 0.2178 | 0.3394 |
| B27 | 0.4775 | 0.2537 |
| B29 | 0.5054 | 0.2422 |
| B30 | 0.3045 | 0.2722 |
| B31 | 1.2897 | 0.1977 |
| B32 | 0.0566 | 0.0756 |
| B33 | 4.4573 | 1.8452 |
| B34 | 2.8245 | 0.3241 |
| B35 | 0.7361 | 0.2726 |
| B36 | 0.1854 | 0.2461 |
| B37 | 0.2257 | 0.2158 |
| B38 | 0.1649 | 0.2573 |
| B39 | 0.5818 | 0.6523 |
| B40 | 3.7714 | 0.6387 |
| B41 | 0.8161 | 0.2178 |
| B42 | 0.4391 | 0.2044 |
| B43 | 9.7995 | 0.4011 |
| B44 | 1.9512 | 0.3148 |
| B45 | 0.3764 | 0.2847 |
| B46 | 0.2025 | 0.1497 |
| OKT3-3C6 | 0.0736 | Not binding |
| Control antibody 1-3C6 | 0.2217 | 0.2802 |

### 2. Binding activity of CD3-MSLN bispecific antibodies to MC38-MSLN cells

(1) Construction of MC38-MSLN cell line: The full-length DNA sequence of MSLN (NCBI genbank: Accession # AAH09272) was synthesized into pLVX-IRES-Puro vector (Ubao Biotech, Cat. No.: VT1464); lentivirus was packaged and infected MC38 cells; after being screened under pressure, monoclonal sorting was performed to obtain a monoclonal cell line that highly expressed MSLN.
(2) Preparation of cells: MC38-MSLN cells were adjusted to 1*10⁷ cells/ml with PBS, and 50 µl of cell suspension was prepared to be added to each sample.
(3) Preparation of antibodies: PBS was used to adjust the antibodies to a final concentration of 2 µg/ml, 3-fold gradient dilution was carried out to obtain 8 gradients. 50 µl of antibody was added to each well.
(4) Incubation of cells and antibodies: 50 µl of the cell suspension was taken, that was, the total number of cells was 5*10⁵ cells/well. The antibody final concentration was the starting concentration of 2 µg/ml, three-fold gradient dilution was carried out in 8 wells, and incubation was carried out at 4°C for 1 hour.
(5) After centrifugation at 500g for 5min, the supernatant was gently removed, and washing was performed twice by adding 200µl of PBS.
(6) Addition of secondary antibody: Fluorescent secondary antibody diluted in PBS (used at 1:100) was added, and incubated at 4°C for 30min.
(7) The fluorescent secondary antibody was washed, centrifugation was carried out at 500g for 5 minutes, the supernatant was gently removed, washing was performed once by adding 200µl of PBS, and then upflow detection was performed.

The results were shown in FIG. 2. The results showed that the CD3-MSLN bispecific antibodies of the present disclosure have good binding activity to MSLN-expressing cells.

### 3. Detection of affinity of CD3-MSLN bispecific antibodies

### (1) Affinity of CD3-MSLN bispecific antibody for CD3εγ recombinant antigen

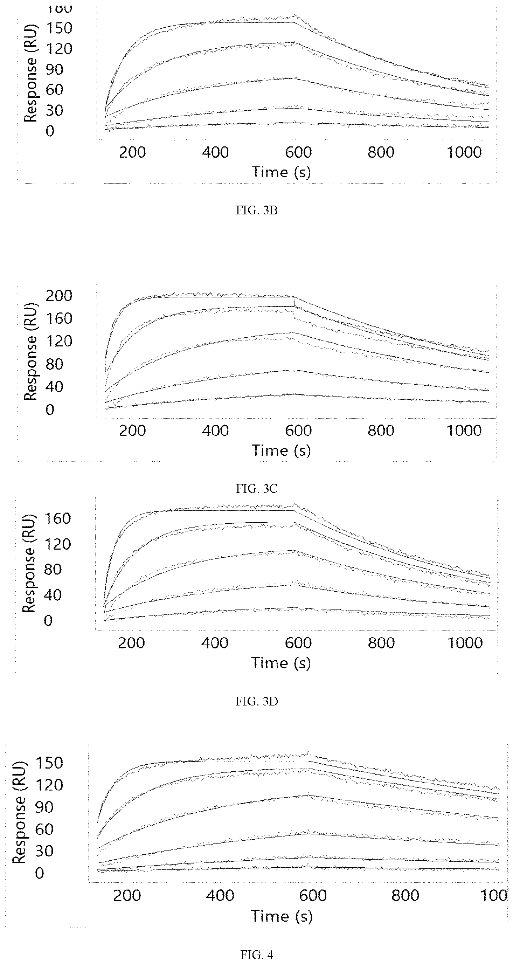

Antibodies at three concentrations of 4, 2, and 1 µg/mL were immobilized on HC200M chip containing anti-human IgG Fc secondary antibody, and the antibody binding amount was controlled to approximately 1000RU. After the baseline was stable, the serially diluted human CD3εγ (Company: acrobiosystems, Cat. No.: CDG-H52W5) recombinant antigen (starting from 592.5nM, 3-fold dilution, 8 gradients) flowed through the chip at a flow rate of 1000 µL/min, the binding time was 8 minutes, and the dissociation time was 20 minutes. The kinetic constants were obtained by fitting with the model 1:1 of Kinetics software from Carterra. The affinity determination results were shown in Table 12 below, and the dynamic affinity diagrams were shown in FIGS. 3A to 3D. The results showed that the affinity of the CD3-MSLN bispecific antibodies of the present disclosure to the human CD3εγ recombinant antigen is in the range of 10⁻⁸ to 10⁻¹⁰ M.

**Table 12: Determination results of dynamic affinity**

| Dynamic affinity | | | |
|---|---|---|---|
| Antibody name | Kon (M-1 s-1) | Koff (s-1) | KD (M) |
| B1 | 2.1E+06 | 4.2E-03 | 2.0E-09 |
| B2 | 5.5E+05 | 3.4E-03 | 6.1E-09 |
| B3 | 1.2E+06 | 1.7E-03 | 1.5E-09 |
| B4 | 1.5E+06 | 2.2E-03 | 1.5E-09 |
| B5 | 8.0E+05 | 2.7E-03 | 3.4E-09 |
| B6 | 8.4E+05 | 2.5E-03 | 3.0E-09 |
| B7 | 5.1E+05 | 1.1E-03 | 2.2E-09 |
| B8 | 6.1E+05 | 3.7E-03 | 6.1E-09 |
| B9 | 1.4E+06 | 3.5E-03 | 2.5E-09 |
| B10 | 9.3E+05 | 2.0E-03 | 2.2E-09 |
| B11 | 1.4E+06 | 2.3E-03 | 1.6E-09 |
| B12 | 1.7E+06 | 2.1E-03 | 1.2E-09 |
| B13 | 1.3E+05 | 6.5E-04 | 5.0E-09 |
| B14 | 2.3E+06 | 1.4E-03 | 6.1E-10 |
| B16 | 9.1E+05 | 1.3E-03 | 1.4E-09 |
| B17 | 6.5E+05 | 1.0E-03 | 1.6E-09 |
| B19 | 1.5E+06 | 4.9E-03 | 3.3E-09 |
| B20 | 1.1E+06 | 1.4E-03 | 1.3E-09 |
| B21 | 9.0E+05 | 2.2E-03 | 2.5E-09 |
| B22 | 1.4E+05 | 2.9E-03 | 2.1E-08 |
| B23 | 1.9E+06 | 2.6E-03 | 1.4E-09 |
| B24 | 6.6E+05 | 1.4E-03 | 2.1E-09 |
| B25 | 9.1E+05 | 5.8E-03 | 6.4E-09 |
| B26 | 1.6E+06 | 5.0E-03 | 3.1E-09 |
| B27 | 1.4E+06 | 1.8E-03 | 1.3E-09 |
| B28 | 5.4E+05 | 2.0E-03 | 3.7E-09 |
| B29 | 1.2E+06 | 4.4E-03 | 3.6E-09 |
| B30 | 6.5E+05 | 8.5E-04 | 1.3E-09 |
| B31 | 2.7E+06 | 1.8E-03 | 6.5E-10 |
| B32 | 1.0E+06 | 3.8E-03 | 3.7E-09 |
| B33 | 2.8E+05 | 6.4E-03 | 2.3E-08 |
| B34 | 4.7E+05 | 3.5E-03 | 7.4E-09 |
| B35 | 1.1E+06 | 6.8E-04 | 6.1E-10 |
| B36 | 1.7E+06 | 2.0E-03 | 1.1E-09 |
| B37 | 8.3E+05 | 4.6E-03 | 5.5E-09 |
| B38 | 1.4E+06 | 2.1E-03 | 1.5E-09 |
| B39 | 4.4E+05 | 5.2E-03 | 1.2E-08 |
| B40 | 4.3E+05 | 6.5E-04 | 1.5E-09 |
| B41 | 1.8E+06 | 2.5E-03 | 1.4E-09 |
| B42 | 1.2E+06 | 1.8E-03 | 1.4E-09 |
| B43 | 1.1E+05 | 1.7E-03 | 1.5E-08 |
| B44 | 1.1E+06 | 2.9E-03 | 2.6E-09 |
| B45 | 1.6E+06 | 1.6E-03 | 9.5E-10 |
| B46 | 1.0E+06 | 1.6E-03 | 1.6E-09 |

### (2) Affinity of CD3-MSLN bispecific antibodies to MSLN recombinant antigen

Antibodies at three concentrations of 4, 2, and 1 µg/mL were immobilized on HC200M chip containing anti-human IgG Fc secondary antibody, and the antibody binding amount was controlled to approximately 1000RU. After the baseline was stable, the serially diluted human MSLN recombinant antigen (Company: acrobiosystems, Cat. No.: MSN-H8223) (starting from 592.5nM, 3-fold dilution, 8 gradients) flowed through the chip at a flow rate of 1000 µL/min, the binding time was 8 minutes, and the dissociation time was 20 minutes. The kinetic constants were obtained by fitting with the model 1:1 of Kinetics software from Carterra. Exemplary bispecific antibody affinity determination results were shown in Table 13, and the dynamic affinity diagram was shown in FIG. 4. The results showed that the CD3-MSLN bispecific antibodies of the present disclosure all have good binding activity to MSLN.

**Table 13: Determination results of dynamic affinity**

| Dynamic affinity | | | |
|---|---|---|---|
| Antibody name | Kon (M-1 s-1) | Koff (s-1) | KD (M) |
| B1 | 1.1E+06 | 7.7E-04 | 7.0E-10 |

### Example 15: Stability experiment of Jurkat-TIGIT-luc cells

### 1. Dilution of antibody

1640+10% FBS culture medium was used to dilute the B8, B13, B22, B33, B37 and B43 antibodies to 10nM, respectively, 2-fold gradient dilution was carried out to obtain a total of 11 gradients, and 50 µl of the diluted antibody was added to each well.

### 2. Addition of Jurkat-TIGIT-luc cells

Jurkat-TIGIT-luc (Nanjing Kebai, Cat. No.: CBP74020) cells in the logarithmic phase of growth were taken, adjusted to 6*10⁵ cells/ml with 1640+10% FBS, and added to the wells containing the above antibodies, in which 50 µl of cells was added to each well. (3*10⁴ cells/well).

### 3. Incubation

The mixtures of the above cells and antibodies were gently mixed and incubated at 37°C and 5% CO₂ for 5 to 6 hours.

### 4. Loading and detection

Chemiluminescent substrate was added at 20 µl/well, and then loaded on the machine for detection.

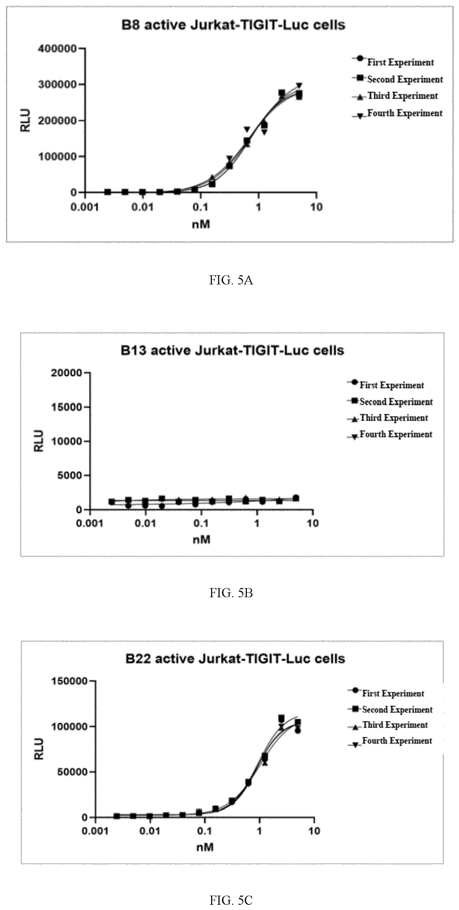

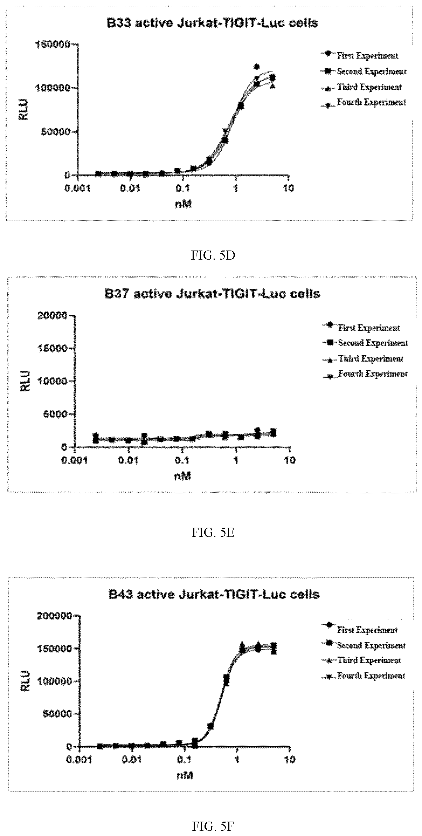

The above steps 1 to 4 were repeated four times to obtain four times experimental data of antibody activation of Jurkat-TIGIT-luc cells so as to verify the stability of Jurkat-TIGIT-luc cells. The detection results of the activated Jurkat-TIGIT-luc cells were shown in FIGS. 5A to 5F. The results showed that there was no significant difference in the results of four repeated experiments of Jurkat-TIGIT-luc cells activated by B8, B13, B22, B33, B37 and B43 antibodies, indicating that the Jurkat-TIGIT-luc cells were stable. Therefore, the Jurkat-TIGIT-luc cell line was used to conduct subsequent activation experiments of T cell activation signaling pathway.

### Example 16: Activation of T cell activation signaling pathway by CD3-MSLN bispecific antibodies

The antibodies were diluted to 2µg/ml with 1640+10% FBS, in which 3-fold gradient dilution was carried out to obtain a total of 12 gradients; 50µl of diluted antibody was added to each well. For the group without tumor cells, Jurkat-TIGIT-luc cells (Nanjing Kebai, Cat. No.: CBP74020) in the logarithmic phase of growth were taken, adjusted to 6*10⁵ cells/ml with 1640+10% FBS, and 50 µl of the cells was added to each well (3*10⁴ cells/well), mixed gently, and incubated at 37°C and 5% COz for 5 to 6 hours, then the chemiluminescent substrate was added at 20 µl/well and loaded on the machine for detection;

For the group with tumor cells, Jurkat-TIGIT-luc cells in the logarithmic phase of growth were taken, adjusted to 1.2*10⁶ cells/ml with 1640+10% FBS, and 25µl of the cells was added to each well (3*10⁴ cells/well); the MC38/MSLN cells constructed in Example 5 were digested, the digested MC38/MSLN cells were adjusted to 1.2*10⁶ cells/ml with 1640+10% FBS, 25µl of the cells was added to each well and mixed gently to achieve 3*10⁴ cells/well, and inoculated at 37°C and 5% COz for 5 to 6 hours, and the chemiluminescent substrate was added at 20 µl/well and loaded on the machine for detection.

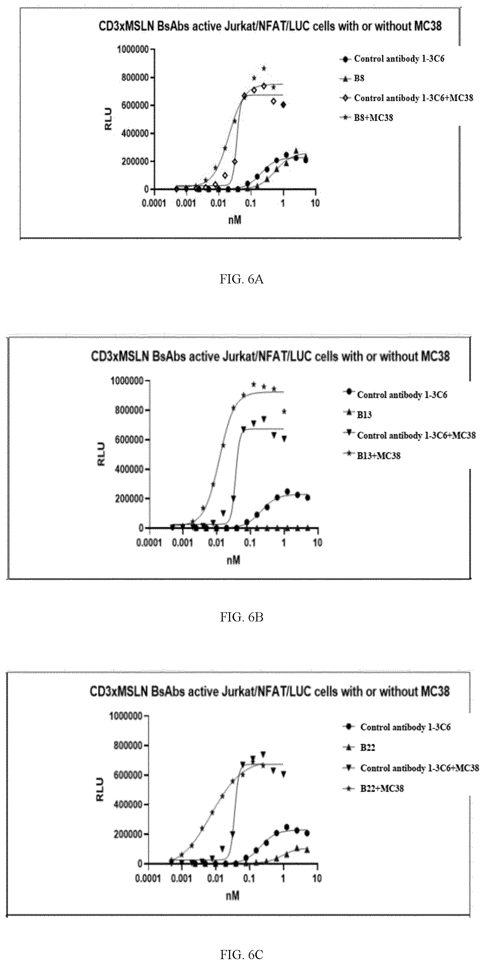

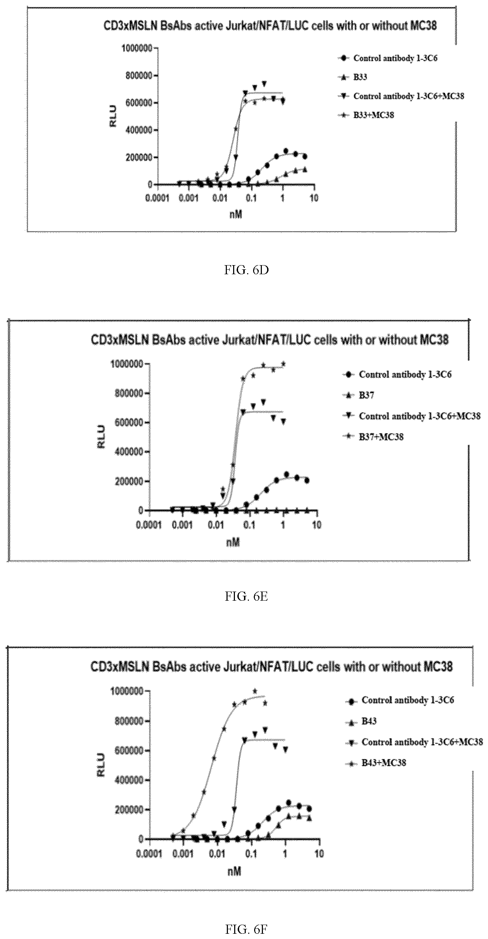

The measurement results of activation of human CD3-expressing T cells (i.e., Jurkat-TIGIT-luc) were shown in FIGS. 6A to 6F and Table 14. The results showed that when no tumor cells were added, the activation of human CD3-expressing T cells by the CD3-MSLN bispecific antibodies of the present disclosure was weaker than that of the control antibody 1-3C6, and even no activated Jurkat!NF A T /L UC cells were detected, but they could moderately activate Jurkat-TIGIT-luc cells after adding tumor cells.

**Table 14: Activation of T cell activation signaling pathway by CD3-MSLN bispecific antibodies**

| | EC₅₀ (nM) | | EC₅₀ (nM) |
|---|---|---|---|
| Control antibody 1-3C6 | 0.21 | Control antibody 1-3C6 + MC38 | 0.036 |
| B8 | 0.57 | B8+MC38 | 0.021 |
| B13 | NA | B13+MC38 | 0.012 |
| B22 | 0.91 | B22+MC38 | 0.007 |
| B33 | 0.88 | B33+MC38 | 0.026 |
| B37 | NA | B37+MC38 | 0.037 |
| B43 | 0.52 | B43+MC38 | 0.006 |

### Example 17: TDCC mediated by CD3-MSLN bispecific antibodies

(1) Resuscitation of PBMC:
   1. A 15ml centrifuge tube was prepared, and added with 5ml of inactivated complete culture medium.
   2. Frozen PBMCs were thawed in a 37°C water bath, 5 ml of the cell solution was taken, added vertically in the tube, mixed well, and centrifuged at 360g for 10 minutes.
   3. The supernatant was discarded, 3 ml of inactivated complete culture medium was added, and the cells were counted.
   4. The cell concentration was adjusted to 1×10⁶ cells/ml with inactivated complete culture medium.
(2) Detection of cell proliferation (Cell trace violet)
   1. MC38/MSLN tumor cells were digested, and centrifuged at 360g for 5 minutes.
   2. The cells were counted, 2×10⁶ cells were taken and centrifuged at 360g for 5 minutes, and the supernatant was discarded.
   3. 1 ml of serum-free 1640 was used to resuspend 2 µl of cell trace (Invitrogen C34571) to form a diluted staining solution.
   4. The cells were then resuspended in 0.5 ml of the staining solution, and incubated at 37°C in the dark for 30 minutes.
   5. The fluorescence intensity was detected by flow cytometry, in which the fluorescence intensity was more than 10² times that of the negative sample.
   6. 5 to 10ml of 1640 complete medium was added to terminate the reaction.
   7. Centrifugation was carried out at 360g for 5 minutes.
   8. The supernatant was discarded, serum-free medium was added to resuspend the cells, the cells were counted and the cell concentration was adjusted so that the tumor cell concentration reached 5×10⁴ cells/ml.
   9. A flat-bottomed 96-well plate was used to dilute the tumor cells to 5×10⁴ cells/ml and the PBMC cells to 1×10⁶ cells/ml, 100 µl of cell suspension of each kind of cells was added each time to reach a total of 200 µl per well.
   10. The final serum concentration was 5%, and culturing was carried out for 48 hours.
   11. A new 96-well plate was used to dilute the antibody, and then the diluted antibody was added.
   12. Centrifugation was carried out at 360g for 5 minutes, the supernatant was discarded, the cells were digested with trypsin, and then the reaction was terminated.
   13. The cells were stained, subjected to flow cytometry, and data were received.

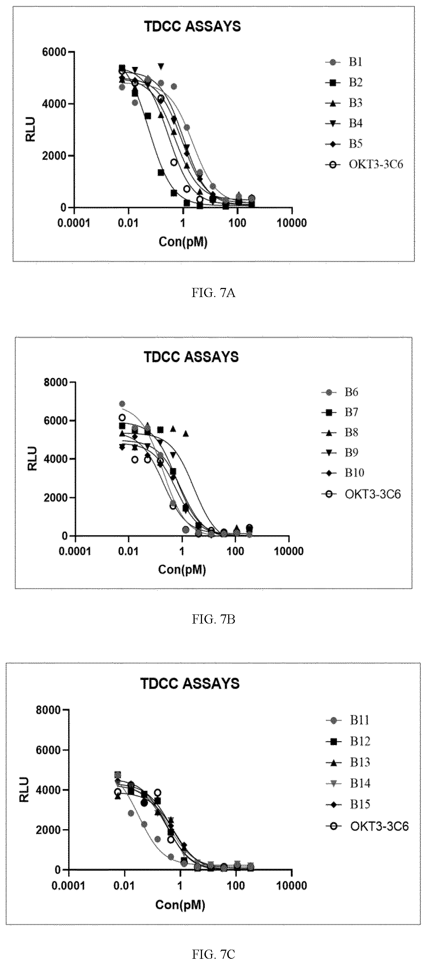

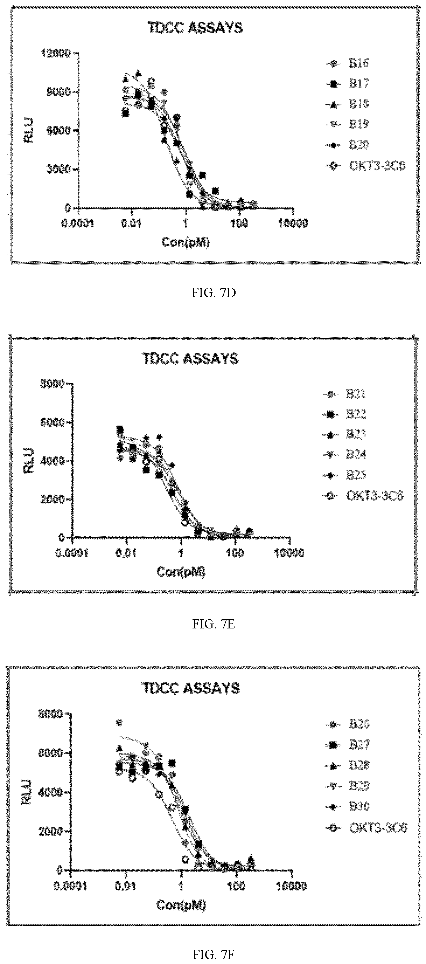

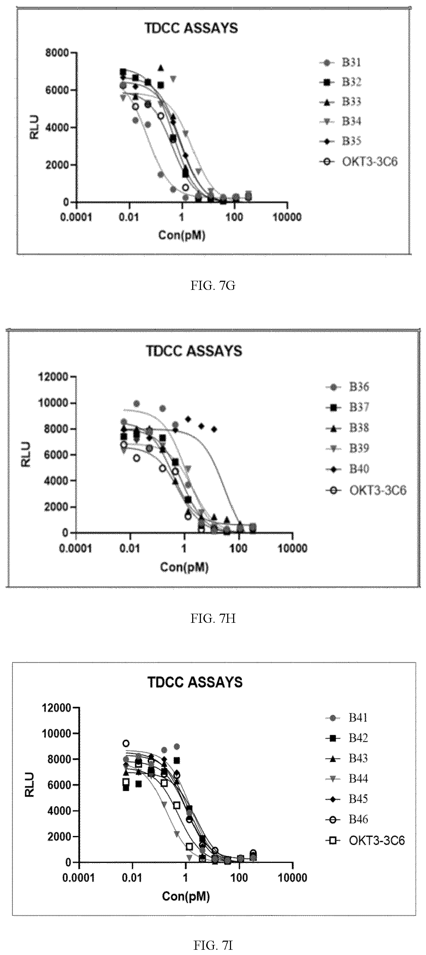

The results were shown in FIGS. 7A to 7I. The results showed that the CD3-MSLN bispecific antibodies of the present disclosure could exert T cell-mediated tumor cell killing (TDCC) effect on the tumor cells expressing tumor antigen MSLN, and the reason might be that the CD3-MSLN bispecific antibodies of the present disclosure specifically bind to the tumor cells expressing tumor antigen MSLN and T cells, recruit the T cells to the periphery of the tumor cells, activate the T cells, and induce TDCC, thereby effectively killing the tumor cells.

### Example 18: TDCC mediated by CD3-CD19 bispecific antibodies

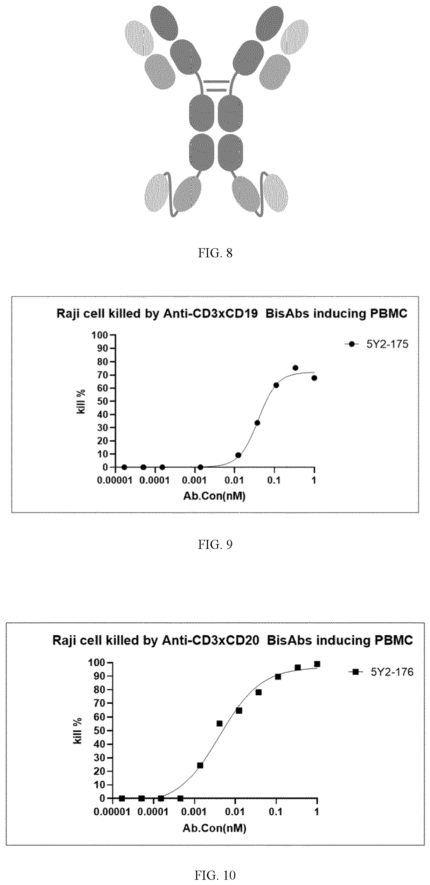

In this example, based on the CD3 antibody of the present disclosure and CD19 antibody, a bispecific antibody was constructed, which was a CD3-CD19 bispecific antibody and named as 5Y2-175, and the TDCC activity mediated by 5Y2-175 was investigated. The 5Y2-175 was prepared by referring to the method of constructing the bispecific antibody in Example 13, its structural schematic diagram was shown in FIG. 8, and its specific structure was that: the VH_{CD3} of the CD3 monoclonal antibody (M33) obtained in Example 1 was ligated to the IgG1 mutant heavy chain constant region (SEQ ID NO: 164), and the C-terminal of the heavy chain constant region was further ligated to the VH_{CD19} (SEQ ID NO: 170) and VL_{CD19} (SEQ ID NO: 171) of the above-mentioned CD19 antibody through linker (GGGGS)₂, the VH_{CD19} and VL_{CD19} were connected through (GGGGS)₂ to form a first polypeptide chain with the structure shown as [VH_{CD3]}-[CH]-[L]-[VH_{CD19}]-[L]-[VL_{CD19]}; and the VL_{CD3} of the humanized CD3 antibody (M33) obtained in Example 1 was ligated to the light chain constant region (SEQ ID NO:165) to form a second polypeptide chain with the structure shown as [VL_{CD3}]-[CL].

### 1.Preparation of Raji-luc cells

A sequence containing Luc (NCBI: GenBank: MF062157.1) was constructed and cloned into a lentiviral vector (plvx-IRES-puro, psPAX2, pMD2G). The constructed lentiviral plasmid and packaging plasmid were co-infected into HEK293T cells, the supernatant of the cells was collected after 48 hours, and added to Raji cells (ATCC, CCL-86), and 4µg/ml puromycin was added at 24 h for screening. Single cells expressing Luc were sorted by a cell sorter (Sony, LESH800SBP), and finally stable Raji-luc cells were obtained.

### 2.Preparation of PBMC

Frozen PBMC cells were resuscitated, centrifuged at 500g for 3 minutes to remove the supernatant, and washed once with 1640+10% FBS (inactivated); then centrifuged at 500g for 3 minutes to remove the supernatant, resuspended with an appropriate amount of 1640+10% FBS (inactivated) and counted, diluted to a corresponding density, and added to a well plate, 25µl per well, so that the total number of PBMC cells was 1.25*10⁵ cells/well.

### 3.Plating Raji-luc cell

25µl of Raji-luc cells were taken and added to the above PBMC well plate, 1.25*10⁴ cells/well.

### 4. Dilution of CD3-CD19 double antibody

The CD3-CD19 bispecific antibody was taken, adjusted to reach a concentration of 2nM with culture medium, and 3-fold dilution was carried out to obtain 9 gradients; 50µl of the diluted antibody was taken and added to the cells comprising PBMC cells and Raji-luc cells.

### 5.Incubation

Incubation was carried out in a 37°C, 5% CO₂ incubator for 2 days.

### 6.Reading

The well plate was taken out, added with 50µl of luciferase substrate, and data were read and analyzed on the machine.

The experimental results were shown in FIG. 9, which indicated that the CD3-CD19 bispecific antibody could exert T cell-mediated tumor cell killing (TDCC) effect on the CD19-expressing lymphoma cells.

### Example 19: TDCC mediated by CD3-CD20 bispecific antibody

In this example, based on the CD3 antibody of the present disclosure and CD20 antibody, a bispecific antibody was constructed, which was a CD3-CD20 bispecific antibody and named as 5Y2-176, and the TDCC activity mediated by the CD3-CD20 bispecific antibody was investigated. The 5Y2-176 was constructed by referring to the CD3-CD19 bispecific antibody of Example 18, in which the VH amino acid sequence of CD20 was set forth in SEQ ID NO: 172, and the VL amino acid sequence of CD20 was set forth in SEQ ID NO: 173.

### 1. Preparation of PBMC

Frozen PBMC cells were resuscitated, centrifuged at 500g for 3 minutes to remove the supernatant, washed once with 1640+10% FBS (inactivated), then centrifuged at 500g for 3 minutes to remove the supernatant, resuspended with an appropriate amount of 1640+10% FBS (inactivated) and counted, diluted to a corresponding density, and added to a well plate, 25µl per well, so that the total number of PBMC cells was 1.25*10⁵ cells/well.

### 2. Plating Raji-luc cells

25 µl of Raji-luc cells as prepared according to the preparation method of Raji-luc cells in Example 18 was taken, and added to the above-mentioned PBMC well plate, 1.25*10⁴ cells/well;

### 3. Dilution of CD3-CD20 double antibody

The CD3-CD20 antibody was taken, adjusted with culture medium to have a concentration of 2nM, and 3-fold dilution was carried out to obtain 9 gradients; and 50µl of the diluted antibody was added to the cells comprising PBMC cells and Raji-luc cells.

### 4. Incubation

Incubation was carried out in a 37°C, 5% COz incubator for 2 days.

### 5. Reading

The well plate was taken out, and added with 50µl of luciferase substrate, and data were read and analyzed on the machine.

The experimental results were shown in FIG. 10, which indicated that the CD3-CD20 bispecific antibody could exert T cell-mediated tumor cell killing (TDCC) effect on the CD20-expressing lymphoma cells.

### Example 20: TDCC mediated by CD3-trop2 bispecific antibody

In this example, based on the CD3 antibody of the present disclosure and trop2 antibody, a bispecific antibody was constructed, which was a CD3-trop2 bispecific antibody and named as 5Y2-174, and the TDCC activity mediated by the CD3-trop2 bispecific antibody was investigated. The 5Y2-174 bispecific antibody was prepared according to the method of constructing bispecific antibody in Example 13, and the humanized CD3 antibody involved in its specific structure was M33, and the VHH amino acid sequence of trop2 was set forth in SEQ ID NO: 174.

### 1. Construction of HCC1806-luc cells:

A sequence containing Luc gene was constructed and cloned into a lentiviral vector (plvx-IRES-puro, psPAX2, pMD2G), and the constructed lentiviral plasmid and packaging plasmid were co-infected into HEK293T cells, and the cell supernatant was collected at 48h, added to HCC1806 cells (Nanjing Kebai, CBP60373), and 4µg/ml puromycin was added at 24h for screening. The Luc-expressing single cells were sorted by a cell sorter (Sony, LESH800SBP), and stable HCC1806-luc cells were finally obtained.

### 2. Preparation of PBMC

Frozen PBMC cells were resuscitated, centrifuged at 500g for 3 minutes to remove the supernatant, washed once with 1640+10% FBS (inactivated), then centrifuged at 500g for 3 minutes to remove the supernatant, resuspended with an appropriate amount of 1640+10% FBS (inactivated) and counted, diluted to a corresponding density, and added to a well plate, 25µl per well, so that the total number of PBMC cells was 1.25*10⁵ cells/well.

### 3. Plating HCC1806-luc cells

25µl of the above HCC 1806-luc cells were taken and added to the above PBMC well plate, 1.25*10⁴ cells/well.

### 4. Dilution of CD3-trop2 double antibody

The CD3-trop2 double antibody was taken, adjusted to have a concentration of 1nM with culture medium, and 3-fold dilution was carried to obtain 10 gradients; and 50µl of the diluted antibody was added to the cells comprising PBMC cells and HCC 1806-luc cells.

### 5. Incubation

Incubation was carried out in a 37°C, 5% COz incubator for 2 days.

### 6. Reading

The well plate was taken out, added with 50µl of luciferase substrate, and data were read and analyzed on the machine.

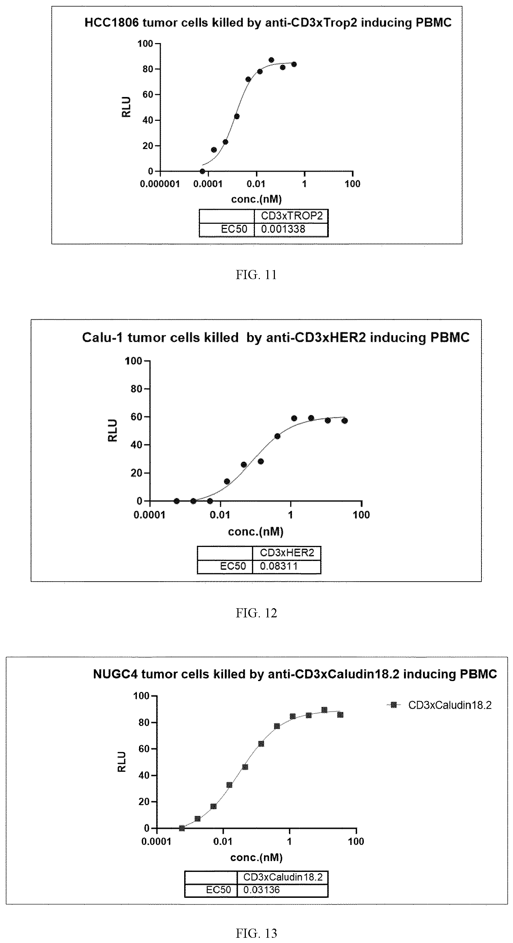

The experimental results were shown in FIG. 11, which indicated that the CD3-trop2 bispecific antibody could exert T cell-mediated tumor cell killing (TDCC) effect on trop2-expressing human breast squamous carcinoma cells.

### Example 21: TDCC mediated by CD3-Her2 bispecific antibody

In this example, based on the CD3 antibody of the present disclosure and Her2 antibody, a bispecific antibody was constructed, which was a CD3-Her2 bispecific antibody and named as 5Y2-177, and the TDCC activity mediated by the CD3-Her2 bispecific antibody was investigated. The 5Y2-177 bispecific antibody was prepared according to the method of constructing bispecific antibody in Example 13. The humanized CD3 antibody involved in its specific structure was M33, and the VHH amino acid sequence of Her2 was set forth in SEQ ID NO: 175.

### 1. Construction of Calu-l-luc cells:

A sequence containing Luc gene was constructed and cloned into a lentiviral vector (plvx-IRES-puro, psPAX2, pMD2G). The constructed lentiviral plasmid and packaging plasmid were co-infected into HEK293T cells, and the cell supernatant was collected at 48h, and added to Calu-1 cells (Nanjing Kebai, CBP60085), and 4µg/ml puromycin was added at 24h for screening. Single cells expressing Luc were sorted by a cell sorter (Sony, LESH800SBP), and finally stable Calu-l-luc cells were obtained.

### 2. Preparation of PBMC

Frozen PBMC cells were resuscitated, centrifuged at 500g for 3 minutes to remove the supernatant, washed once with 1640+10% FBS (inactivated), then centrifuged at 500g for 3 minutes to remove the supernatant, suspended with an appropriate amount of 1640+10% FBS (inactivated) and counted, diluted to a corresponding density, and added to a well plate, 25µl per well, so that the total number of PBMC cells was 1.25*10⁵ cells/well.

### 3. Plating Calu-l-luc cells

25µl of the above Calu-l-luc cells was taken and added to the above PBMC well plate, 1.25*10⁴ cells/well.

### 4. Dilution of CD3-Her2 double antibody

CD3-Her2 was taken, adjusted to have a concentration of 30nM with culture medium, and 3-fold dilution was carried out to obtain 11 gradients; and 50µl of the diluted antibody was added to the cells comprising PBMC cells and Calu-1-luc cells.

### 5. Incubation

Incubation was carried out in a 37°C, 5% CO₂ incubator for 2 days.

### 6. Reading

The well plate was taken out, and added with 50µl of luciferase substrate, and data were read and analyzed on the machine.

The experimental results were shown in FIG. 12, which indicated that the CD3-Her2 bispecific antibody could exert T cell-mediated tumor cell killing (TDCC) effect on the Her2-expressing human lung cancer cells.

### Example 22: TDCC medicated by CD3-Caludin18.2 bispecific antibody

In this example, based on the CD3 antibody of the present disclosure and Caludin18.2 antibody, a bispecific antibody was constructed, which was a CD3-Caludin18.2 bispecific antibody and named as 5Y2-178, and the TDCC activity mediated by the CD3-Caludin18.2 bispecific antibody was investigated. The 5Y2-178 bispecific antibody was prepared according to the method of constructing bispecific antibody in Example 13. The humanized CD3 antibody involved in its specific structure was M33, and the VHH amino acid sequence of Caludin18.2 was set forth in SEQ ID NO: 176.

### 1. Construction ofNUGC4-luc cells

A sequence containing Luc gene was constructed and cloned into a lentiviral vector (plvx-IRES-puro, psPAX2, pMD2G). The constructed lentiviral plasmid and packaging plasmid were co-infected into HEK293T cells, and the cell supernatant was collected at 48 h, added to NUGC4 cells (Nanjing Kebai, CBP60493), and 4µg/ml puromycin was added at 24 h for screening. The single cells expressing Luc were sorted by a cell sorter (Sony, LESH800SBP), and finally stable NUGC4-lucc cells were obtained.

### 2. Preparation of PBMC

Frozen PBMC cells were resuscitated, centrifuged at 500g for 3 minutes to remove the supernatant, washed once with 1640+10% FBS (inactivated), then centrifuged at 500g for 3 minutes to remove the supernatant, resuspended with an appropriate amount of 1640+10% FBS (inactivated) and counted, diluted to a corresponding density, and added to a well plate, 25µl per well, so that the total number of PBMC cells was 1.25*10⁵ cells/well.

### 3. Plating NUGC4-luc cells

25µl of the above NUGC4-luc cells was taken and added to the above PBMC well plate, 1.25*10⁴ cells/well.

### 4. Dilution of CD3-Caludin18.2 double antibody

The CD3-Caludin18.2 double antibody was taken, adjusted to have a concentration of 100nM with culture medium, and 3-fold dilution was carried out to obtain 12 gradients; and 50µl of the diluted antibody was added to the cells comprising PBMC cells and NUGC4-luc cells.

### 5. Incubation

Incubation was carried out in a 37°C, 5% COz incubator for 2 days.

### 6. Reading

The well plate was taken out, and added with 50µl of luciferase substrate, and data were read and analyzed on the machine.

The experimental results were shown in FIG. 13, which indicated that the CD3-Caludin18.2 bispecific antibody could exert T cell-mediated tumor cell killing (TDCC) effect on the Caludin18.2-expressing human gastric cancer cells.

### Example 23: Cytokine experiment of CD3-MSLN bispecific antibody

The bispecific antibody was adjusted to have a concentration of 1nM, 3-fold dilution was carried out to obtain 9 gradients, the diluted bispecific antibody was added (50µl/well), and the bispecific antibody was not added to the last well; the PBMC cells were adjusted to have a density of 2*10⁶ cells/ml, 50µl/ well; culturing was carried out at 37°C and 5% COz for 48 hours; 30 µl of the supernatant was taken at 24 h and 48 h, respectively, and the IL2 and INF-γ contents in the supernatant were detected by HTRF kits (cisbio 62HIL02PEH, 62HIFNGPEH).

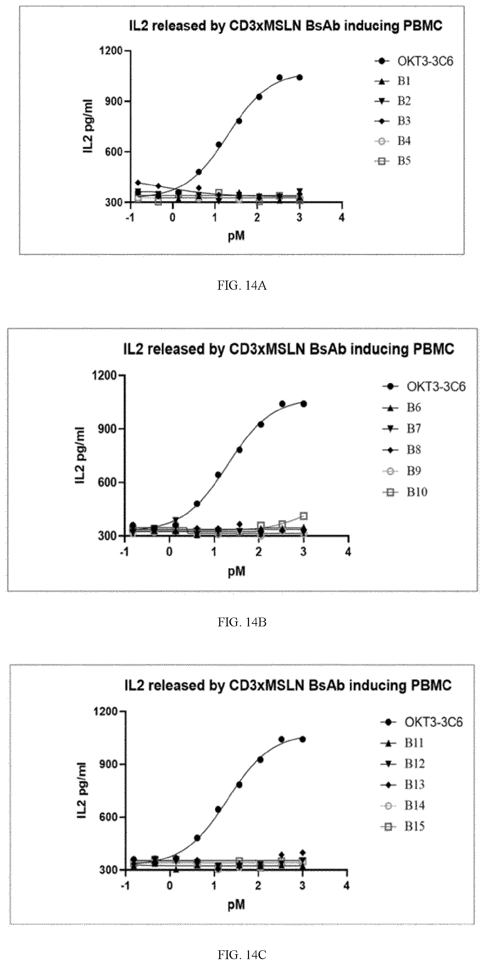

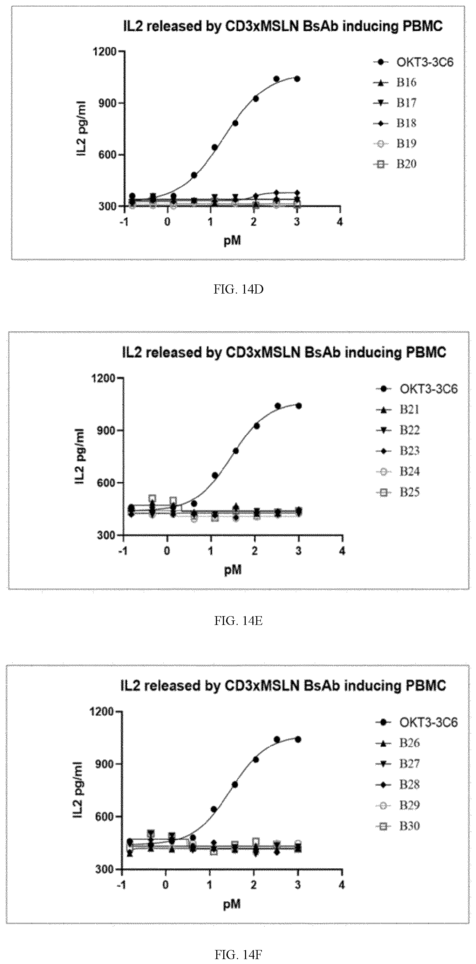

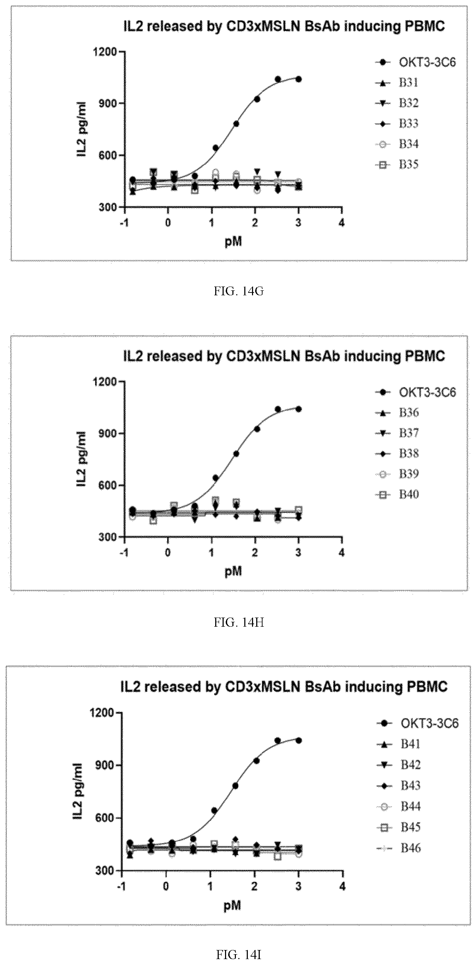

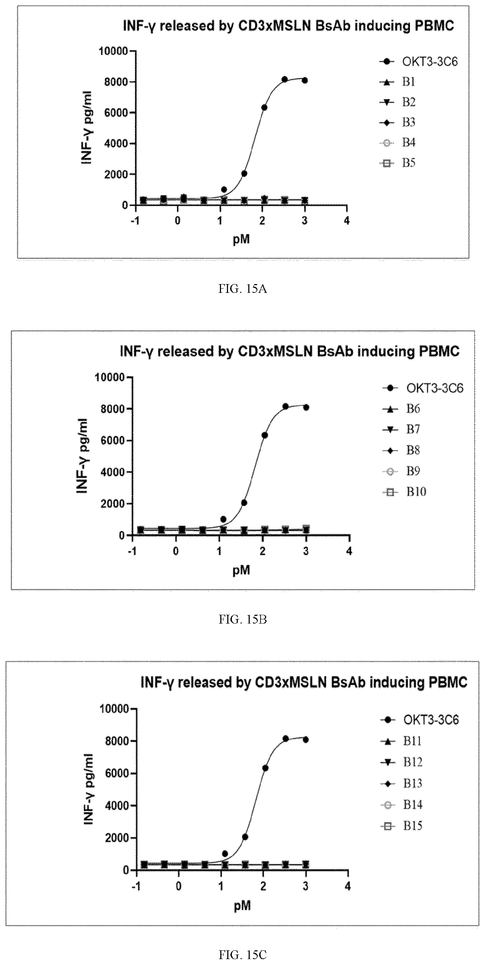

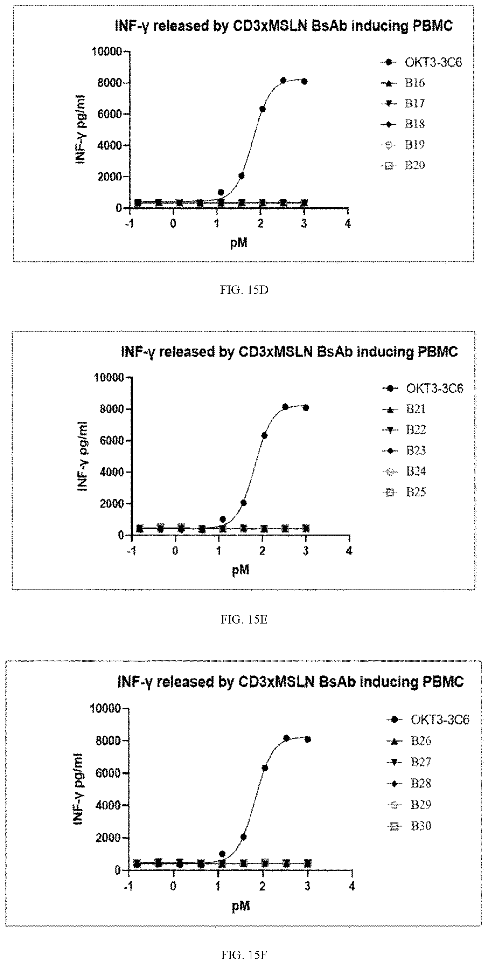

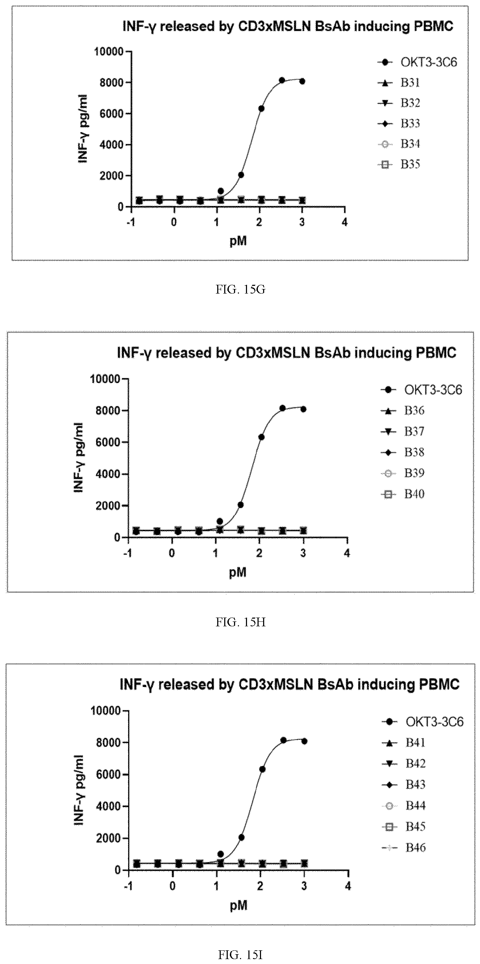

The IL2 detection results were shown in FIGS. 14A to 14I, and the INF-γ detection results were shown in FIGS. 15A to 15I. The results showed that the co-incubation of the CD3-MSLN bispecific antibody of the present disclosure with PBMC produced significantly lower IL2 and INF-γ as compared to the positive control OKT3-3C6, indicating that the CD3-MSLN bispecific antibody of the present disclosure have good safety.

Although the specific embodiments of the present disclosure have been described in detail, those skilled in the art will understand that various modifications and changes can be made to the details based on all teachings that have been disclosed, and these changes are within the protection scope of the present disclosure. The full scope of the present disclosure is given by the appended claims and any equivalents thereof.

## Claims

1. An antibody or antigen-binding fragment thereof capable of specifically binding to CD3, the antibody or antigen-binding fragment thereof comprising a heavy chain variable region (VH) and a light chain variable region (VL), wherein,
the VH comprises: an HCDR1 comprising the sequence as set forth in SEQ ID NO: 107, an HCDR2 comprising the sequence as set forth in SEQ ID NO: 108, and an HCDR3 comprising the sequence as set forth in X₁X₂X₃X₄X₅X₆X₇X₈X₉X₁₀WX₁₁X₁₂X₁₃ (SEQ ID NO: 112); wherein X₁ is A, H or P; X₂ is A, E, G, H, K, Q or S; X₃ is D, N or R; X₄ is F or P; X₅ is G, K, L, P, Q, R, S, V, W or Y; X₆ is M, N, Q or R; X₇ is G, N, S or T; X₈ is A, Q, R or Y; X₉ is G, I or V; X₁₀ is N or S; X₁₁ is F or W; X₁₂ is A, E, K or Q; X₁₃ is H, L, M, S or Y; with the proviso that the HCDR3 is not set forth in SEQ ID NO: 90.

2. The antibody or antigen-binding fragment thereof according to claim 1, wherein the HCDR3 comprises: the sequence as set forth in X₁X₂X₃FX₄NX₅YX₆SWFAX₇ (SEQ ID NO: 148), wherein X₁ is H or P; X₂ is G, E, or A; X₃ is N or R; X₄ is G, K, S, or P; X₅ is T, S, or N; X₆ is V or G; X₇ is M, Y, S, or L;
preferably, the HCDR3 comprises the sequence as set forth in any one of SEQ ID NOs: 56, 61, 69, 79, 82, and 87.

3. The antibody or antigen-binding fragment thereof according to claim 1, wherein the HCDR3 comprises:
(1) the sequence as set forth in HXzNFGNSYVSWFAY (SEQ ID NO: 113), wherein X₂ is A, E, H, K, Q or S, preferably H;
(2) the sequence as set forth in HGNFX₅NSYVSWFAY (SEQ ID NO: 114), wherein X₅ is K, L, P, Q, R, S, V, W or Y, preferably K;
(3) the sequence as set forth in HGNFGNSX₈VSWFAY (SEQ ID NO: 115), wherein X₈ is A, Q or R, preferably R; or,
(4) the sequence as set forth in HGNFGNSYVSWFX₁₂Y (SEQ ID NO: 116), where X₁₂ is E, K or Q;
preferably, the HCDR3 comprises the sequence as set forth in any one of SEQ ID NOs: 75, 53, 72, 51, 57, and 64.

4. The antibody or antigen-binding fragment thereof according to claim 1, wherein the HCDR3 comprises:
(1) the sequence as set forth in X₁X₂NFGNSYVSWFAY (SEQ ID NO: 117), wherein X₁ is A or P, preferably A; X₂ is A, E, H, K, Q or S, preferably K; preferably, X₁ is A, and X₂ is K;
(2) the sequence as set forth in X₁GNFGNSYVX₁₀WFAY (SEQ ID NO: 118), wherein X₁ is A or P, preferably A; X₁₀ is N; preferably, X₁ is A, and X₁₀ is N; or,
(3) the sequence as set forth in XiGNFGNSYVSWFXizY (SEQ ID NO: 119), wherein X₁ is A or P, preferably A; X₁₂ is E, K or Q, preferably E or Q; preferably, X₁ is A, and X₁₂ is E or Q;
preferably, the HCDR3 comprises the sequence as set forth in any one of SEQ ID NOs: 50, 66, 55, and 85.

5. The antibody or antigen-binding fragment thereof according to claim 1, wherein the HCDR3 comprises:
(1) the sequence as set forth in X₁X₂NFGNSYVSWFAY (SEQ ID NO: 117), wherein X₁ is A or P, preferably A; X₂ is A, E, H, K, Q or S, preferably K; preferably, X₁ is A, and X₂ is K;
(2) the sequence as set forth in HX₂X₃FGNSYVSWFAY (SEQ ID NO: 120), wherein X₂ is A, E, H, K, Q or S, preferably E; X₃ is D or R, preferably R; preferably, X₂ is E, and X₃ is R;
(3) the sequence as set forth in HX₂NFX₅NSYVSWFAY (SEQ ID NO: 121), wherein X₂ is A, E, H, K, Q or S, preferably A or S; X₅ is K, L, P, Q, R, S, V, W or Y, preferably L, P, S, Q, V or R; preferably, X₂ is A or S, and X₅ is L, P, S, Q, V or R; preferably, X₂ and X₅ are A/L, S/P, S/L, A/S, S/Q, S/V or S/R, respectively;
(4) the sequence as set forth in HX₂NFGX₆SYVSWFAY (SEQ ID NO: 122), wherein X₂ is A, E, H, K, Q or S, preferably S; X₆ is M, Q or R, preferably R; preferably, X₂ is S, and X₆ is R;
(5) the sequence as set forth in HX₂NFGNX₇YVSWFAY (SEQ ID NO: 123), wherein X₂ is A, E, H, K, Q or S, preferably S; X₇ is G, N or T, preferably T or G; preferably, X₂ is S, and X₇ is T or G;
(6) the sequence as set forth in HX₂NFGNSX₈VSWFAY (SEQ ID NO: 124), wherein X₂ is A, E, H, K, Q or S, preferably S; X₈ is A, Q or R, preferably R; preferably, X₂ is S, and X₈ is R;
(7) the sequence as set forth in HXzNFGNSYVSWXi iAY (SEQ ID NO: 125), wherein X₂ is A, E, H, K, Q or S, preferably S; X₁₁ is W; preferably, X₂ is S, and X₁₁ is W;
(8) the sequence as set forth in HX₂NFGNSYVSWFX₁₂Y (SEQ ID NO: 126), wherein X₂ is A, E, H, K, Q or S, preferably S; X₁₂ is E, K or Q, preferably E or Q; preferably, X₂ is S or Q, and X₁₂ is E or Q; preferably, X₂ and X₁₂ are S/E, Q/Q or S/Q, respectively; or,
(9) the sequence as set forth in HX₂NFGNSYVSWFAX₁₃ (SEQ ID NO: 127), wherein X₂ is A, E, H, K, Q or S, preferably A; X₁₃ is H, L, M or S, preferably S; preferably, X₂ is A, and X₁₃ is S;
preferably, the HCDR3 comprises the sequence as set forth in any one of SEQ ID NOs: 50, 69, 49, 58, 59, 60, 70, 82, 86, 71, 68, 77, 78, 81, 62, 84, 89, and 74.

6. The antibody or antigen-binding fragment thereof according to claim 1, wherein the HCDR3 comprises:
(1) the sequence as set forth in HX₂NFX₅NSYVSWFAY (SEQ ID NO: 121), wherein X₂ is A, E, H, K, Q or S, preferably A or S; X₅ is K, L, P, Q, R, S, V, W or Y, preferably L, P, S, Q, V or R; preferably, X₂ is A or S, and X₅ is L, P, S, Q, V or R; preferably, X₂ and X₅ are A/L, S/P, S/L, A/S, S/Q, S/V or S/R, respectively;
(2) the sequence as set forth in HGNFX₅X₆SYVSWFAY (SEQ ID NO: 129), wherein X₅ is K, L, P, Q, R, S, V, W or Y, preferably P or K; X₆ is M, Q or R, preferably M or Q; preferably, X₅ is P or K, and X₆ is M or Q; preferably, X₅ and X₆ are P/M or K/Q, respectively;
(3) the sequence as set forth in HGNFX₅NSYX₉SWFAY (SEQ ID NO: 130), wherein X₅ is K, L, P, Q, R, S, V, W or Y, preferably K or Q; X₉ is G or I; preferably, X₅ is K or Q, and X₉ is G or I; preferably, X₅ and X₉ are K/G or Q/I, respectively;
(4) the sequence as set forth in HGNFXsNSYVSWFXizY (SEQ ID NO: 131), wherein X₅ is K, L, P, Q, R, S, V, W or Y, preferably Q or L; X₁₂ is E, K or Q, preferably Q; preferably, X₅ is Q or L, and X₁₂ is Q; or,
(5) the sequence as set forth in HGNFX₅NSYVSWFAX₁₃ (SEQ ID NO: 132), wherein X₅ is K, L, P, Q, R, S, V, W or Y, preferably Y or W; X₁₃ is H, L, M or S, preferably H or S; preferably, X₅ is Y or W, and X₁₃ is H or S; preferably, X₅ and X₁₃ are Y/H or W/S, respectively;
preferably, the HCDR3 comprises the sequence as set forth in any one of SEQ ID NOs: 49, 58, 59, 60, 70, 82, 86, 76, 80, 61, 88, 52, 83, 54, and 65.

7. The antibody or antigen-binding fragment thereof according to claim 1, wherein the HCDR3 comprises:
(1) the sequence as set forth in XiGNFGNSYVSWFXizY (SEQ ID NO: 119), wherein X₁ is A or P, preferably A; X₁₂ is E, K or Q, preferably Q; preferably, X₁ is A, and X₁₂ is Q or E;
(2) the sequence as set forth in HX₂NFGNSYVSWFX₁₂Y (SEQ ID NO: 126), wherein X₂ is A, E, H, K, Q or S, preferably S; X₁₂ is E, K or Q, preferably E or Q; preferably, X₂ is S, and X₁₂ is E or Q; preferably, X₂ and X₁₂ are S/E, Q/Q or S/Q, respectively;
(3) the sequence as set forth in HGNFX₅NSYVSWFX₁₂Y (SEQ ID NO: 131), wherein X₅ is K, L, P, Q, R, S, V, W or Y, preferably Q or L; X₁₂ is E, K or Q, preferably Q; preferably, X₅ is Q or L, and X₁₂ is Q; or,
(4) the sequence as set forth in HGNFGNSX₈VSWFX₁₂Y (SEQ ID NO: 134), wherein X₈ is A, Q or R, preferably A or Q; X₁₂ is E, K or Q, preferably Q; preferably, X₈ is A or Q, and X₁₂ is Q;
preferably, the HCDR3 comprises the sequence as set forth in any one of SEQ ID NOs: 55, 85, 62, 84, 89, 52, 83, 67, and 73.

8. The antibody or antigen-binding fragment thereof according to claim 1, wherein the HCDR3 comprises:
(1) the sequence as set forth in HX₂NFGNSYVSWFAX₁₃ (SEQ ID NO: 127), wherein X₂ is A, E, H, K, Q or S, preferably A; X₁₃ is H, L, M or S, preferably S; preferably, X₂ is A, and X₁₃ is S;
(2) the sequence as set forth in HGNFX₅NSYVSWFAX₁₃ (SEQ ID NO: 132), wherein X₅ is K, L, P, Q, R, S, V, W or Y, preferably Y or W; X₁₃ is H, L, M or S, preferably H or S; preferably, X₅ is Y or W, and X₁₃ is H or S; preferably, X₅ and X₁₃ are Y/H or W/S, respectively; or,
(3) the sequence as set forth in HGNFGNX₇YVSWFAX₁₃ (SEQ ID NO: 133), wherein X₇ is G, N or T, preferably T or N; X₁₃ is H, L, M or S, preferably M or S; preferably, X₇ is T or N, and X₁₃ is M or S; preferably, X₇ and X₁₃ are T/M or N/S, respectively;
preferably, the HCDR3 comprises the sequence as set forth in any one of SEQ ID NOs: 74, 54, 65, 56, and 79.

9. The antibody or antigen-binding fragment thereof according to claim 1, wherein the HCDR3 comprises:
(1) the sequence as set forth in HX₂X₃FGNSYVSWFAY (SEQ ID NO: 120), wherein X₂ is A, E, H, K, Q or S, preferably E; X₃ is D or R, preferably R; preferably, X₂ is E, and X₃ is R; or,
(2) the sequence as set forth in HGX₃X₄GNSYVSWFAY (SEQ ID NO: 128), wherein X₃ is D or R, preferably D; X₄ is P; preferably, X₃ is D, and X₄ is P;
preferably, the HCDR3 comprises the sequence as set forth in any one of SEQ ID NOs: 69 and 63.

10. The antibody or antigen-binding fragment thereof according to claim 1, wherein the HCDR3 comprises: the sequence as set forth in X₁GNFX₅NSYVSWFAX₁₃ (SEQ ID NO: 135), wherein X₁ is A or P, preferably P; X₅ is K, L, P, Q, R, S, V, W or Y, preferably P; X₁₃ is H, L, M or S, preferably L; preferably, X₁ is P, X₅ is P, and X₁₃ is L;
preferably, the HCDR3 comprises the sequence as set forth in SEQ ID NO: 87.

11. The antibody or antigen-binding fragment thereof according to any one of claims 1 to 10, provided that the HCDR3 is not set forth in any one of SEQ ID NOs: 60, 64, 77, and 89.

12. The antibody or antigen-binding fragment thereof according to any one of claims 1 to 11, wherein the VH comprises: an HCDR1 comprising the sequence as set forth in SEQ ID NO: 107, an HCDR2 comprising the sequence as set forth in SEQ ID NO: 108, and an HCDR3 comprising the sequence as set forth in any one of SEQ ID NOs: 49-89;
preferably, the proviso is that the HCDR3 is not set forth in any one of SEQ ID NOs: 60, 64, 77, and 89.

13. The antibody or antigen-binding fragment thereof according to any one of claims 1 to 12, wherein the VL comprises: an LCDR1 comprising the sequence as set forth in SEQ ID NO: 137, an LCDR2 comprising the sequence as set forth in SEQ ID NO: 138, and an LCDR3 comprising the sequence as set forth in SEQ ID NO: 139.

14. The antibody or antigen-binding fragment thereof according to any one of claims 1 to 13, wherein the antibody or antigen-binding fragment thereof further comprises a framework region derived from an human immunoglobulin;
preferably, the antibody or antigen-binding fragment thereof comprises a heavy chain framework region contained in an amino acid sequence encoded by a gene derived from an human heavy chain germline, and/or a light chain framework region contained in an amino acid sequence encoded by a gene derived from a human light chain germline.

15. The antibody or antigen-binding fragment thereof according to any one of claims 1 to 14, wherein the VH comprises an HFR1, an HFR2, an HFR3 and an HFR4, wherein:
the HFR1 comprises the sequence as set forth in SEQ ID NO: 109;
the HFR2 comprises the sequence as set forth in SEQ ID NO: 110;
the HFR3 comprises the sequence as set forth in VKX₁RFTISRDDSKSX₂LYLQMNX₃LKTEDTAX₄YYCVR (SEQ ID NO: 136); wherein, X₁ is G or D, X₂ is I or S, X₃ is N or S, and X₄ is M or V;
the HFR4 comprises the sequence as set forth in SEQ ID NO: 111;
preferably, the HFR3 comprises the sequence as set forth in any one of SEQ ID NOs: 91-106.

16. The antibody or antigen-binding fragment thereof according to any one of claims 1 to 15, wherein the VH comprises:
(1) the amino acid sequence as set forth in any one of SEQ ID NOs: 1-46 or variant thereof;
(2) the amino acid sequence as set forth in any one of SEQ ID NOs: 8, 13, 22, 33, 37, and 43 or variant thereof;
(3) the amino acid sequence as set forth in any one of SEQ ID NOs: 3, 5, 9, 16, 20, 25, and 28 or variant thereof;
(4) the amino acid sequence as set forth in any one of SEQ ID NOs: 2, 7, 18, and 40 or variant thereof;
(5) the amino acid sequence as set forth in any one of SEQ ID NOs: 2, 22, 1, 10, 11, 12, 23, 36, 37, 41, 24, 21, 30, 32, 35, 14, 31, 39, 45, 27, and 46 or variant thereof;
(6) the amino acid sequence as set forth in any one of SEQ ID NOs: 1, 10, 11, 12, 23, 36, 37, 41, 29, 34, 13, 44, 4, 38, 42, 6, and 17 or variant thereof;
(7) the amino acid sequence as set forth in any one of SEQ ID NOs: 7, 40, 14, 31, 39, 45, 4, 38, 42, 19, and 26 or variant thereof;
(8) the amino acid sequence as set forth in any one of SEQ ID NOs: 27, 46, 6, 17, 8, and 33 or variant thereof;
(9) the amino acid sequence as set forth in any one of SEQ ID NOs: 22 and 15 or variant thereof;
(10) the amino acid sequence as set forth in SEQ ID NO: 43 or variant thereof; or,
(11) the amino acid sequence as set forth in any one of SEQ ID NOs: 1-11, 13-15, 17-19, 21-29, 31-35, 37-44, and 46 or variant thereof;
wherein, the variant described in any one of (1) to (11) has a substitution, deletion or addition of one or several amino acids, or has a sequence identity of at least 90%, at least 91%, at least 92%, at least 93%, at least 94%, at least 95%, at least 96%, at least 97%, at least 98%, at least 99%, or 100%, as compared to the sequence from which it is derived.

17. The antibody or antigen-binding fragment thereof according to any one of claims 1 to 16, wherein the VL comprises an LFR1, an LFR2, an LFR3 and an LFR4, wherein:
the LFR1 comprises the sequence as set forth in SEQ ID NO: 140;
the LFR2 comprises the sequence as set forth in WX₁QQTPGQAX₂RX₃LIX₄ (SEQ ID NO: 144); wherein, X₁ is V or Y, X₂ is F or P, X₃ is G or T, and X₄ is G or Y;
the LFR3 comprises the sequence as set forth in GVPARFSGSX₄X₅GX₆KAALTITGAQADDESX₇YFCA (SEQ ID NO: 147); wherein, X₄ is L or I, X₅ is L or I, X₆ is D or N, and X₇ is I or D;
the LFR4 comprises the sequence as set forth in SEQ ID NO: 141;
preferably, the LFR2 comprises the sequence as set forth in SEQ ID NO: 142 or 143;
preferably, the LFR3 comprises the sequence as set forth in SEQ ID NO: 145 or 146;
preferably, the VL comprises: an LFR1 as set forth in SEQ ID NO: 140, an LFR2 as set forth in SEQ ID NO: 142, an LFR3 as set forth in SEQ ID NO: 145, and an LFR4 as set forth in SEQ ID NO: 141;
preferably, the VL comprises: an LFR1 as set forth in SEQ ID NO: 140, an LFR2 as set forth in SEQ ID NO: 143, an LFR3 as set forth in SEQ ID NO: 146, and an LFR4 as set forth in SEQ ID NO: 141.

18. The antibody or antigen-binding fragment thereof according to any one of claims 1 to 17, wherein the VL comprises: the amino acid sequence as set forth in SEQ ID NO: 47 or 48 or variant thereof, and the variant has a substitution, deletion or addition of one or several amino acids, or has a sequence identity of at least 90%, at least 91%, at least 92%, at least 93%, at least 94%, at least 95%, at least 96%, at least 97%, at least 98%, at least 99%, or 100%, as compared to the sequence from which it is derived.

19. The antibody or antigen-binding fragment thereof according to any one of claims 1 to 18, which comprises:
- a VH comprising the sequence as set forth in any one of SEQ ID NOs: 1-46 or variant thereof, and a VL comprising the sequence as set forth in SEQ ID NO: 47 or variant thereof; or,
- a VH comprising the sequence as set forth in any one of SEQ ID NOs: 1-46 or variant thereof, and a VL comprising the sequence as set forth in SEQ ID NO: 48 or variant thereof;
wherein, the variant has a substitution, deletion or addition of one or several amino acids, or has a sequence identity of at least 90%, at least 91%, at least 92%, at least 93%, at least 94%, at least 95%, at least 96%, at least 97%, at least 98%, at least 99%, or 100%, as compared to the sequence from which it is derived.

20. The antibody or antigen-binding fragment thereof according to any one of claims 1 to 19, which further comprises a constant region derived from a human immunoglobulin;
preferably, the heavy chain of the antibody or antigen-binding fragment thereof comprises a heavy chain constant region derived from a human immunoglobulin; preferably, the antibody or antigen-binding fragment thereof comprises a mutated or chemically modified Fc region that has an altered effector function as compared to a wild-type Fc region;
preferably, the light chain of the antibody or antigen-binding fragment thereof comprises a light chain constant region derived from a human immunoglobulin.

21. The antibody or antigen-binding fragment thereof according to any one of claims 1 to 20, wherein the antigen-binding fragment is selected from the group consisting of Fab, Fab', (Fab')₂, Fv, disulfide-linked Fv, scFv, and double antibody.

22. A single-domain antibody or antigen-binding fragment thereof capable of specifically binding to MSLN, wherein the single-domain antibody or antigen-binding fragment thereof comprises:
(1) the following CDRs defined by the IMGT numbering system: a CDR1 comprising the sequence as set forth in SEQ ID NO: 150 or variant thereof, a CDR2 comprising the sequence as set forth in SEQ ID NO: 151 or variant thereof, and a CDR3 comprising the sequence as set forth in SEQ ID NO: 152 or variant thereof;
(2) the following CDRs defined by the Kabat numbering system: a CDR1 comprising the sequence as set forth in SEQ ID NO: 153 or variant thereof, a CDR2 comprising the sequence as set forth in SEQ ID NO: 154 or variant thereof, and a CDR3 comprising the sequence as set forth in SEQ ID NO: 155 or variant thereof;
(3) the following CDRs defined by the AbM numbering system: a CDR1 comprising the sequence as set forth in SEQ ID NO: 156 or variant thereof, a CDR2 comprising the sequence as set forth in SEQ ID NO: 157 or variant thereof, and a CDR3 comprising the sequence as set forth in SEQ ID NO: 155 or variant thereof;
(4) the following CDRs defined by the Chothia numbering system: a CDR1 comprising the sequence as set forth in SEQ ID NO: 158 or variant thereof, a CDR2 comprising the sequence as set forth in SEQ ID NO: 159 or variant thereof, and a CDR3 comprising the sequence as set forth in SEQ ID NO: 155 or variant thereof; or,
(5) the following CDRs defined by the Contact numbering system: a CDR1 comprising the sequence as set forth in SEQ ID NO: 160 or variant thereof, a CDR2 comprising the sequence as set forth in SEQ ID NO: 161 or variant thereof, and a CDR3 comprising the sequence as set forth in SEQ ID NO: 162 or variant thereof;
wherein, the variant described in any one of (1) to (5) has a substitution, deletion or addition of one or several amino acids as compared to the sequence from which it is derived; preferably, the substitution is a conservative substitution;
preferably, the single-domain antibody or antigen-binding fragment thereof comprises:
(1) the following CDRs defined by the IMGT numbering system: a CDR1 comprising the sequence as set forth in SEQ ID NO: 150, a CDR2 comprising the sequence as set forth in SEQ ID NO: 151, and a CDR3 comprising the sequence as set forth in SEQ ID NO: 152;
(2) the following CDRs defined by the Kabat numbering system: a CDR1 comprising the sequence as set forth in SEQ ID NO: 153, a CDR2 comprising the sequence as set forth in SEQ ID NO: 154, and a CDR3 comprising the sequence as set forth in SEQ ID NO: 155;
(3) the following CDRs defined by the AbM numbering system: a CDR1 comprising the sequence as set forth in SEQ ID NO: 156, a CDR2 comprising the sequence as set forth in SEQ ID NO: 157, and a CDR3 comprising the sequence as set forth in SEQ ID NO: 155;
(4) the following CDRs defined by the Chothia numbering system: a CDR1 comprising the sequence as set forth in SEQ ID NO: 158, a CDR2 comprising the sequence as set forth in SEQ ID NO: 159, and a CDR3 comprising the sequence as set forth in SEQ ID NO: 155; or,
(5) the following CDRs defined by the Contact numbering system: a CDR1 comprising the sequence as set forth in SEQ ID NO: 160, a CDR2 comprising the sequence as set forth in SEQ ID NO: 161, and a CDR3 comprising the sequence as set forth in SEQ ID NO: 162.

23. The single-domain antibody or antigen-binding fragment thereof according to claim 22, which comprises the sequence as set forth in SEQ ID NO: 149 or variant thereof, wherein the variant has a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence from which it is derived, or has a substitution, deletion or addition of one or several amino acids as compared thereto; preferably, the substitution is a conservative substitution.

24. A multispecific antibody, which comprises a CD3-targeting antigen-binding domain and at least one antigen-binding domain targeting an additional antigen, wherein the CD3-targeting antigen-binding domain is selected from the antibody or antigen-binding fragment thereof according to any one of claims 1 to 21, and the additional antigen is selected from tumor-associated antigen (TAA) and/or immune checkpoint molecule.

25. The multispecific antibody according to claim 24, wherein the multispecific antibody is a bispecific antibody, a trispecific antibody or a tetraspecific antibody;
preferably, the multispecific antibody is a bispecific antibody, which comprises the CD3-targeting antigen-binding domain and the tumor-associated antigen-targeting antigen-binding domain;
preferably, the multispecific antibody is a trispecific antibody, which comprises the CD3-targeting antigen-binding domain, the tumor-associated antigen-targeting antigen-binding domain, and the immune checkpoint molecule-targeting antigen-binding domain.

26. The multispecific antibody according to claim 24 or 25, wherein the antigen-binding domain targeting tumor-associated antigen or immune checkpoint molecule is optionally ligated to the N-terminal and/or C-terminal of the heavy chain of the CD3-targeting antigen-binding domain via a linker, and/or ligated to the N-terminal and/or C-terminal of the light chain of the CD3-targeting antigen-binding domain;
preferably, the CD3-targeting antigen-binding domain comprises: at least one heavy chain and at least one light chain, and the antigen-binding domain targeting tumor-associated antigen or immune checkpoint molecule is ligated to the heavy chain; or, the CD3-targeting antigen-binding domain comprises: two identical heavy chains and two identical light chains, and the antigen-binding domain targeting tumor-associated antigen or immune checkpoint molecule is ligated to the two heavy chains.

27. The multispecific antibody according to any one of claims 24 to 26, wherein the tumor-associated antigen is selected from the group consisting of CD19, BCMA, EGFR, HER2, HER3, HER4, PSMA, EpCAM, EphA2, CD33, CD123, CD38, CLDN18, MSLN, TROP2, Mucin1, AFP, CD79b, GUCY2C, LRRC15, gp100, STEAP1, ROR1, 5T4, CEA, DLL3, CD20, CD7, PRAME, CDH19, CDH17, GPA33, HLA-A2, CD34, FAP, GPRC5D, GPC3, B7-H3, CLL-1, CLDN6, Flt3, NY-ESO-1, PSCA, NECTIN-4, ENPP3, IGFR-1, TSA1, Melan-A, MUC16 (CA125), MUC17, SSTR2, c-Met, B7-H6, CSPG4, CAIX, MCSP, BIRC5, BIRC7, BRCA1, BORIS, CCR5, GD2, GD3, GloboH, GM3, hTERT, LMP2, p53, PAP, PAX₃, PAX₅, PCTA-1, PLAC1, PRLR, Ras, SART-3, TRP-1, TRP-2, CD22, CD30, FOLR1, Caludin18.2, and any combination thereof; preferably, the tumor-associated antigen is selected from the group consisting of MSLN, CD19, CD20, TROP2, HER2 and Caludin18.2;
the immune checkpoint molecule is selected from the group consisting of PD-1, PD-L1, PD-L2 CTLA-4, TIM-3, Lag-3, TIGIT, CD73, VISTA, B7-H3, NKG2D, NKG2A, OX40, OX40L, CD40, CD47, LIGHT, ICOS, HVEM, BTLA, B7-H4, 4-1BB, 4-1BBL, and any combination thereof.

28. The multispecific antibody according to any one of claims 24 to 27, which comprises the CD3-targeting antigen-binding domain and an MSLN-targeting antigen-binding domain, wherein the MSLN-targeting antigen-binding domain is selected from the single-domain antibody or antigen-binding fragment thereof according to claim 22 or 23.

29. The multispecific antibody according to any one of claims 24 to 27, which comprises the CD3-targeting antigen-binding domain and a CD19-targeting antigen-binding domain, wherein the CD19-targeting antigen-binding domain comprises a light chain variable region and a heavy chain variable region, the light chain variable region comprises the sequence as set forth in SEQ ID NO: 171 or variant thereof, and the heavy chain variable region comprises the sequence as set forth in SEQ ID NO: 170 sequence or variant thereof.

30. The multispecific antibody according to any one of claims 24 to 27, which comprises the CD3-targeting antigen-binding domain and a CD20-targeting antigen-binding domain, wherein the CD20-targeting antigen-binding domain comprises a light chain variable region and a heavy chain variable region, the light chain variable region comprises the sequence as set forth in SEQ ID NO: 173 or variant thereof, and the heavy chain variable region comprises the sequence as set forth in SEQ ID NO: 172 sequence or variant thereof.

31. The multispecific antibody according to any one of claims 24 to 27, which comprises the CD3-targeting antigen-binding domain and a TROP2-targeting antigen-binding domain, wherein the TROP2-targeting antigen-binding domain comprises a VHH, and the VHH comprises the sequence as set forth in SEQ ID NO: 174 or variant thereof.

32. The multispecific antibody according to any one of claims 24 to 27, which comprises the CD3-targeting antigen-binding domain and an HER2-targeting antigen-binding domain, wherein the HER2-targeting antigen-binding domain comprises a VHH, and the VHH comprises the sequence as set forth in SEQ ID NO: 175 or variant thereof.

33. The multispecific antibody according to any one of claims 24 to 27, which comprises the CD3-targeting antigen-binding domain and a Caludin18.2-targeting antigen-binding domain, wherein the Caludin18.2-targeting antigen-binding domain comprises a VHH, and the VHH comprises the sequence as set forth in SEQ ID NO: 176 or variant thereof.

34. A multispecific antibody, which comprises the single-domain antibody or antigen-binding fragment thereof according to claim 22 or 23; preferably, the multispecific antibody is a bispecific antibody, a trispecific antibody or a tetraspecific antibody.

35. An isolated nucleic acid molecule, which encodes:
- the antibody or antigen-binding fragment thereof according to any one of claims 1 to 21, or its heavy chain variable region and/or light chain variable region;
- the single-domain antibody or antigen-binding fragment thereof according to any one of claims 22 to 23;
- the multispecific antibody according to any one of claims 24 to 33, or its polypeptide chain; or,
- the multispecific antibody according to claim 34, or its polypeptide chain.

36. A vector, which comprises the nucleic acid molecule according to claim 35.

37. A host cell, which comprises the nucleic acid molecule according to claim 35 or the vector according to claim 36.

38. A method for preparing an antibody or antigen-binding fragment thereof, a single-domain antibody or antigen-binding fragment thereof, or a multispecific antibody, comprising: culturing the host cell according to claim 37 under conditions that allow protein expression, and collecting the antibody or antigen-binding fragment thereof, the single-domain antibody or antigen-binding fragment thereof, or the multispecific antibody from a culture of the cultured host cell.

39. A pharmaceutical composition, which comprises the antibody or antigen-binding fragment thereof according to any one of claims 1 to 21, the single-domain antibody or antigen-binding fragment thereof according to any one of claims 22 to 23, or the multispecific antibody according to any one of claims 24 to 33, the multispecific antibody according to claim 34, the isolated nucleic acid molecule according to claim 35, the vector according to claim 36, or the host cell according to claim 37, and a pharmaceutically acceptable carrier and/or excipient.

40. Use of the antibody or antigen-binding fragment thereof according to any one of claims 1 to 21, the single-domain antibody or antigen-binding fragment thereof according to any one of claims 22 to 23, or the multispecific antibody according to any one of claims 24 to 33, the multispecific antibody according to claim 34, the isolated nucleic acid molecule according to claim 35, the vector according to claim 36, the host cell according to claim 37, or the pharmaceutical composition according to claim 39, in the manufacture of a medicament for preventing and/or treating a disease.

41. A method for preventing and/or treating a disease, comprising administering to a subject in need an effective amount of the antibody or antigen-binding fragment thereof according to any one of claims 1 to 21, the single-domain antibody or antigen-binding fragment thereof according to any one of claims 22 to 23, or the multispecific antibody according to any one of claims 24 to 33, the isolated nucleic acid molecule according to claim 34, the vector according to claim 35, the host cell according to claim 36, or the pharmaceutical composition according to claim 38.

42. The use according to claim 40 or the method according to claim 41, wherein the disease is a tumor, an inflammatory disease or an autoimmune disease; preferably, the tumor is selected from the group consisting of mesothelioma, ovarian cancer, pancreatic cancer, breast cancer, bile duct cancer, colon cancer, gastric cancer, fallopian tube cancer, lung cancer, acute myeloid leukemia and colorectal cancer.
